# EUROPEAN PATENT APPLICATION

(11) **EP 2 112 214 A1**
(43) Date of publication of application: **28.10.2009**
(21) Application number: 09156056.5
(22) Date of filing: 24.03.2009
(51) Int. Cl.: C09K 11/06

(54) **Dibenzo[g,p]chrysene derivatives and organic electroluminescent device using the same**

(30) Priority: 23.04.2008 KR 20080037527
(71) Applicant: Gracel Display Inc., Seoul 133-833 (KR)
(72) Inventor: Lee, Soo Yong, 472-120, Gyeonggi-do (KR); Shin, Hyo Nim, 136-060, Seoul (KR); Cho, Young Jun, 136-060, Seoul (KR); Kwon, Hyuck Joo, 130-100, Seoul (KR); Kim, Bong Ok, 135-090, Seoul (KR); Kim, Sung Min, 157-886, Seoul (KR); Yoon, Seung Soo, 135-884, Seoul (KR)
(74) Representative: Kent, Venetia Katherine

(57) **Abstract**

Provided are novel organic electroluminescent compounds, and organic electroluminescent devices employing the same in an electroluminescent layer. Specifically, the organic electroluminescent compounds according to the invention are **characterized in that t**hey are represented by Chemical Formula (1).

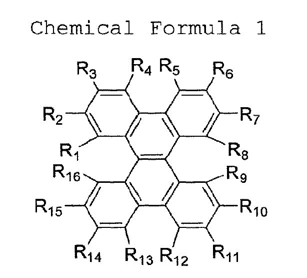

Since the organic electroluminescent compounds according to the invention have good luminous efficiency and excellent color purity and life property of material, OLED's having very good operation life can be manufactured therefrom.

## Description

### FIELD OF THE INVENTION

The present invention relates to novel organic electroluminescent compounds, and organic electroluminescent devices employing the same in an electroluminescent layer. More specifically, the invention relates to novel organic electroluminescent compounds to be employed as green or blue electroluminescent material, and organic electroluminescent devices employing the same as host.

### BACKGROUND OF THE INVENTION

Three electroluminescent materials (for red, green and blue) are employed to realize a full-colored OLED display. The important issue is to develop red, green and blue electroluminescent materials with high efficiency and long life, in order to enhance the overall feature of the organic electroluminescent (EL) devices. The EL materials are classified into host materials and dopant materials from the aspect of their functions. It is generally known that a device structure having the most excellent EL properties can be fabricated with an EL layer prepared by doping a dopant to a host. Recently, development of organic EL devices with high efficiency and long life comes to the fore as an urgent subject, and particularly urgent is development of a material with far better EL properties as compared to conventional EL materials as considering EL properties required for a medium to large sized OLED panel. From this point of view, development of host material is one of the most important issues to be settled. The desired properties for the host material (serving as a solvent and energy conveyer in solid state) are high purity and appropriate molecular weight to enable vapor-deposition in vacuo. In addition, glass transition temperature and thermal decomposition temperature should be high enough to ensure thermal stability. Further, the host material should have high electrochemical stability for providing long life. It is to be easy to form an amorphous thin film, with high adhesiveness to other materials of adjacent layers but without interlayer migration.

In the meanwhile, for conventional blue materials, a number of materials have been developed and commercialized since the development of diphenylvinyl-biphenyl (DPVBi) (Compound a) by Idemitsu-Kosan. In addition to the blue material system from Idemitsu-Kosan, dinaphthylanthracene (DNA) (Compound b) of Kodac, tetra(t-butyl)perylene (Compound c) system or the like have been known. However, extensive research and development should be performed with respect to these materials. The distryl compound system of Idemitsu-Kosan, which is known to have highest efficiency up to now, has 6 lm/W power efficiency and beneficial device lifetime of more than 30,000 hr. However, when it is applied to a full-colored display, the lifetime is merely several thousand hours, owing to decrease of color purity over operation time. In case of blue electroluminescence, it becomes advantageous from the aspect of the luminous efficiency, if the electroluminescent wavelength is shifted a little toward longer wavelength. However, it is not easy to apply the material to a display of high quality because of unsatisfactory color purity in blue. Furthermore, the research and development of such materials are urgent because of the problems in color purity, efficiency and thermal stability.

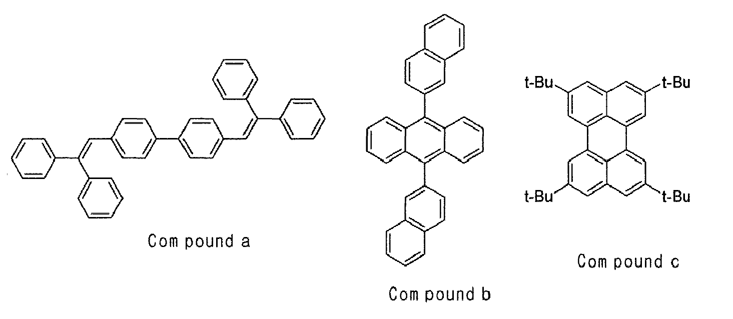

In order to develop a host material with high efficiency and long life, compounds based on different backbones have been disclosed, such as dispiro-fluorene-anthracene (TBSA), ter-spirofluorene (TSF) and bitriphenylene (BTP). These compounds, however, did not result in color purity and luminous efficiency at a sufficient level.

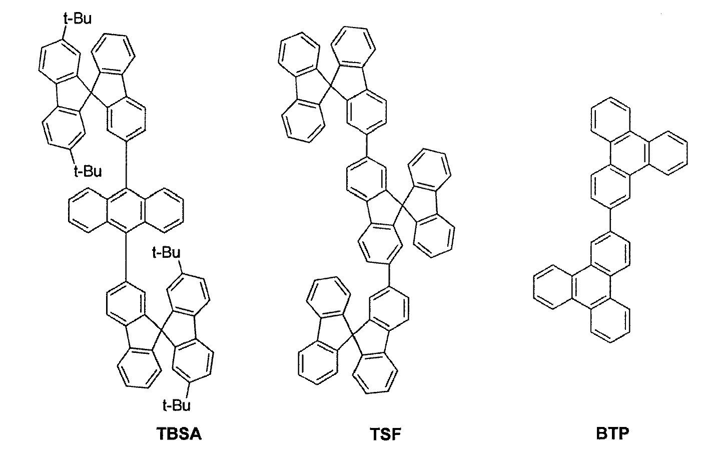

The compound TBSA reported by Gyeongsang National University and Samsung SDI (Kwon, S. K. et al., Advanced Materials, 2001, 13, 1690; Japanese Patent Laid-Open No. 2002121547), showed luminous efficiency of 3 cd/A at 7.7 V, and relatively good color coordinate of (0.15, 0.11), but it was applied as a material for single layer, being inappropriate for practical use. The compound TSF reported by Taiwan National University (Wu, C. -C. et al., Advanced Materials, 2004, 16, 61; US Patent Publication No. 2005040392) showed relatively good external quantum efficiency of 5.3%, but it was still inappropriate for practical use. The compound BTP reported by Chingwha National University of Taiwan (Cheng, C. -H. et al., Advanced Materials, 2002, 14, 1409; US Patent Publication US 2004076852) showed luminous efficiency of 2.76 cd/A and relatively good color coordinate of (0.16, 0.14), but this was still insufficient for practical use.

As described above, conventional materials are constituted of a single layer, not forming a host-dopant thin layer, and is difficult to be used practically from the aspect of color purity and efficiency. There are not enough data reliable, with respect to its long life.

In the meanwhile, according to a patent application of Mitsui Chemicals (Japan) (US Patent Publication US 7,166,240), the compounds shown below have the absorption spectra at 390 to 430 nm, with luminous efficiency of 4.6 cd/A. However, on the basis of these data, the compounds with above absorption wavelength range, electroluminescence of greenish blue color is anticipated, and the Patent Publication indicates the color as bluish green color.

Particularly, embodiment of pure blue color is impossible with the symmetrical structure of the Patent Publication, and the material, which cannot provide pure blue luminescence, is inadequate to be practically applied to a full-colored display.

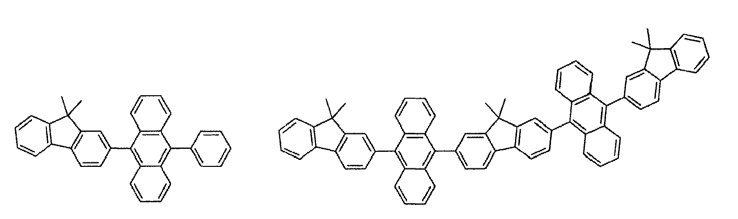

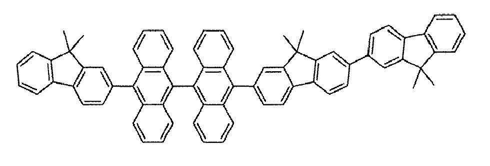

### SUMMARY OF THE INVENTION

With intensive efforts to overcome the problems of conventional techniques as described above, the present inventors have invented novel electroluminescent compounds to realize an organic electroluminescent device having excellent luminous efficiency and noticeably improved lifetime.

The object of the present invention is to provide organic electroluminescent compounds having the backbone to give more excellent electroluminescent properties, longer device life and appropriate color coordinate, as compared to those of conventional host materials, with overcoming disadvantages of them.

Another object of the invention is to provide organic electroluminescent devices of high efficiency and long life, which employ said organic electroluminescent compounds as electroluminescent material.

Thus, the present invention relates to organic electroluminescent compounds represented by Chemical Formula (1), and organic electroluminescent devices comprising the same. Since the organic electroluminescent compounds according to the invention have good luminous efficiency and excellent color purity and life property of material, OLED's having very good operation life can be manufactured therefrom:

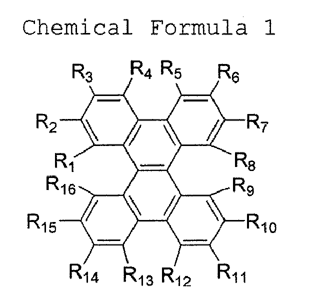

In Chemical Formula (1),

R₁ through R₁₆ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked to an adjacent substituent via (C3-C60)alkylene or (C3-C60)alkenylene to form an alicyclic ring, or a monocyclic or polycyclic aromatic ring, which may be selected from the following substituents: 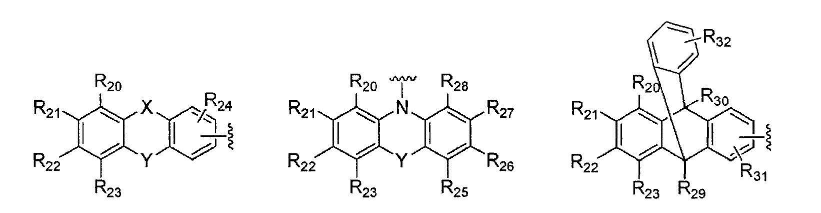 wherein, R₂₀ through R₃₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀ through R₃₂ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or -(CR₃₃R₃₄)ₙ-, -N(R₃₅)-, -S-, -O-, -Si(R₃₆)(P₃₇)-, -P(R₃₈)-, -C(=O)-, -B(R₃₉)-, -In(R₄₀)-, -Se-, -Ge(R₄₁)(R₄₂)-, -Sn(R₄₃)(R₄₄)-, -Ga(R₄₅)- or -(R₄₆)C=C(R₄₇)-; n is an integer from 1 to 4; R₃₃ through R₄₇ represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, adamantyl, bicycloalkyl, arylsilyl, alkylsilyl, alkylamino and arylamino of R₁ through R₄₇ may be further substituted by halogen, (C1-C60)alkyl with or without halogen substituent(s), (C6-C60)aryl, (C3-C60)heteroaryl with or without (C6-C60)aryl substituent, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, or an alicyclic ring, or a monocyclic or polycyclic aromatic ring formed by linkage via (C3-C60)alkylene or (C3-C60)alkenylene with or without a fused ring.

### BRIEF DESCRIPTION OF THE DRAWINGS

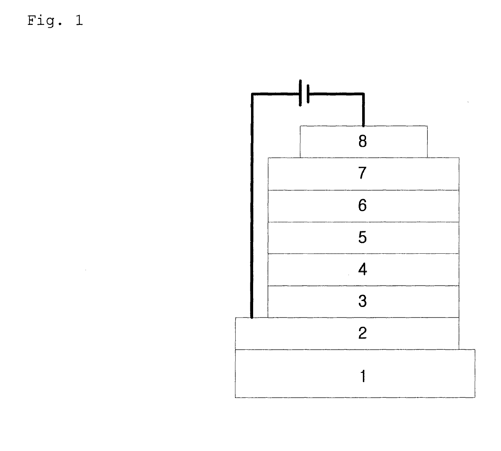

Fig. 1 is a cross-sectional view of an OLED.

### DETAILED DESCRIPTION OF THE INVENTION

Referring now to the Drawings, Fig. 1 illustrates a cross-sectional view of an OLED of the present invention comprising a Glass 1, Transparent electrode 2, Hole injecting layer 3, Hole transport layer 4, Electroluminescent layer 5, Electron transport layer 6, Electron injecting layer 7 and Al cathode 8.

The "alkyl", "alkoxy" or other substituents containing "alkyl" moiety described in the present invention include both linear and branched species.

The term "aryl" described herein means an organic radical derived from aromatic hydrocarbon via elimination of one hydrogen atom. Each ring suitably comprises a monocyclic or fused ring system containing from 4 to 7, preferably from 5 to 6 cyclic atoms. Specific examples include phenyl, naphthyl, biphenyl, anthryl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O, Si and S for the aromatic cyclic backbone atoms, and carbon atom(s) for remaining aromatic cyclic backbone atoms. The heteroaryl may be 5- or 6-membered monocyclic heteroaryl or a polycyclic heteroaryl which is fused with one or more benzene ring(s), and may be partially saturated. The heteroaryl groups may include divalent aryl groups of which the heteroatoms are oxidized or quarternized to form N-oxides, quaternary salts, or the like. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The compounds of Chemical Formula (1) may be selected from the compounds represented by Chemical Formula (2): 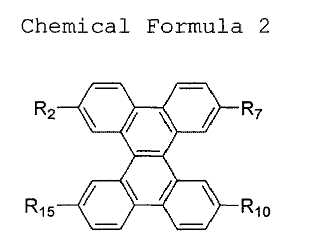 wherein, R₂, R₇, R₁₀ and R₁₅ are defined as in Chemical Formula (1).

In Chemical Formula (1) and (2), R₁ through R₁₆ may be independently selected from hydrogen, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluorethyl, trifluoroethyl, perfluoropropyl, perfluorobutyl, methoxy, ethoxy, butoxy, hexyloxy, cyclopentyl, cycloheptyl, cyclooctyl, fluoro, cyano, trimethylsilyl, triethylsilyl, tripropylsilyl, tri(t-butyl)silyl, t-butyldimethylsilyl, dimethylphenylsilyl, triphenylsilyl, and the following structures, without restriction: 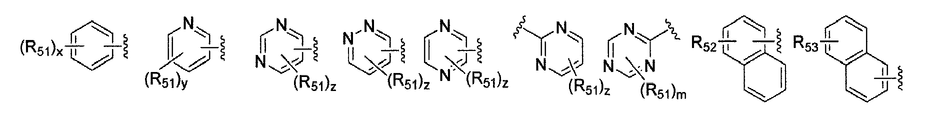 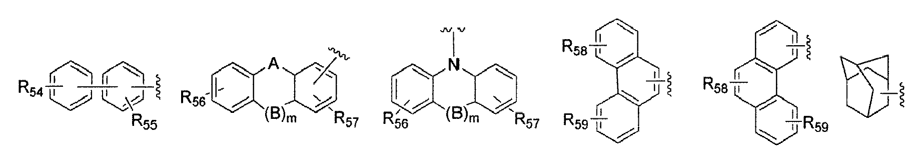 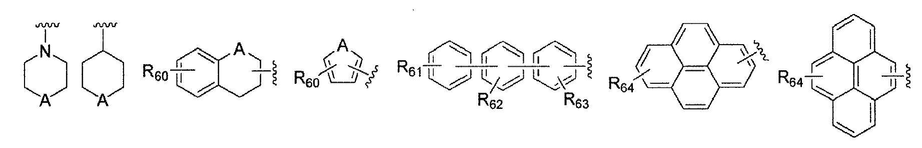 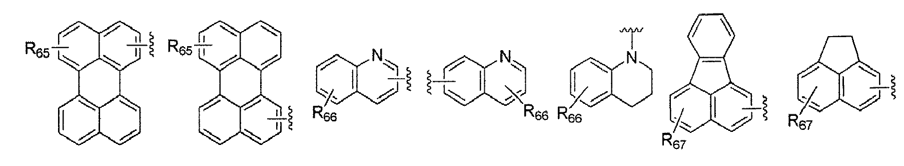 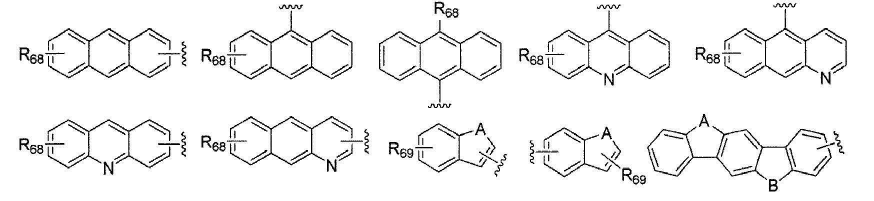 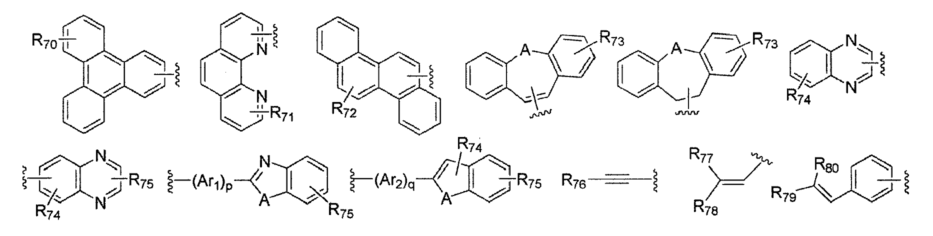 wherein, R₅₁ through R₈₀ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₅₁ through R₈₀ may be further substituted by halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
A and B independently represent a chemical bond, or they are selected from -C(R₉₁)(R₉₂)-, -N(R₉₃)-, -O-, -S-, - Si(R₉₄₎(R₉₅₎-, -P(R₉₆)-, -C(=O)- and -(R₉₇)C=C(R₉₈)-, wherein R₉₁ through R₉₈ independently represent hydrogen, (C1-C60)alkyl, (C1-C30)alkoxy, cyano, -CF₃, halogen, (C6-C60)aryl, tri(C1-C30)alkylsilyl, di(C1-C30)alkyl(C6-C30)arylsilyl or tri(C6-C30)arylsilyl; the alkyl or aryl of R₉₁ through R₉₈ may be further substituted by one or more substituent(s) selected from (C1-C60)alkyl, (C1-C30)alkoxy, halogen, cyano, tri(C1-C30)alkylsilyl, di(C1-C30)alkyl(C6-C30)arylsilyl, tri(C6-C30)arylsilyl, (C5-C60)heteroaryl and (C6-C60)aryl; or R₉₁ and R₉₂, R₉₄ and R₉₅, or R₉₇ and R₉₈ may be linked via (C3-C20)alkylene or (C3-C20)alkenylene with or without a fused ring to form an alicyclic ring, or a monocyclic or polycyclic aromatic ring;
Ar₁ and Ar₂ independently represent (C6-C60)arylene or (C5-C60)heteroarylene; the arylene or heteroarylene of Ar₁ and Ar₂ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, tri(C1-C30)alkylsilyl, di(C1-C30)alkyl(C6-C30)arylsilyl and tri(C6-C30)arylsilyl;
x is an integer from 0 to 5, y is an integer from 0 to 4, z is an integer from 0 to 3; and
m, p and q represent an integer from 0 to 2.

More specifically, R₁ through R₁₆ are independently selected from the following structures, but not restricted thereto.

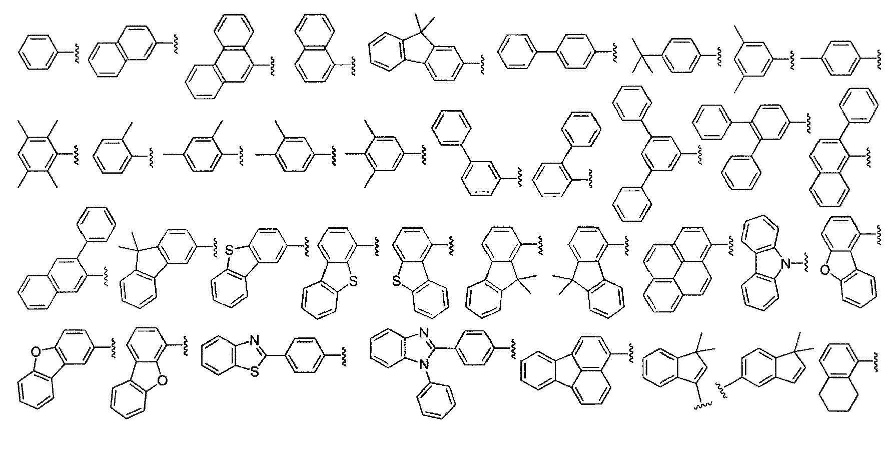

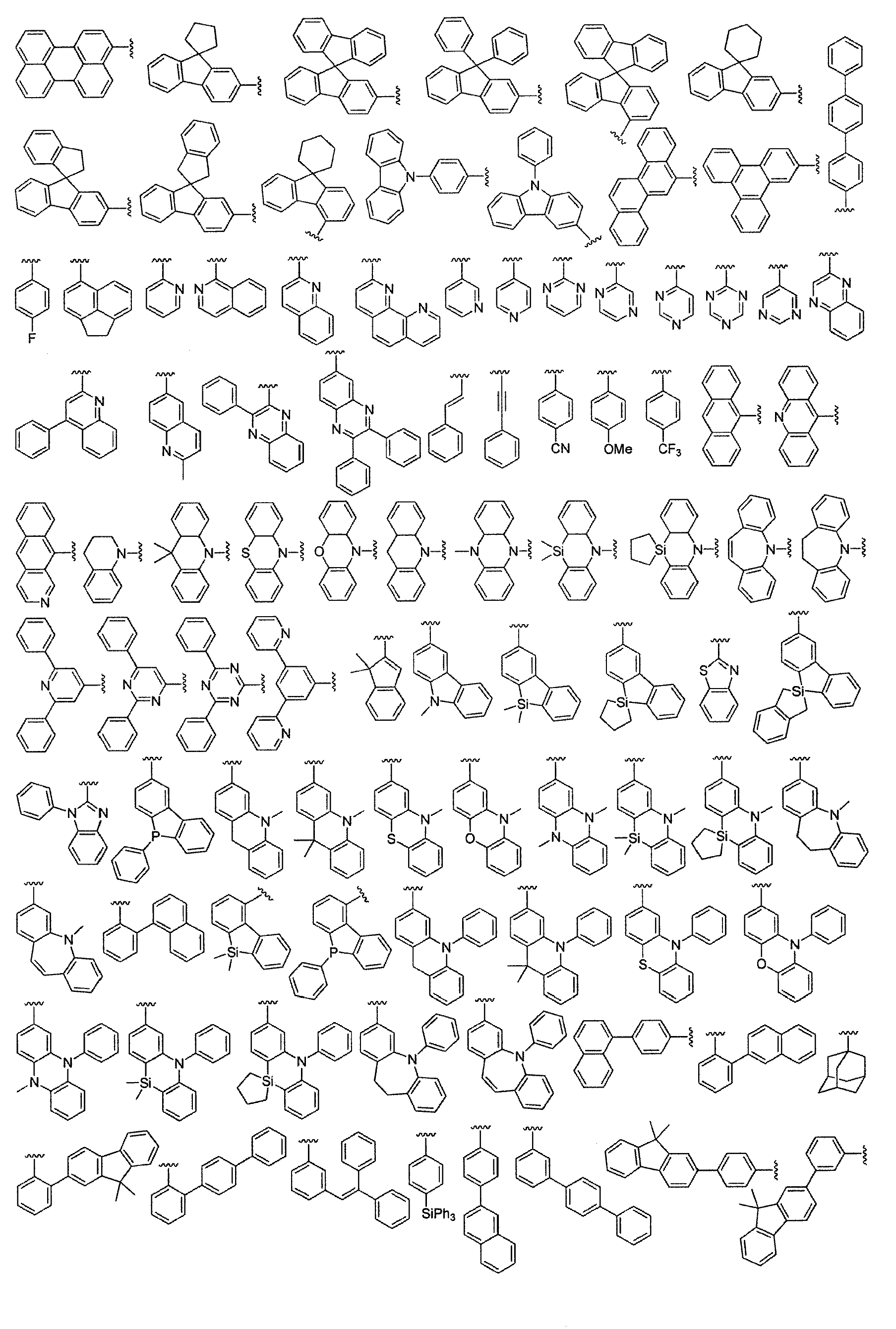

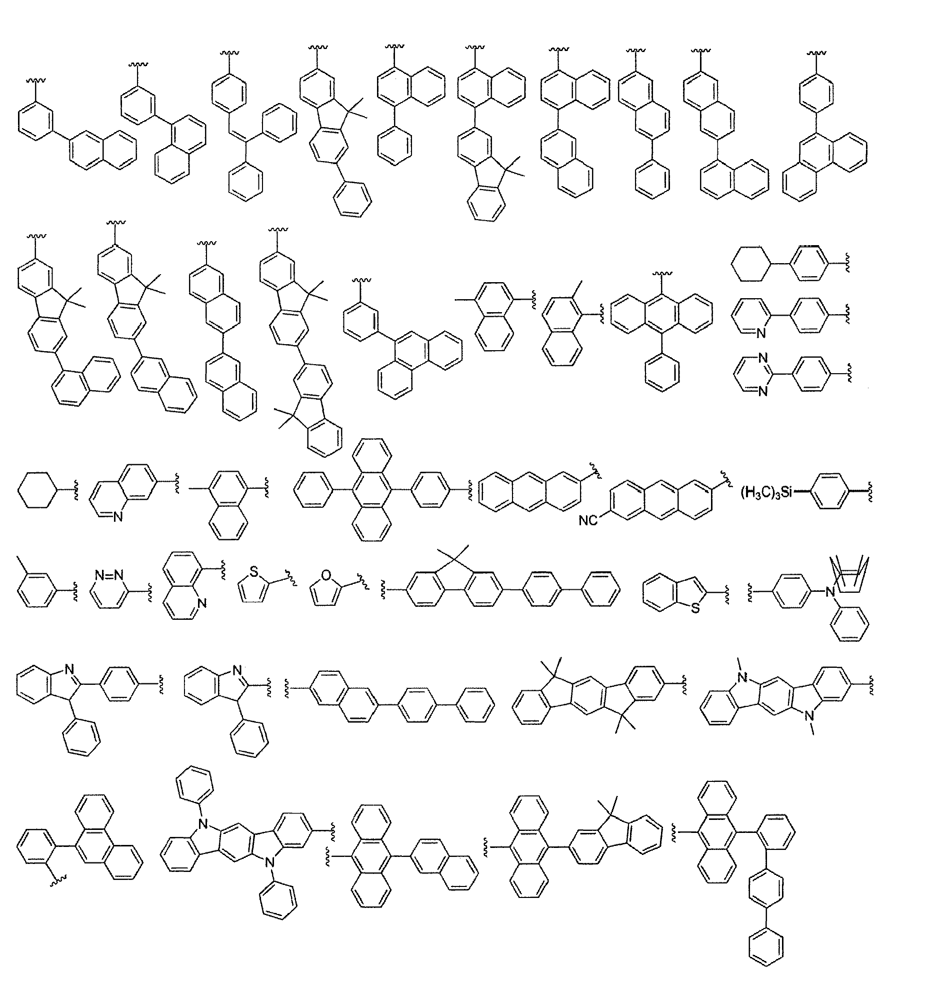

The organic electroluminescent compounds according to the present invention can be more specifically exemplified by the following compounds, but they are not restricted thereto:

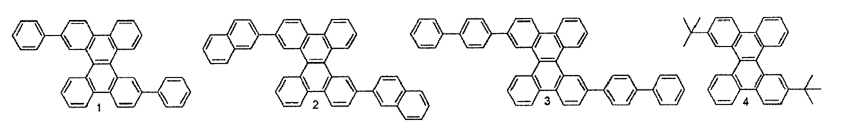

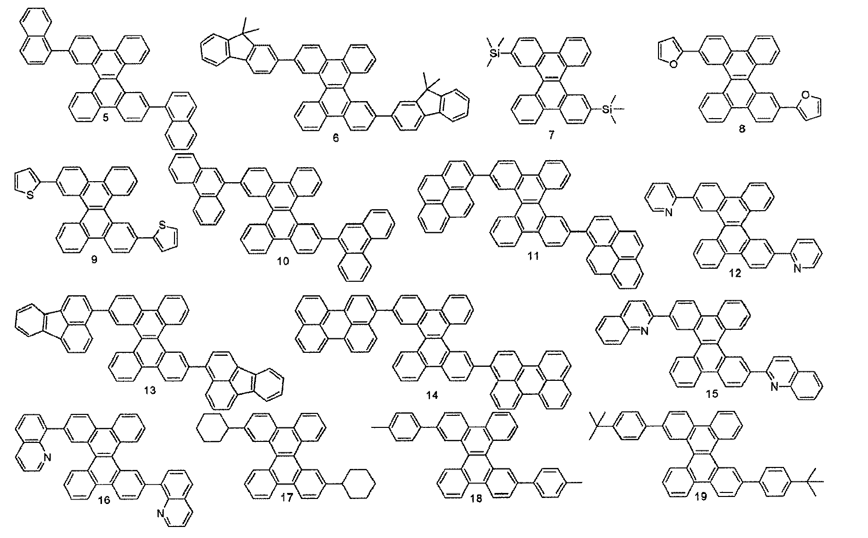

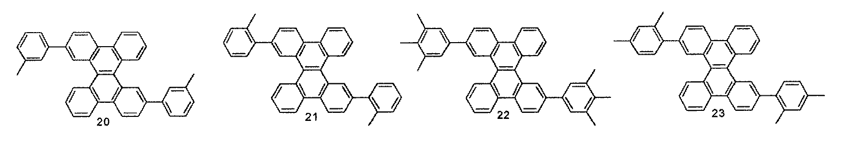

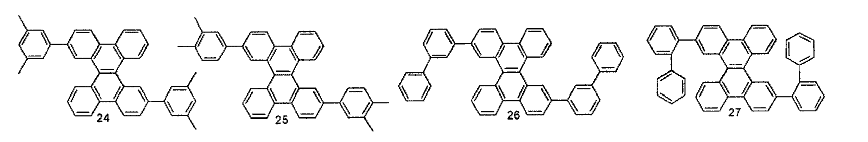

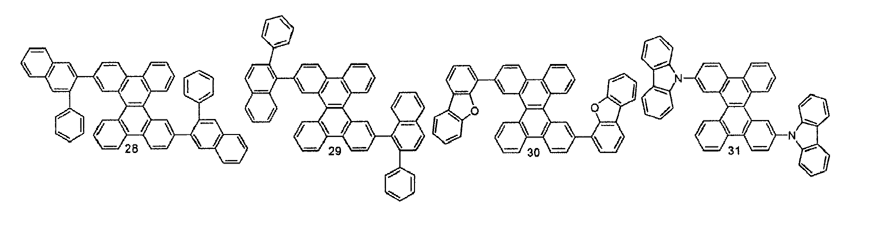

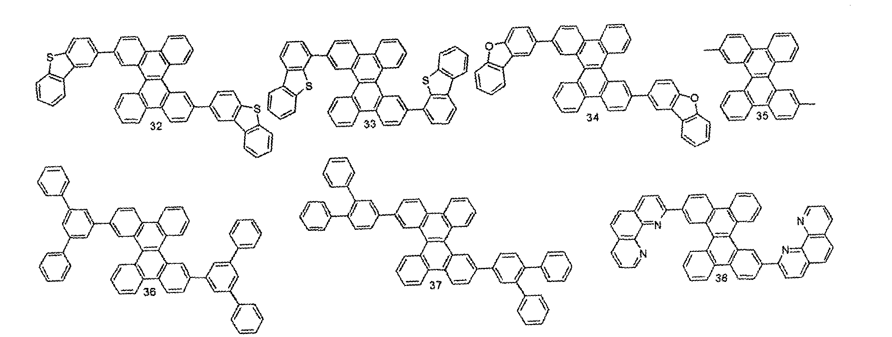

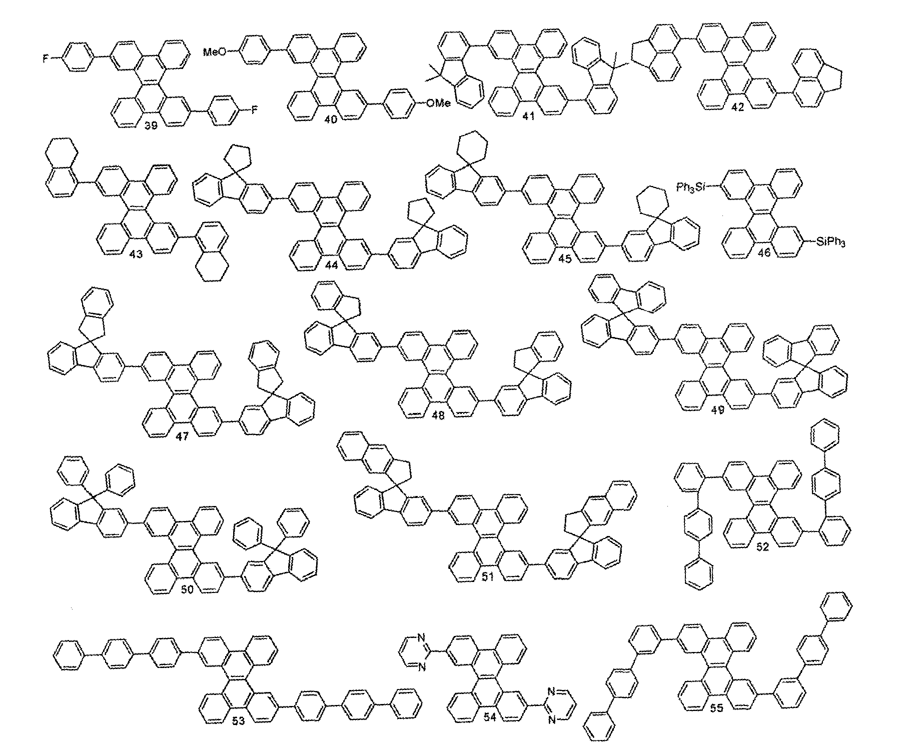

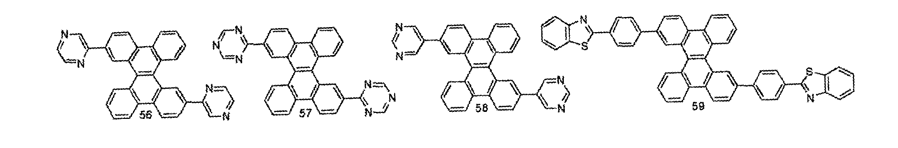

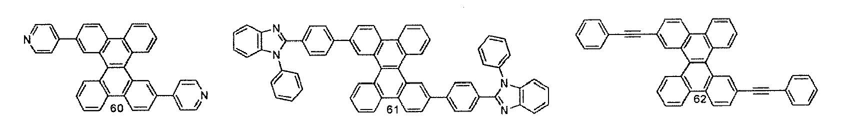

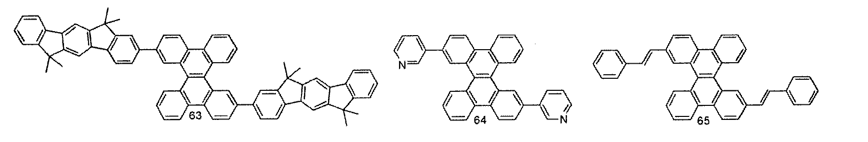

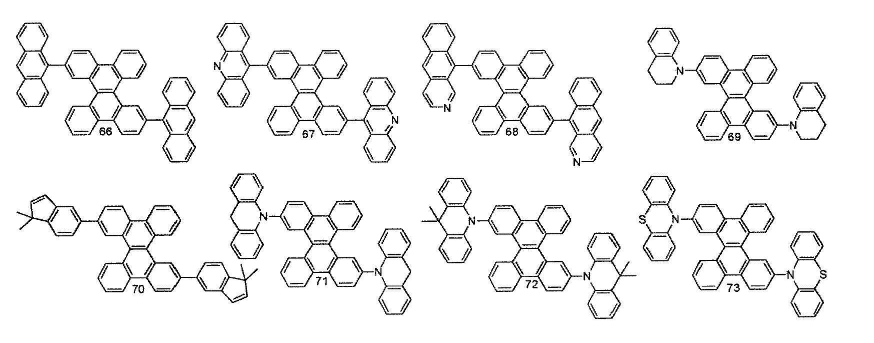

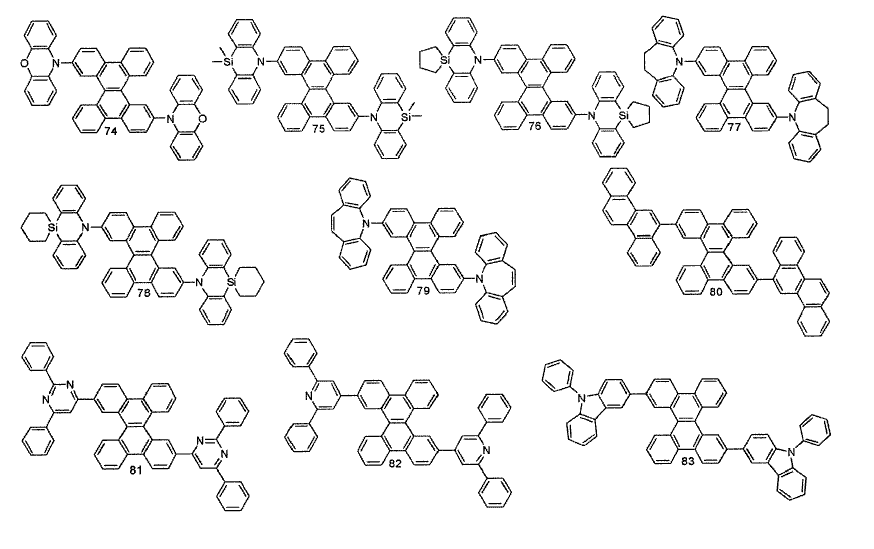

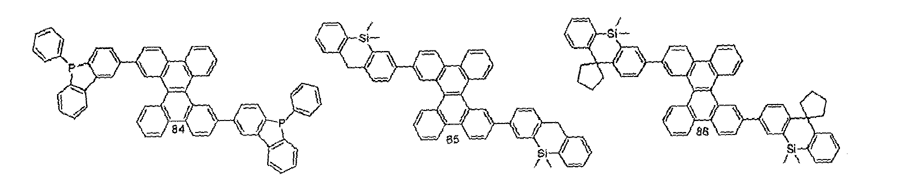

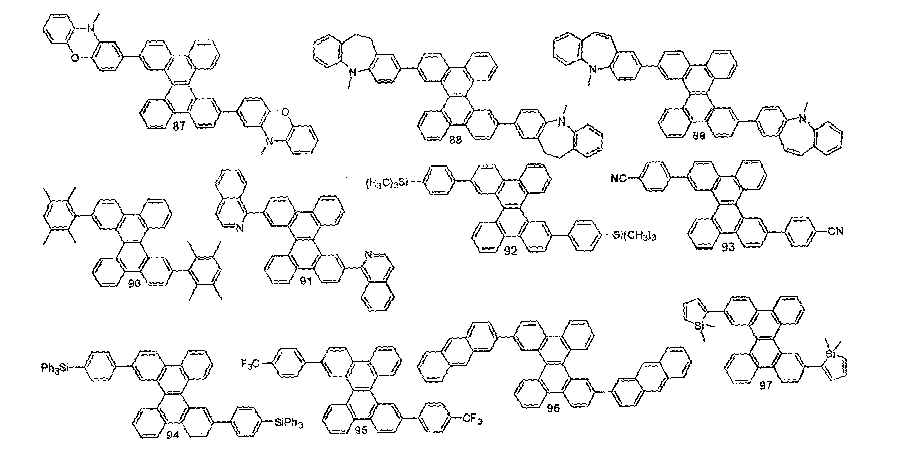

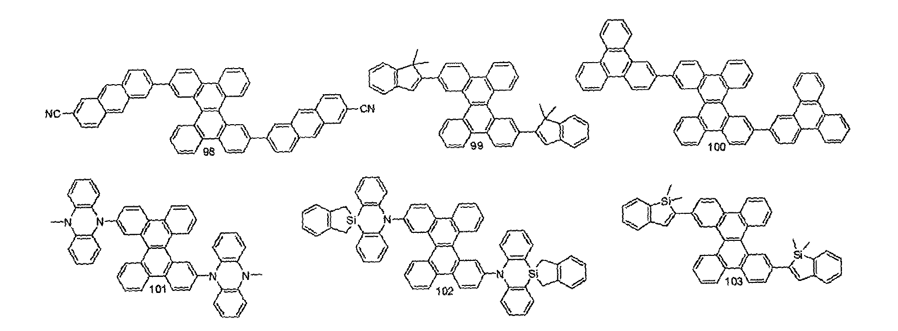

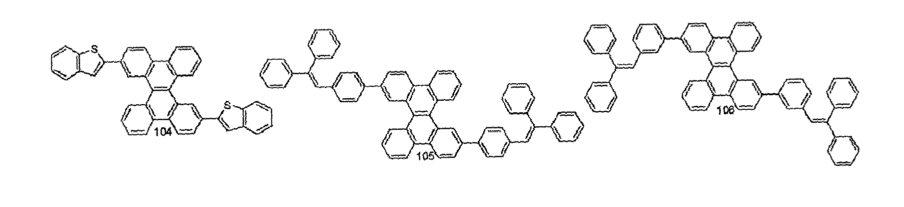

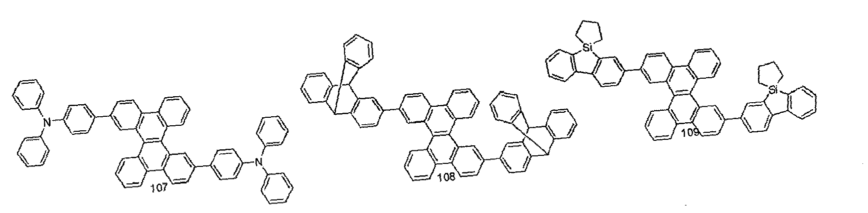

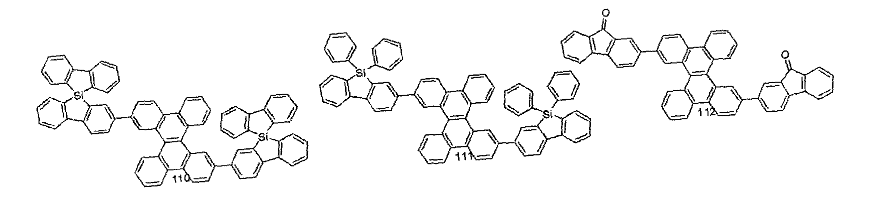

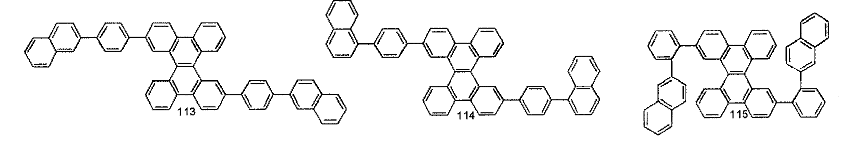

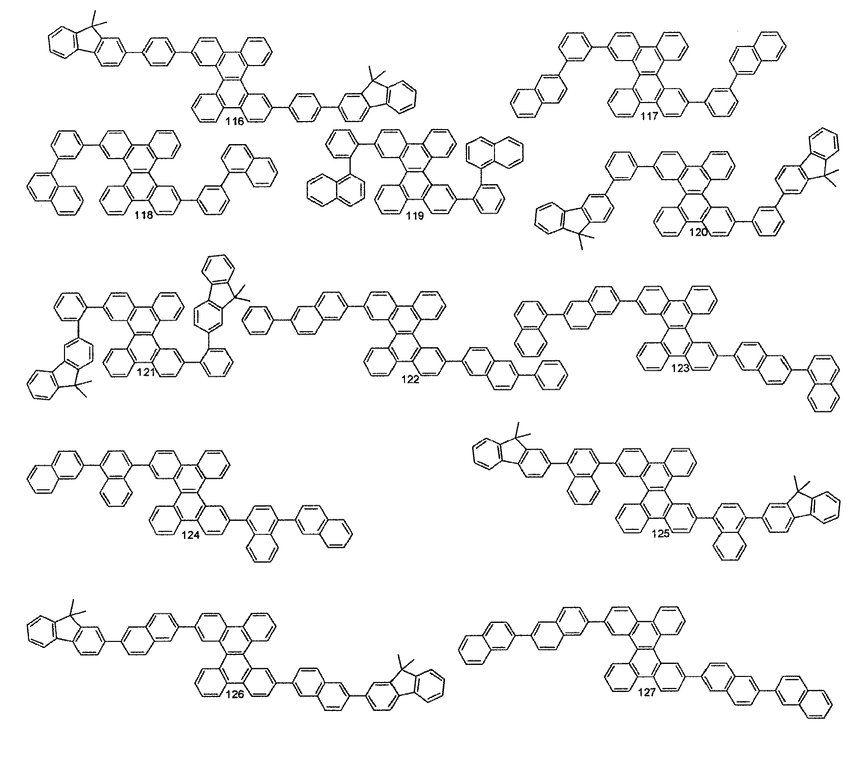

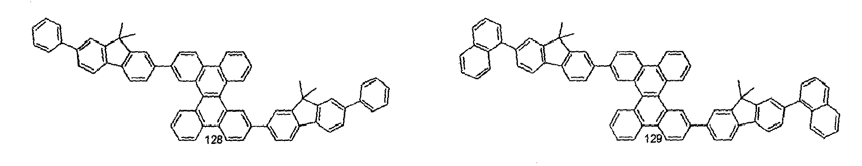

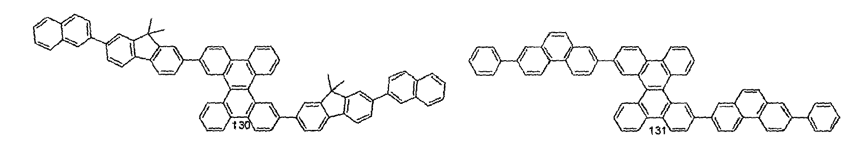

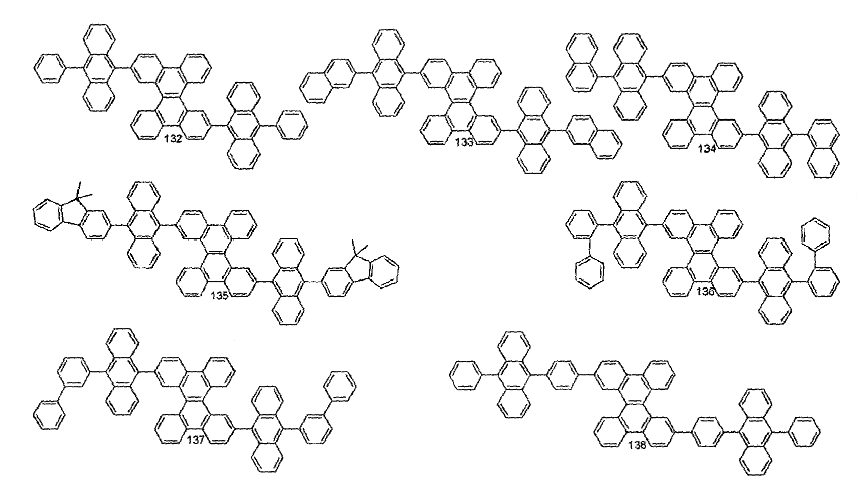

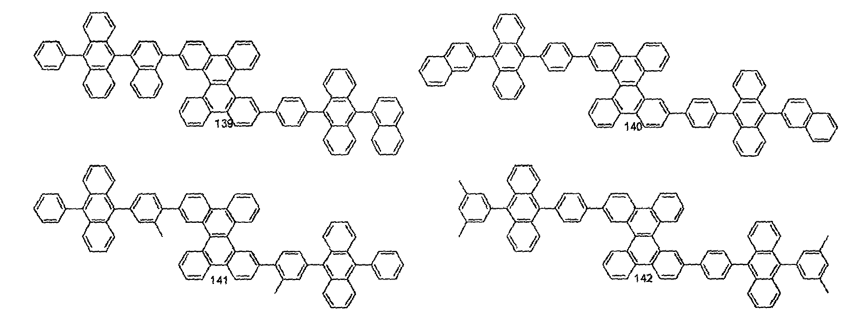

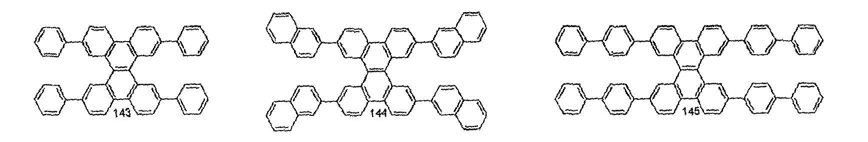

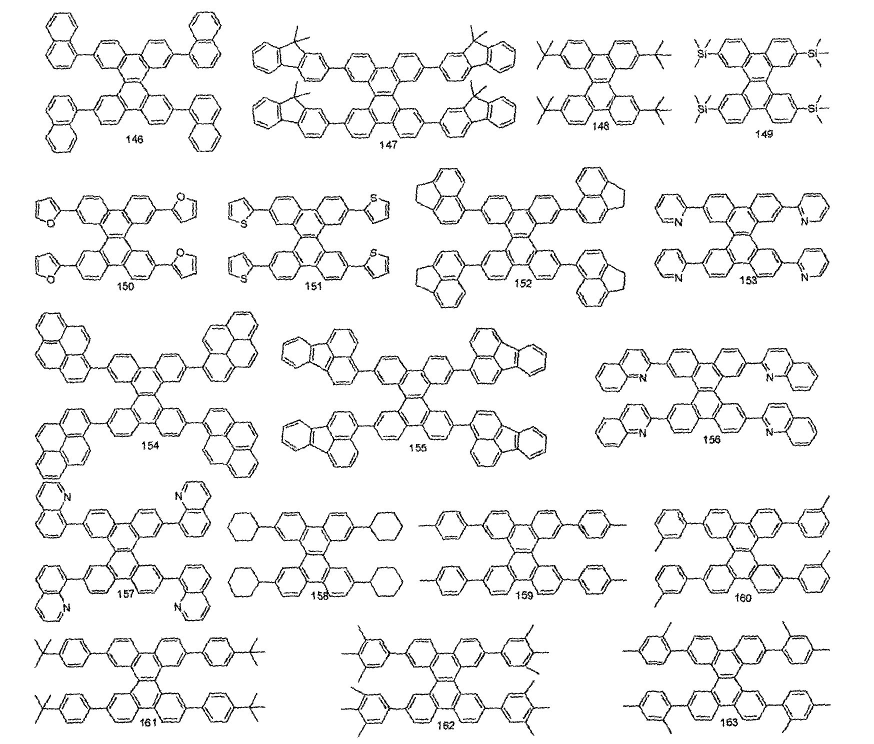

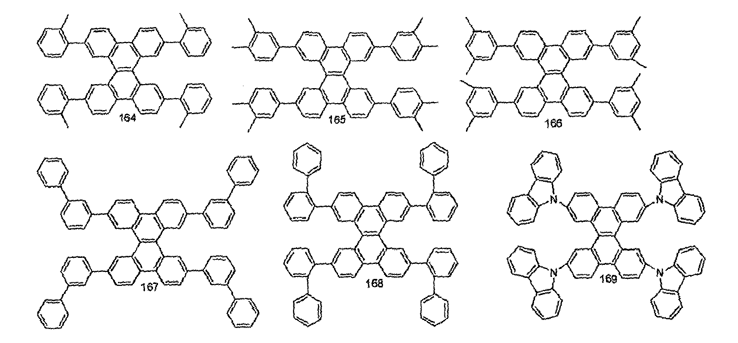

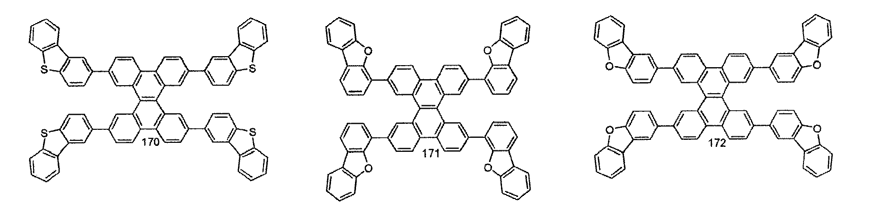

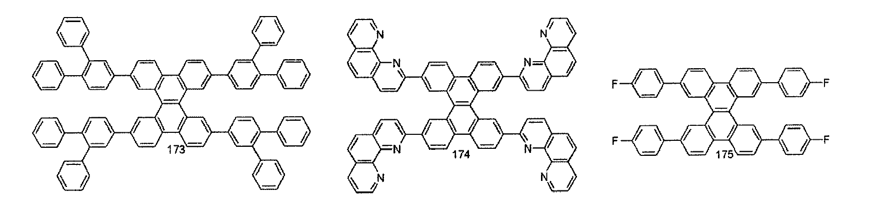

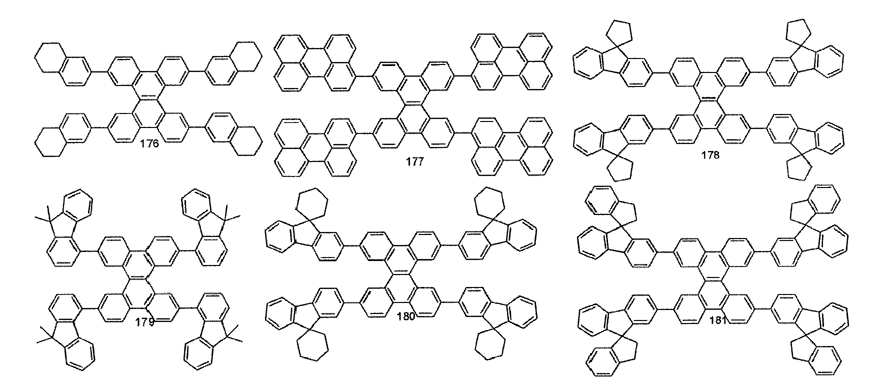

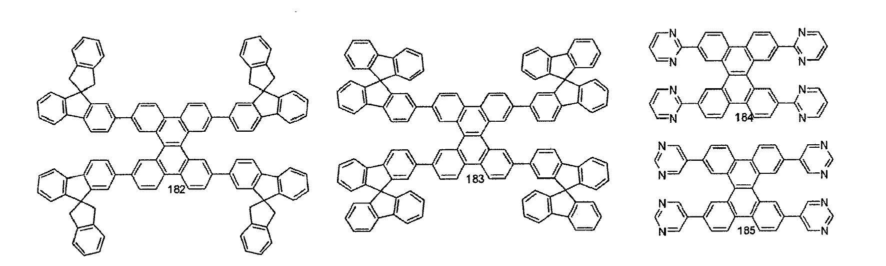

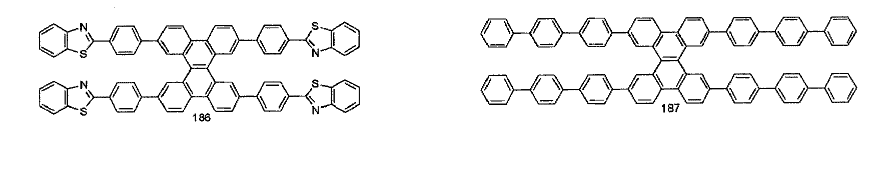

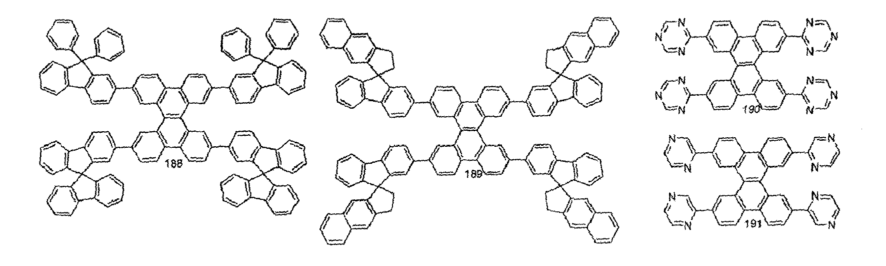

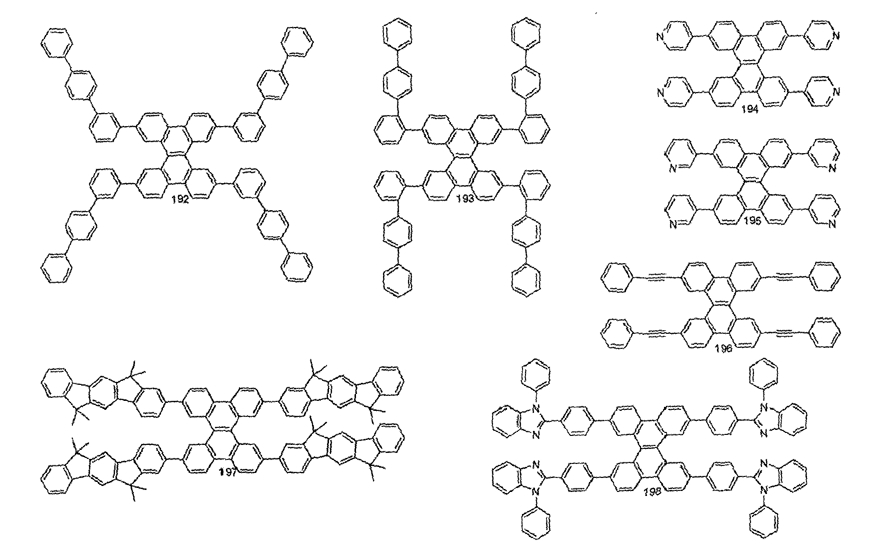

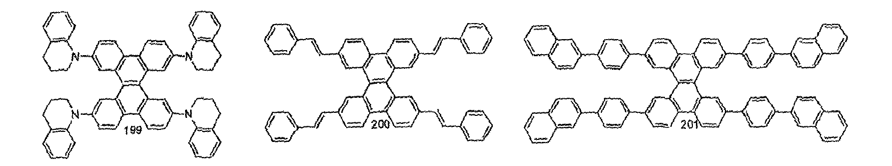

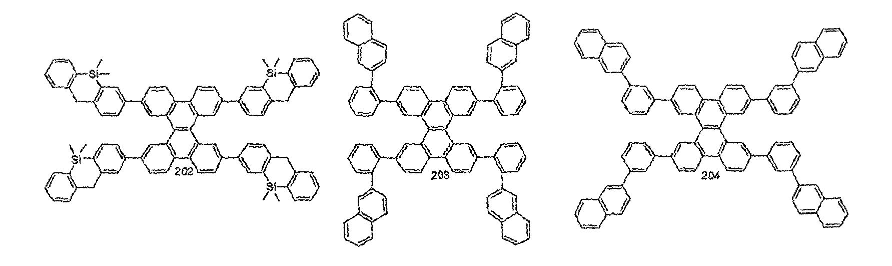

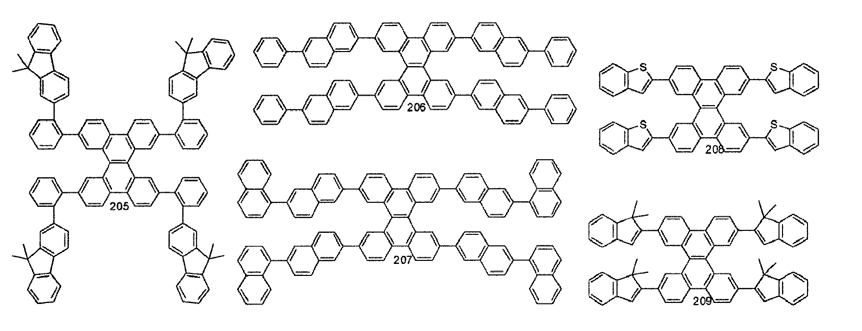

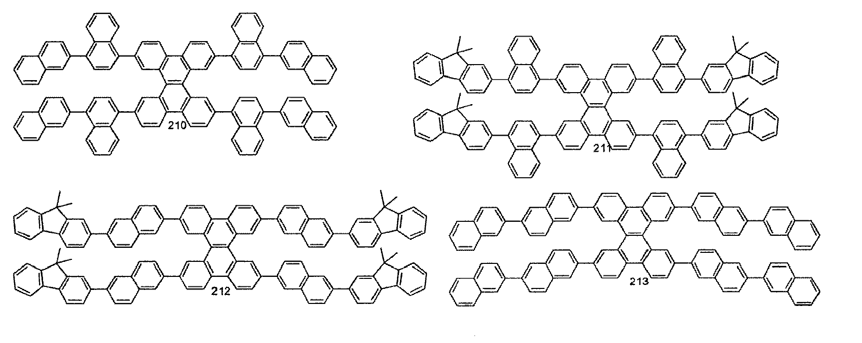

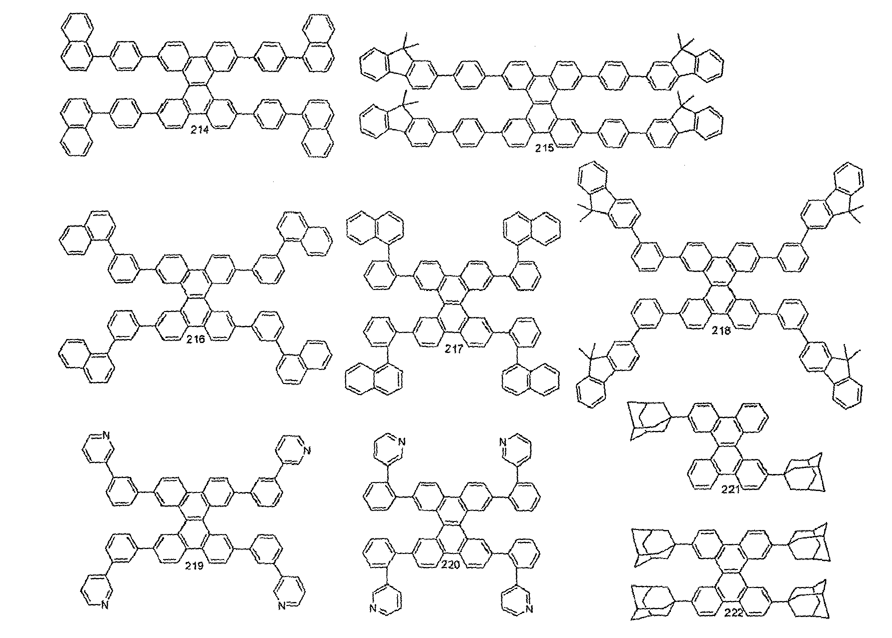

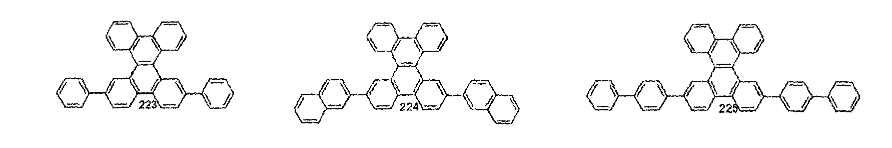

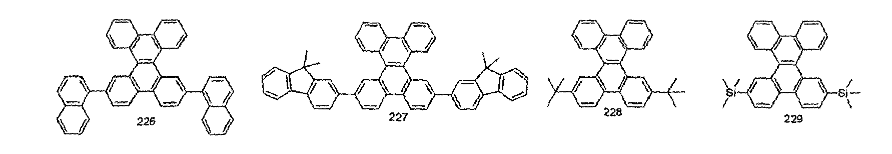

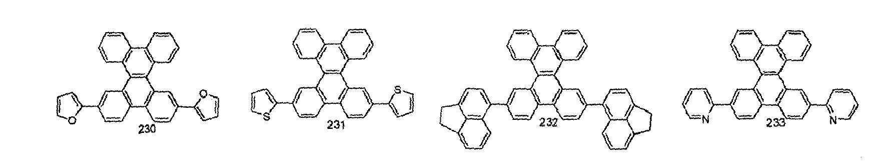

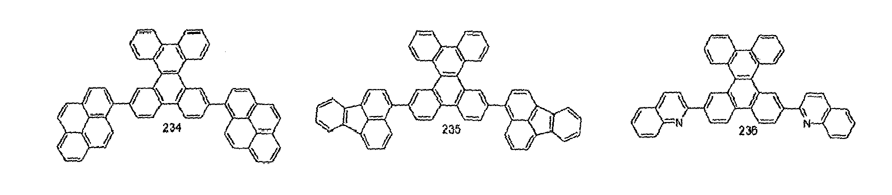

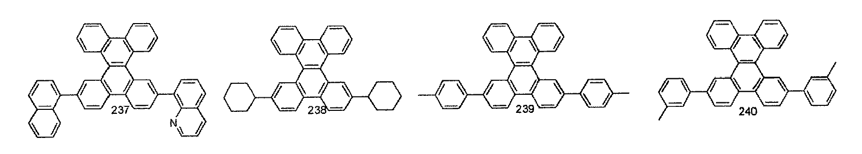

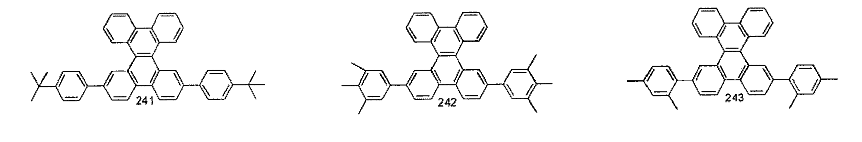

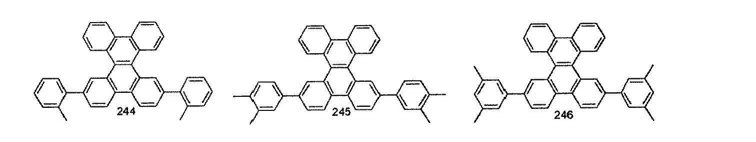

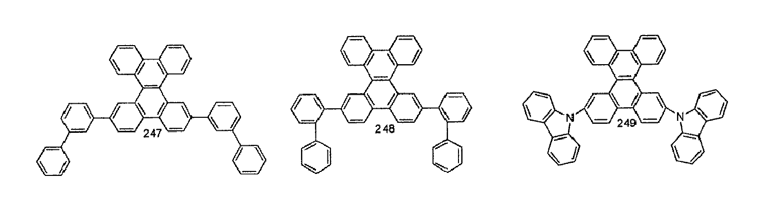

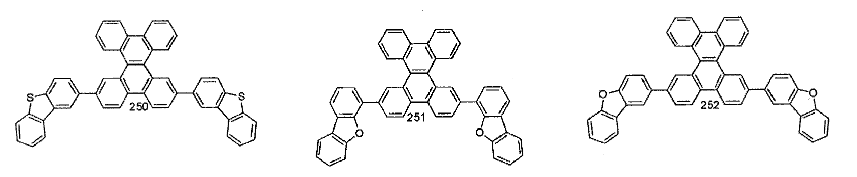

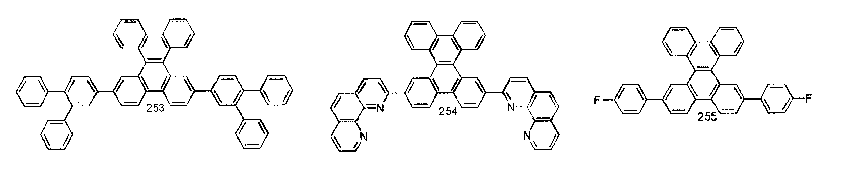

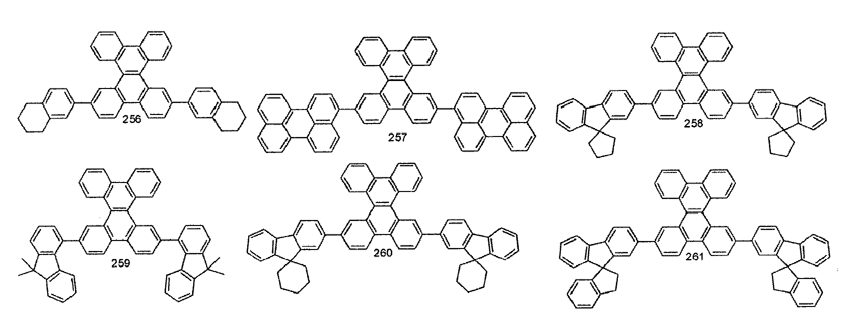

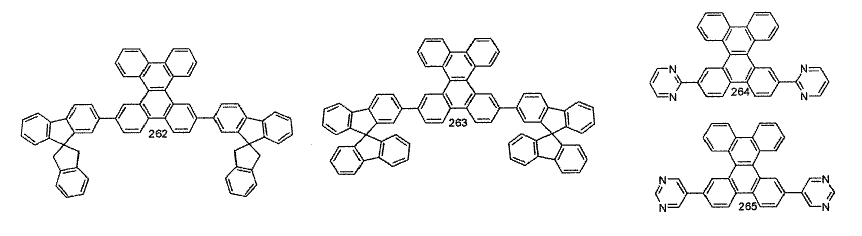

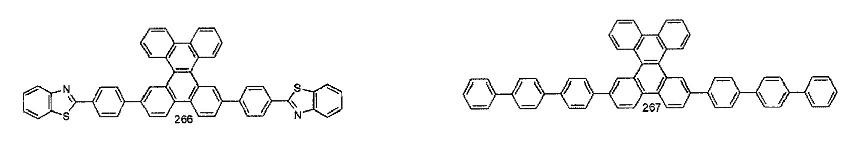

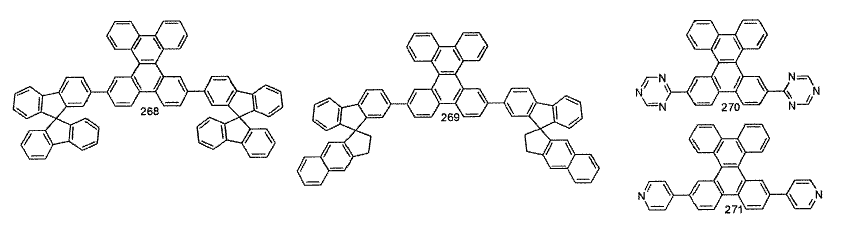

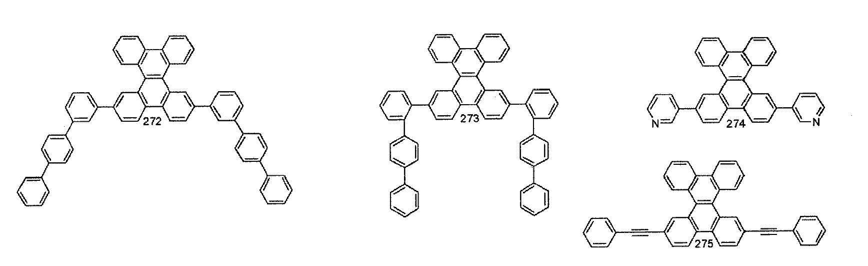

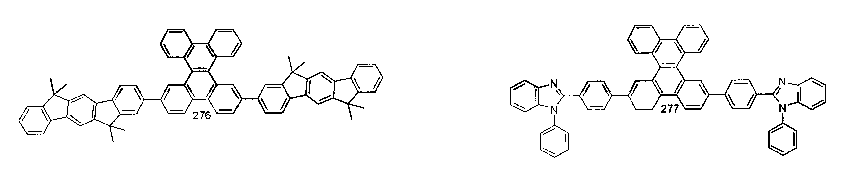

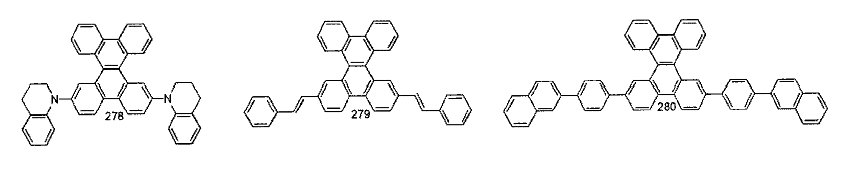

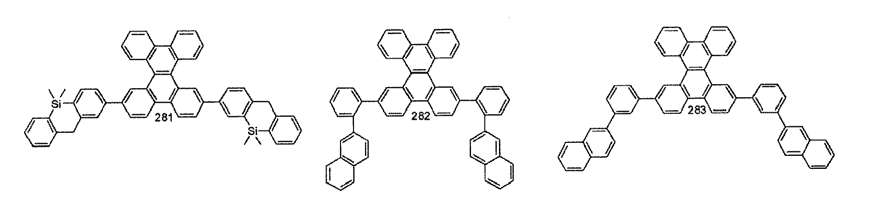

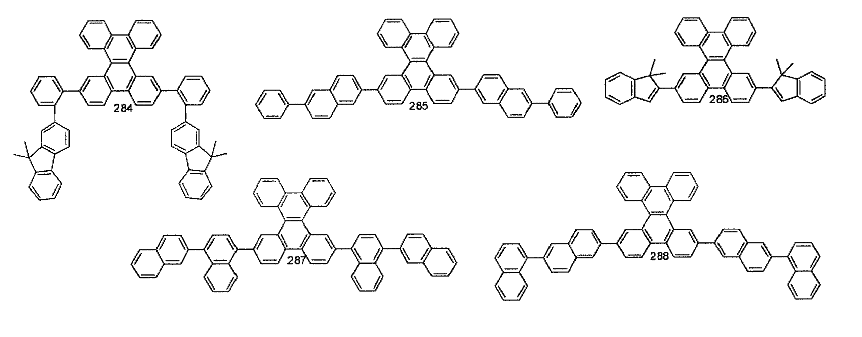

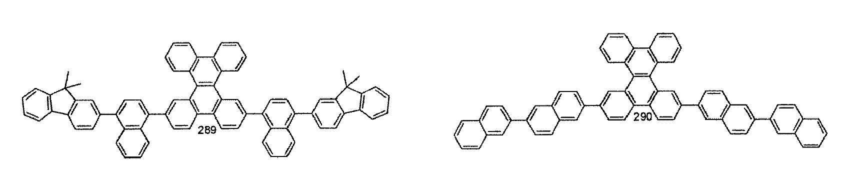

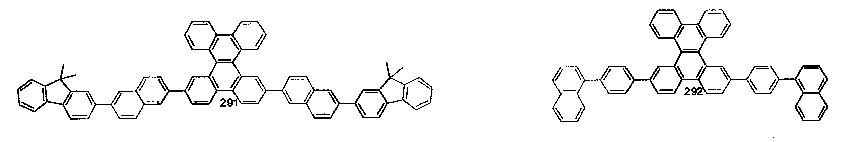

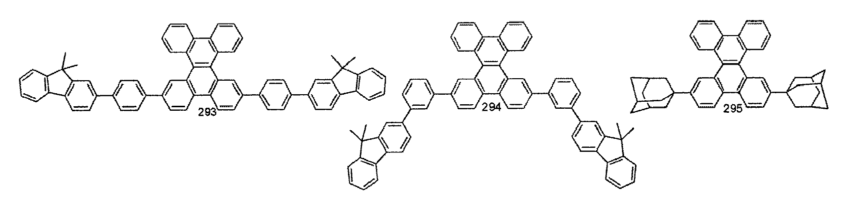

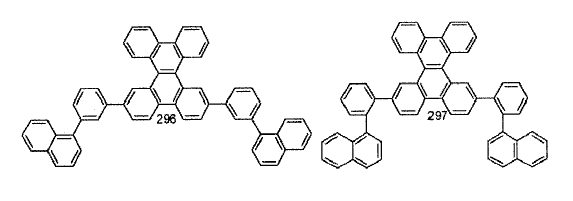

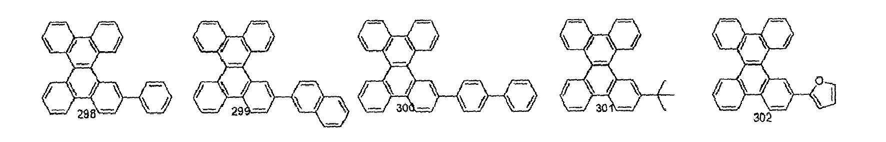

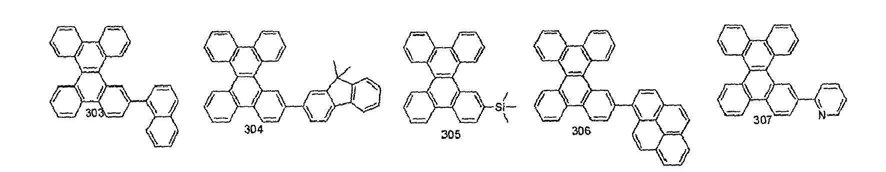

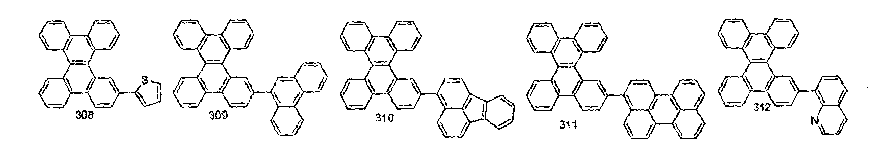

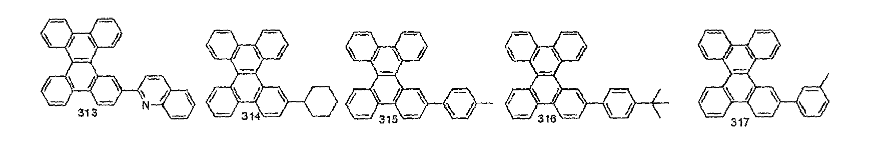

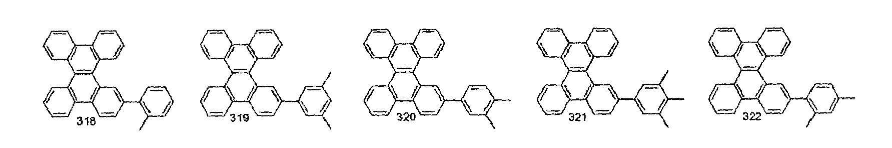

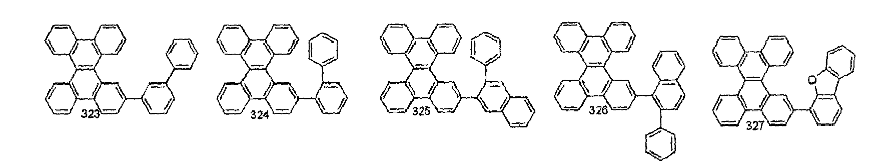

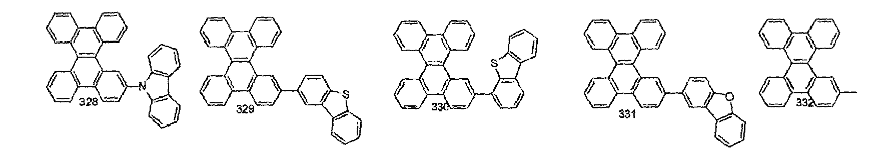

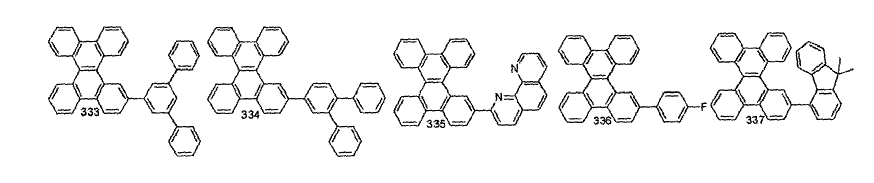

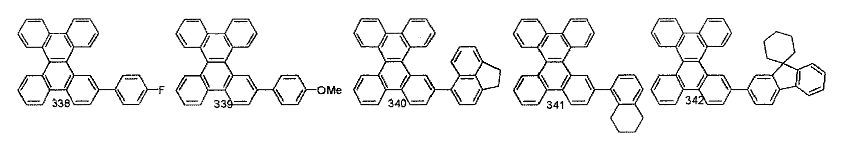

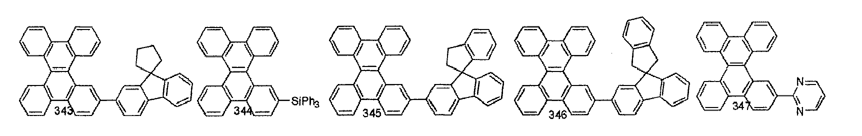

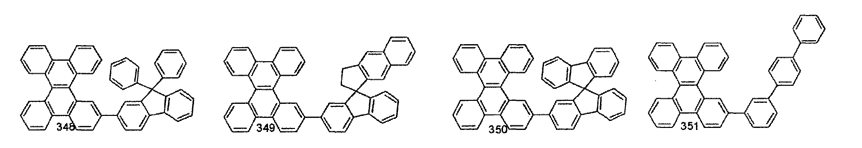

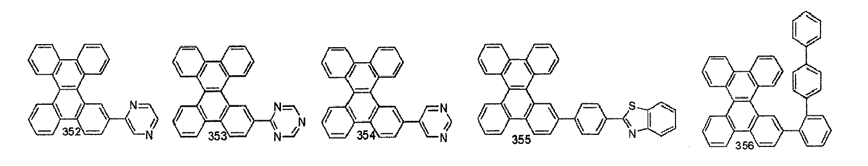

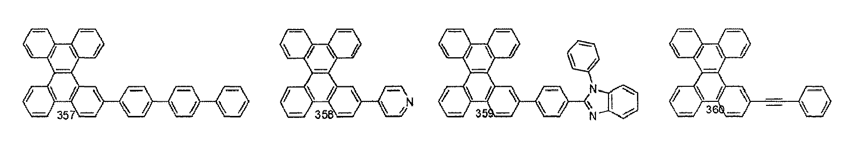

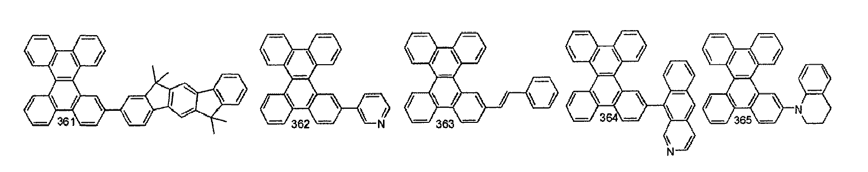

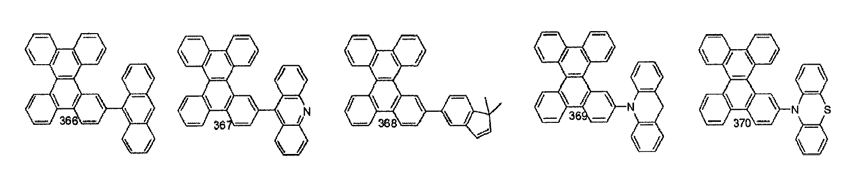

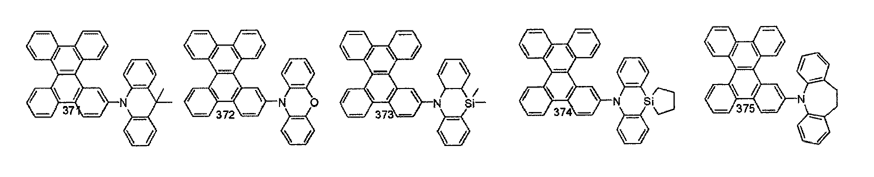

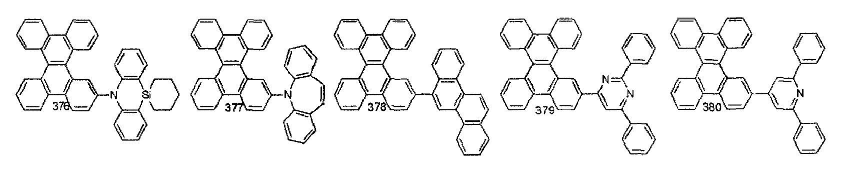

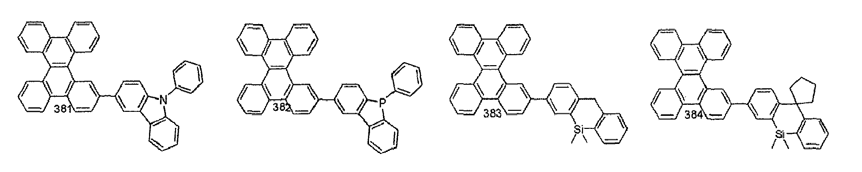

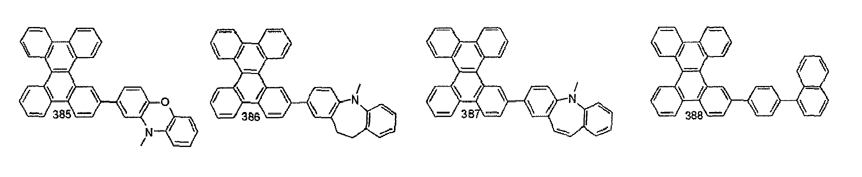

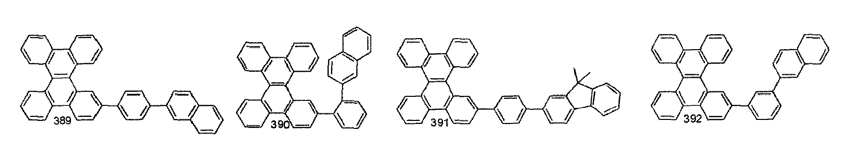

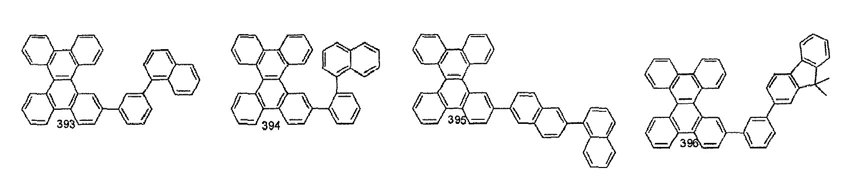

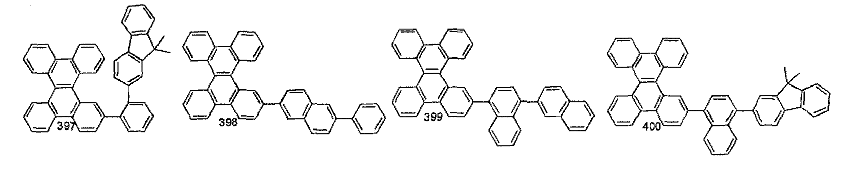

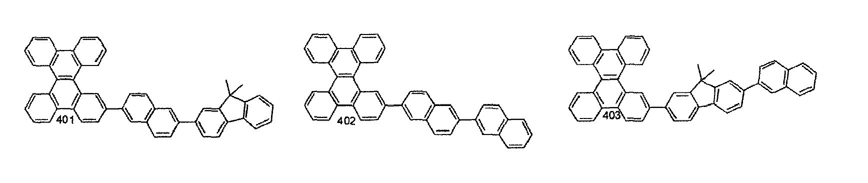

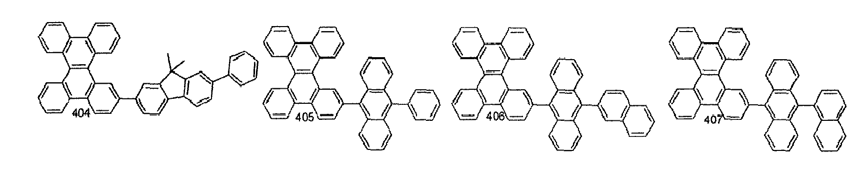

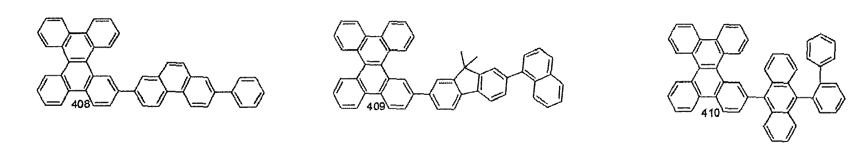

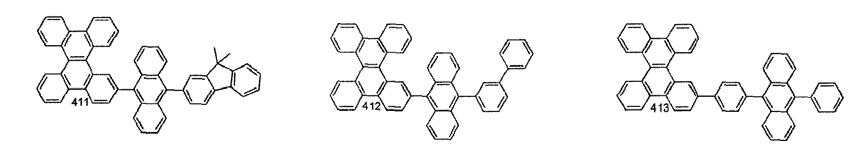

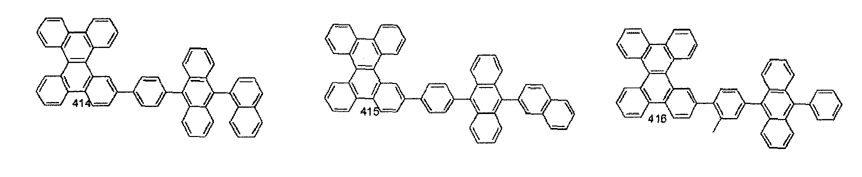

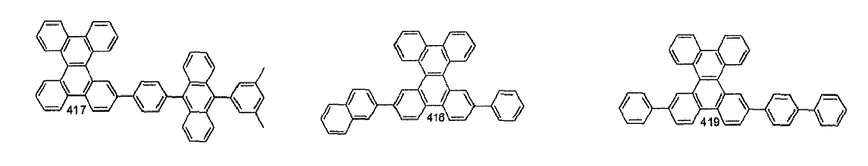

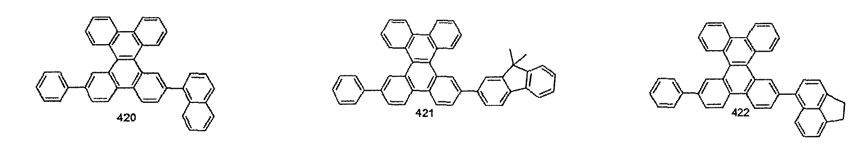

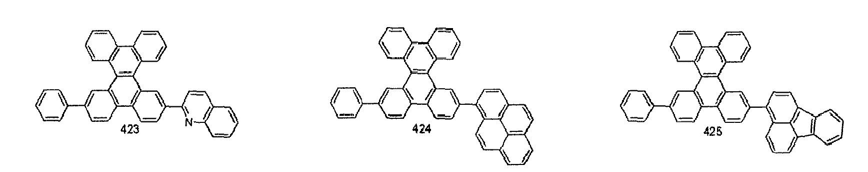

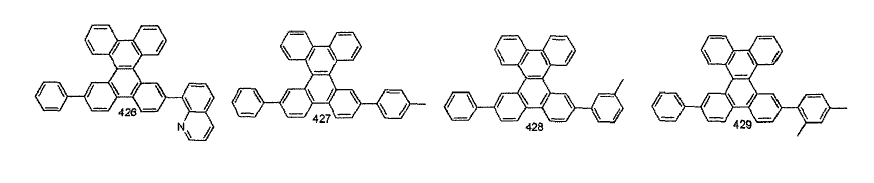

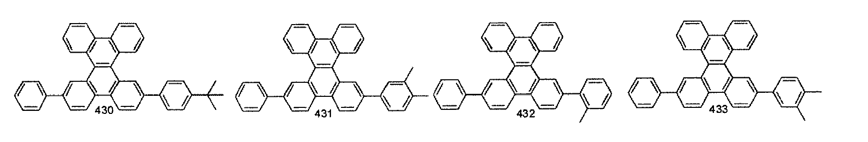

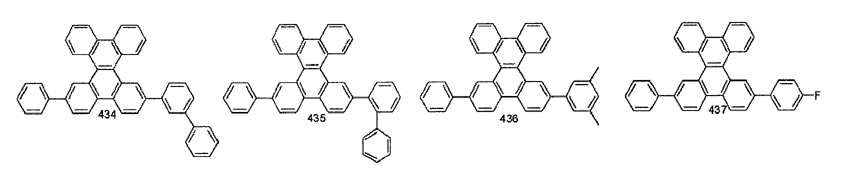

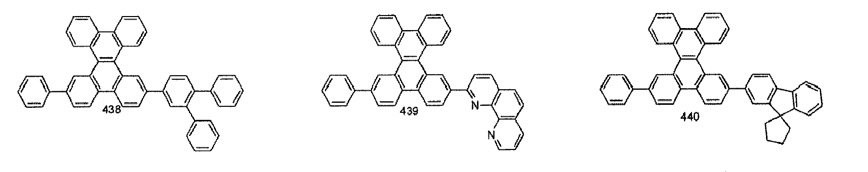

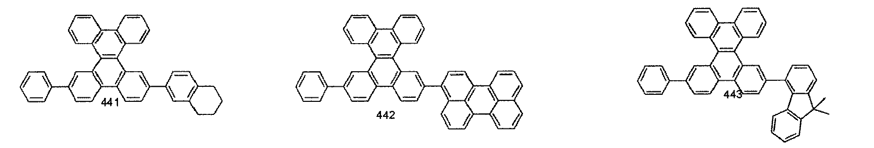

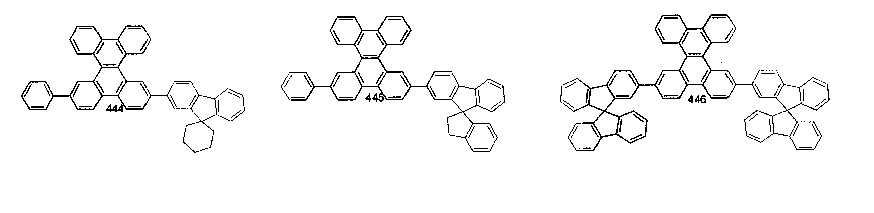

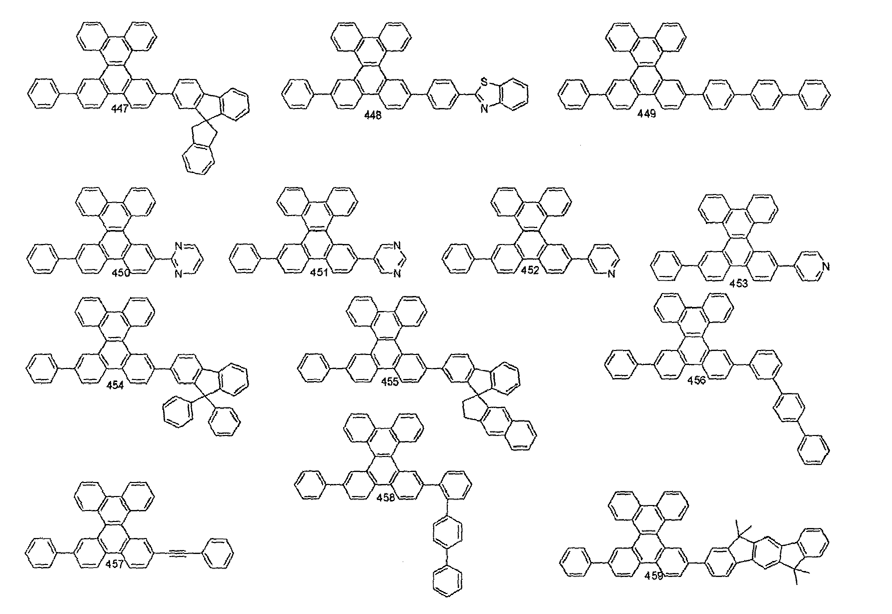

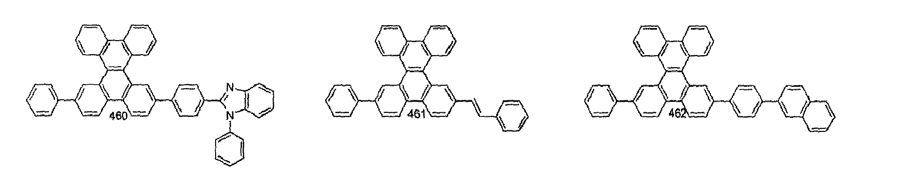

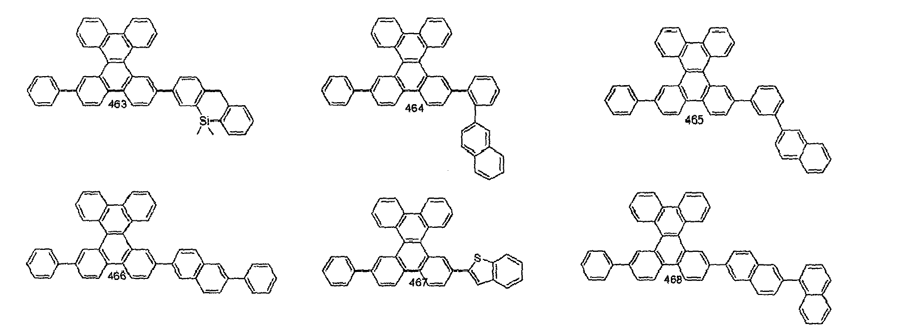

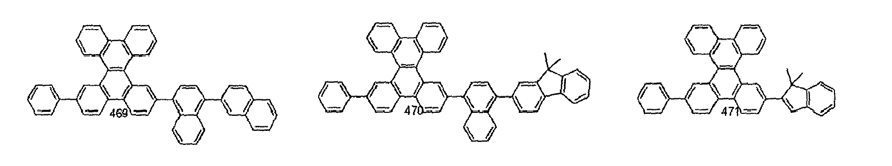

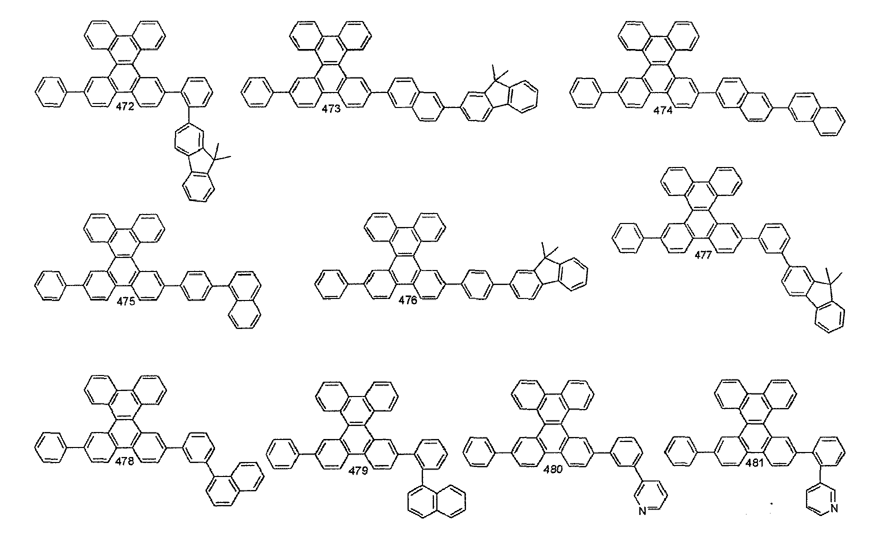

The organic electroluminescent compounds of the present invention can be prepared according to the procedures illustrated by one of Reaction Schemes (1) to (3): 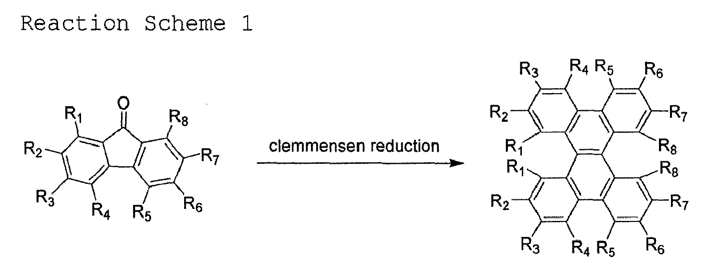 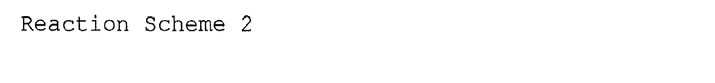 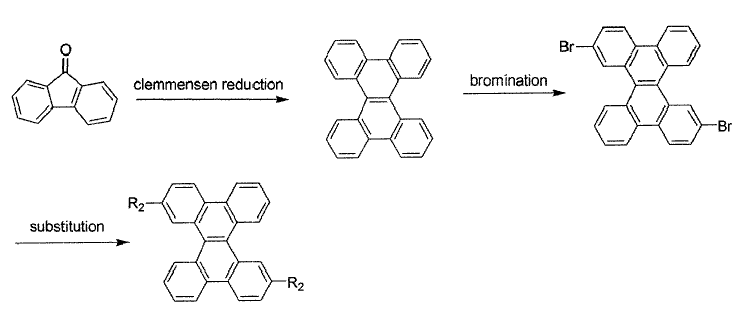 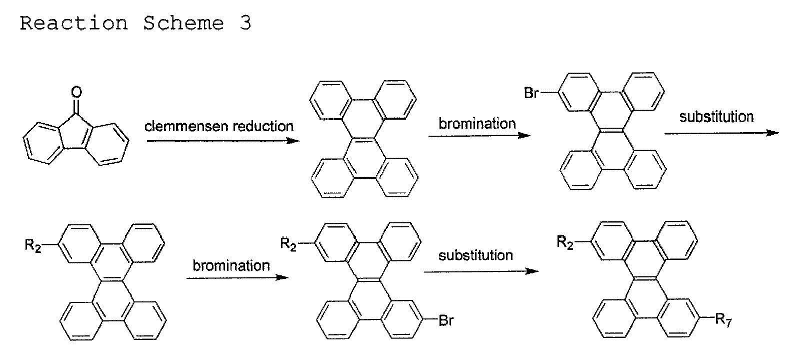 wherein, R₁ through R₈ are defined as in Chemical Formula (1).

In the bromination of Reaction Scheme (2) or (3), one or more bromine can be substituted at certain position(s) by suitably adjusting the reaction equivalent and reaction condition. For instance, brominated compounds shown below can be selectively formed.

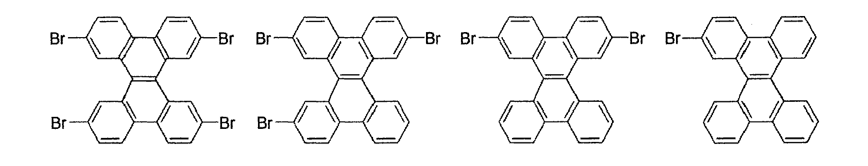

The present invention also provides organic solar cells, which comprises one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more compound(s) represented by Chemical Formula (1).

The organic electroluminescent device according to the present invention is **characterized in that** the organic layer comprises an electroluminescent layer, which comprises one or more organic electroluminescent compound(s) represented by Chemical Formula (1) as electroluminescent host, and one or more dopant(s). The dopant to be applied to the organic electroluminescent device according to the invention is not particularly restricted, but preferably selected from the compounds represented by one of Chemical Formulas (3) to (5).

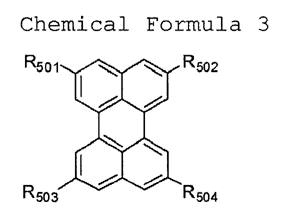

In Chemical Formula (3), R₅₀₁ through R₅₀₄ independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; or each of R₅₀₁ through R₅₀₄ may be linked to an adjacent substituent via (C3-C60)alkylene or (C3-C60)alkenylene with or without fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino, arylamino of R₅₀₁ through R₅₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

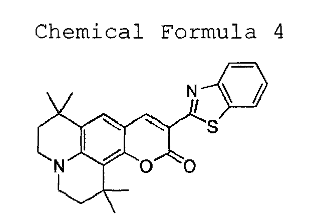

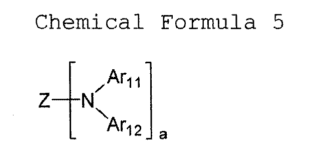

In Chemical Formula (5), Ar₁₁ and Ar₁₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or Ar₁₁ and Ar₁₂ may be linked via (C3-C60)alkylene or (C3-C60)alkenylene with or without a fused ring to form an alicyclic ring, or a monocyclic or polycyclic aromatic ring, or they are selected from the arylene groups having one of the structures shown below:

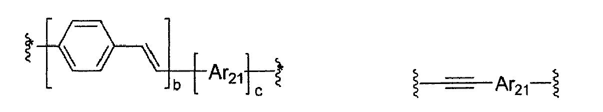

Z represents (C6-C60)arylene, (C4-C60)heteroarylene or arylene having one of the structures shown below: 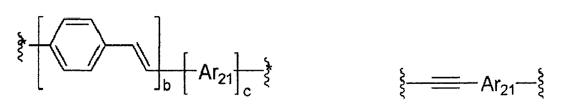 wherein, Ar₂₁ represents (C6-C60)arylene or (C4-C60)heteroarylene;
a is an integer from 1 to 4, b is an integer from 1 to 4, and c is an integer of 0 or 1; and
the alkyl, aryl, heteroaryl, arylamino, alkylamino, cycloalkyl or heterocycloalkyl of Ar₁₁ and Ar₁₂; or the arylene or heteroarylene of Z and Ar₂₁ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The electroluminescent layer means the layer where electroluminescence occurs, and it may be a single layer or a multi-layer consisting of two or more layers laminated. When a mixture of host-dopant is used according to the constitution of the present invention, noticeable improvement in luminous efficiency by the electroluminescent host according to the invention may be confirmed. Those results can be achieved by doping concentration of 0.5 to 20% by weight. The host according to the present invention exhibits higher hole and electron conductivity, and excellent stability of the material as compared to other conventional host materials, and provides improved device life as well as luminous efficiency.

Thus, it can be described that use of the compound represented by one of Chemical Formulas (3) to (5) as an electroluminescent dopant significantly supplements electronic drawback of the organic electroluminescent compounds of Chemical Formula (1) according to the present invention.

The dopant compounds represented by one of Chemical Formulas (3) to (5) can be exemplified by the following compounds, but are not restricted thereto.

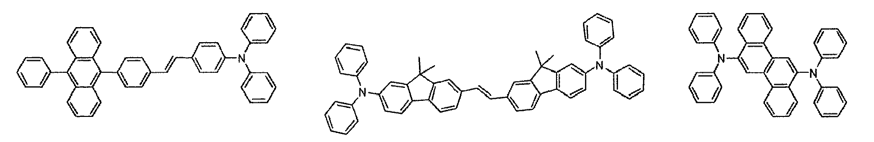

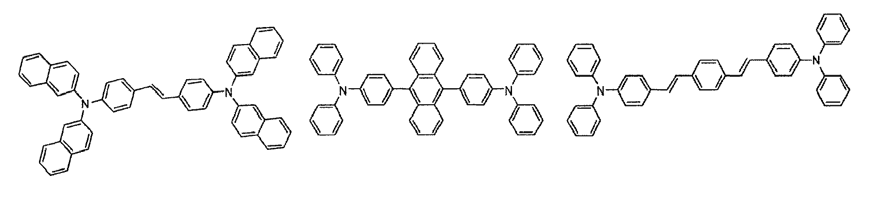

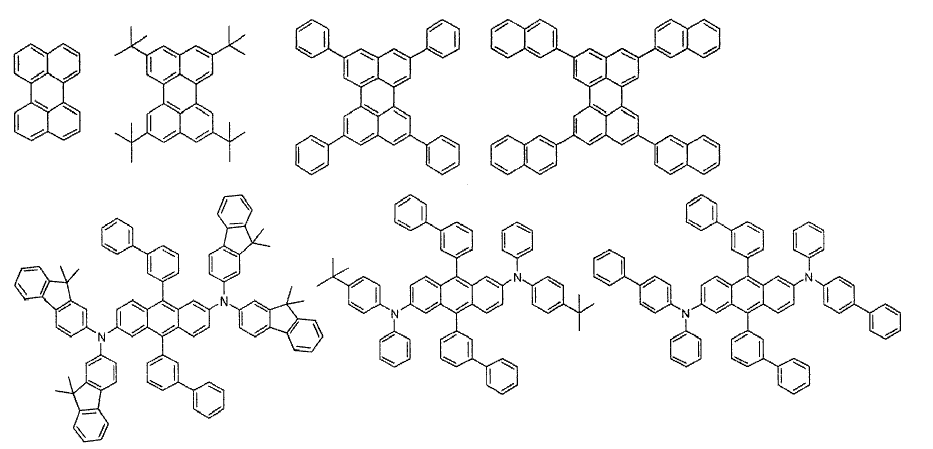

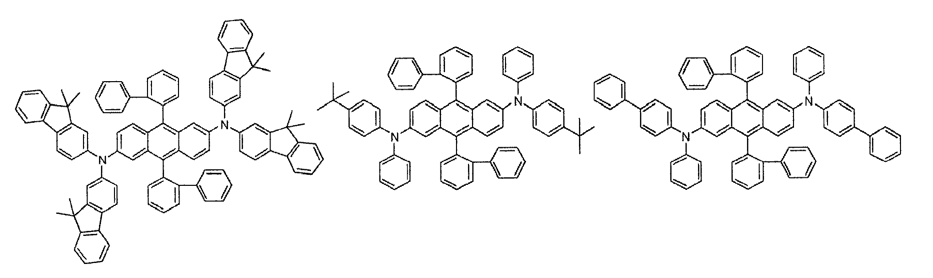

The organic electroluminescent device according to the invention may further comprise one or more compound(s) selected from a group consisting of arylamine compounds and styrylarylamine compounds, as well as the organic electroluminescent compound represented by Chemical Formula (1). Examples of the arylamine or styrylarylamine compounds include the compounds represented by Chemical Formula (6), but they are not restricted thereto: 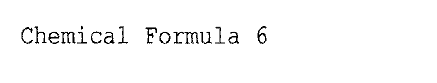 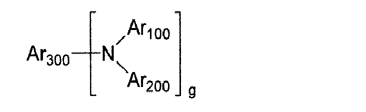 wherein, Ar₁₀₀ and Ar₂₀₀ independently represent (C1-C60)alkyl, (C6-C60)aryl, (C4-C60)heteroaryl, (C6-C60)arylamino, (C1-C60)alkylamino, morpholino, thiomorpholino, 5- or 6-membered heterocycloalkyl containing one or more heteroatom(s) selected from N, O and S, or (C3-C60)cycloalkyl, or Ar₁₀₀ and Ar₂₀₀ may be linked via (C3-C60)alkylene or (C3-C60)alkenylene with or without a fused ring to form an alicyclic ring, or a monocyclic or polycyclic aromatic ring; the aryl, heteroaryl, arylamino or heterocycloalkyl of Ar₁₀₀ and Ar₂₀₀ may be further substituted by one or more substituent(s) selected from halogen, (C1-C60)alkyl, (C2-C60)alkenyl, (C2-C60)alkynyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
Ar₃₀₀ represents (C6-C60)aryl, (C5-C60)heteroaryl or (C6-C60)arylamino; the aryl, heteroaryl or arylamino of Y may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and
g is an integer from 1 to 4.

The arylamine compounds and styrylarylamine compounds can be more specifically exemplified by the following compounds, but are not restricted thereto.

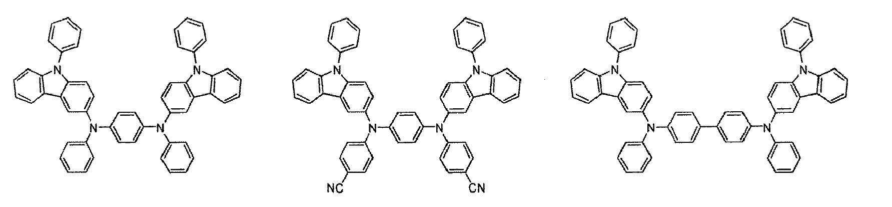

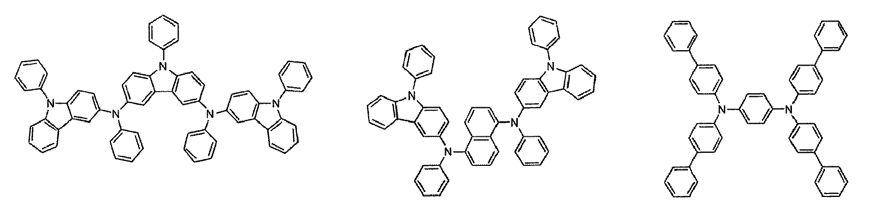

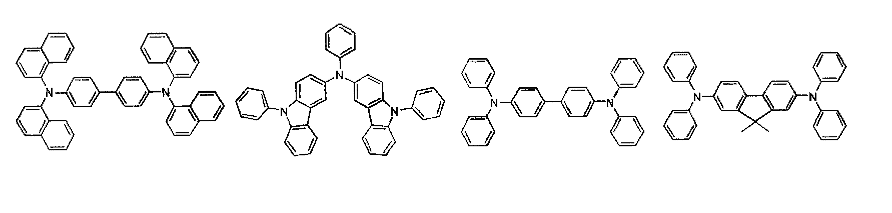

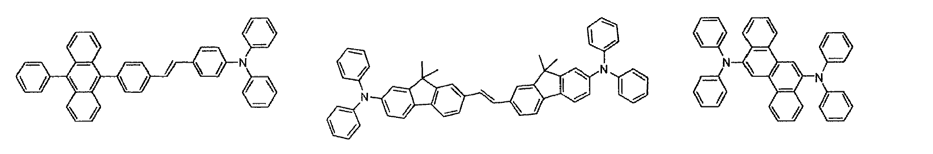

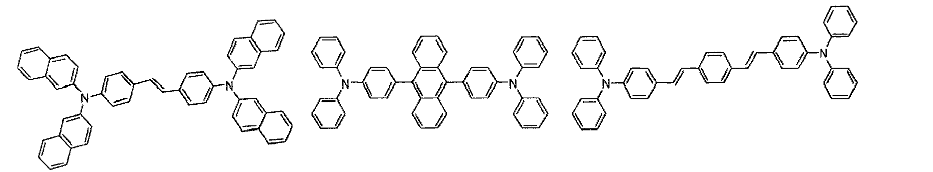

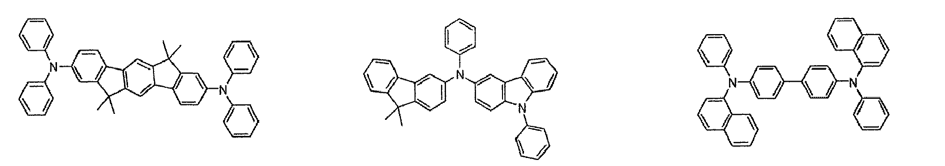

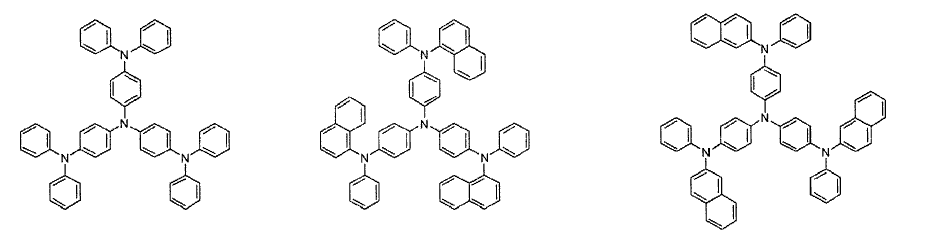

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements in the Periodic Table of Elements, as well as the organic electroluminescent compound represented by Chemical Formula (1). The organic layer may comprise a charge generating layer in addition to the electroluminescent layer.

The present invention can realize an organic electroluminescent device having a pixel structure of independent light-emitting mode, which comprises an organic electroluminescent device containing the compound of Chemical Formula (1) as a sub-pixel and one or more sub-pixel(s) comprising one or more metallic compound(s) selected from a group consisting of Ir, Pt, Pd, Rh, Re, Os, Tl, Pb, Bi, In, Sn, Sb, Te, Au and Ag, patterned in parallel at the same time.

Further, the organic layer may comprise one or more compound(s) selected from compounds having electroluminescent peak of wavelength of not less than 560 nm, at the same time, to form a white electroluminescent device. Those compounds having electroluminescent peak of wavelength of not less than 560 nm can be exemplified by the compounds represented by one of Chemical Formulas (7) to (11). Chemical Formula 7 M¹L³L⁴L⁵

In Chemical Formula (7), M¹ is selected from metals of Group 7, 8, 9, 10, 11, 13, 14, 15 and 16 in the Periodic Table of Elements, and ligands L³, L⁴ and L⁵ are independently selected from the following structures: 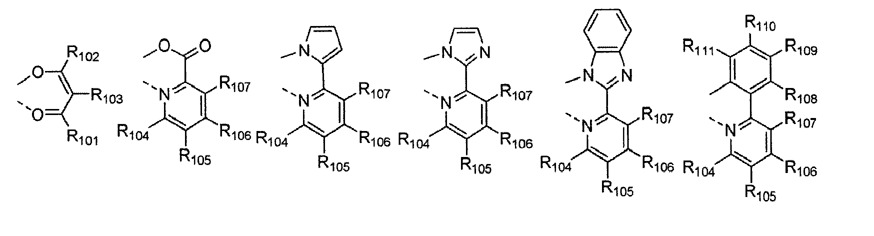 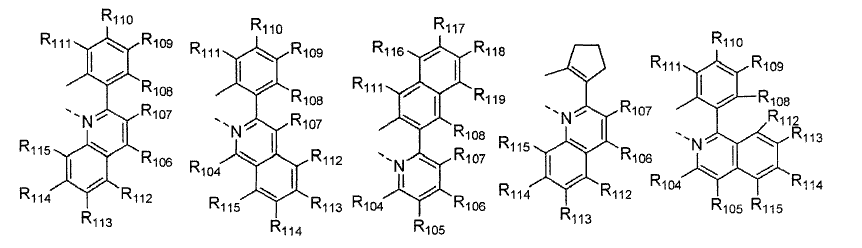 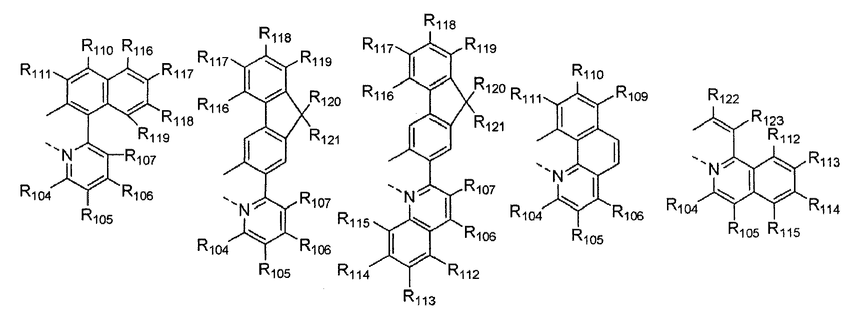 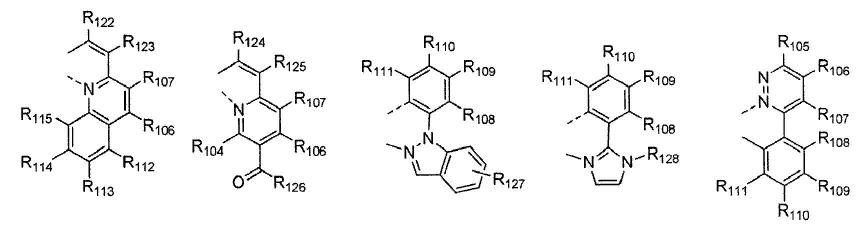 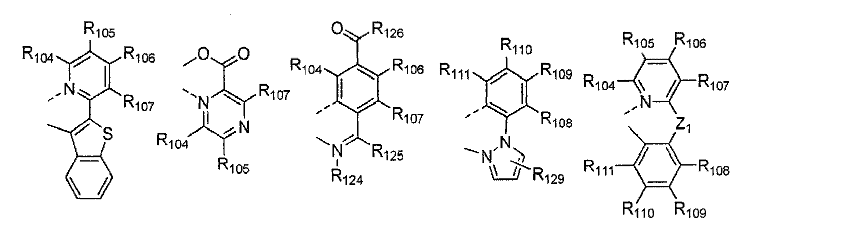 wherein, R₁₀₁ through R₁₀₃ independently represent hydrogen, (C1-C60)alkyl with or without halogen substituent(s), (C6-C60)aryl with or without (C1-C60)alkyl substituent(s), or halogen;
R₁₀₄ through R₁₁₉ independently represent hydrogen, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen, or R₁₁₀ and R₁₁₆ may be linked to an adjacent substituent via (C2-C12)alkylene or (C2-C12)alkenylene to form a fused ring or a multi-fused ring, and the alkyl, cycloalkyl, alkenyl or aryl of R₁₀₄ through R₁₁₉ , or the fused ring or the multi-fused ring formed from R₁₁₀ and R₁₁₆ by linkage via alkylene or alkenylene may be further substituted by one or more substituent(s) selected from (C1-C60)alkyl, (C6-C60)aryl and halogen;
R₁₂₀ through R₁₂₃ independently represent hydrogen, (C1-C60)alkyl with or without halogen substituent(s), (C6-C60)aryl with or without (C1-C60)alkyl substituent(s);
R₁₂₄ and R₁₂₅ independently represent hydrogen, linear or branched (C1-C60)alkyl, (C6-C60)aryl or halogen, or R₁₂₄ and R₁₂₅ may be linked via (C3-C12)alkylene or (C3-C12)alkenylene with or without a fused ring to form an alicyclic ring, or a monocyclic or polycyclic aromatic ring; the alkyl or aryl of R₁₂₄ and R₁₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from linear or branched (C1-C60)alkyl with or without halogen substituent(s), (C1-C30)alkoxy, halogen, tri(C1-C30)alkylsilyl, tri(C6-C30)arylsilyl and (C6-C60)aryl;
R₁₂₆ represents (C1-C60)alkyl, (C6-C60)aryl, (C5-C60)heteroaryl or halogen;
R₁₂₇ through R₁₂₉ independently represent hydrogen, (C1-C60)alkyl, (C6-C60)aryl or halogen; the alkyl or aryl of R₁₂₆ through R₁₂₉ may be further substituted by halogen or (C1-C60)alkyl;
Z₁ represents 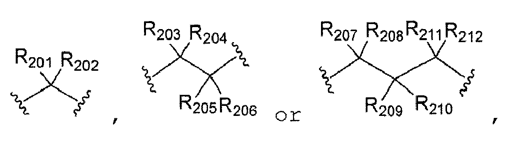 and R₂₀₁ through R₂₁₂ independently represent hydrogen, (C1-C60)alkyl with or without halogen substituent(s), (C1-C30)alkoxy, halogen, (C6-C60)aryl, cyano or (C5-C60)cycloalkyl, or each of R₂₀₁ through R₂₁₂ may be linked to an adjacent substituent via alkylene or alkenylene to form a (C5-C7) spiro-ring or a (C5-C9) fused ring, or each of them may be linked to R₁₀₇ or R₁₀₈ via alkylene or alkenylene to form a (C5-C7) fused ring.

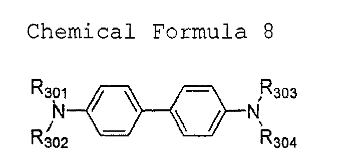

In Chemical Formula (8), R₃₀₁ through R₃₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₃₀₁ through R₃₀₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

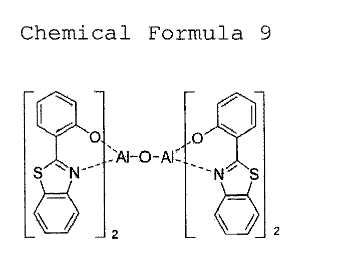

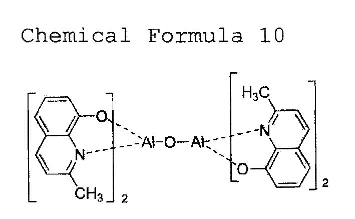

Chemical Formula 11 L⁶L⁷M²(Q)_{d}

In Chemical Formula (11), the ligands, L⁶ and L⁷ are independently selected from the following structures:

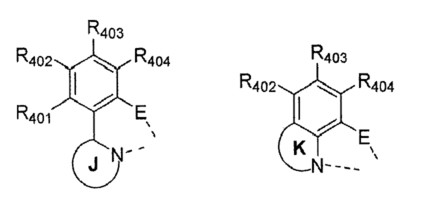

M² is a bivalent or trivalent metal; d is 0 when M² is a bivalent metal, while d is 1 when M² is a trivalent metal;

Q represents (C6-C60)aryloxy or tri(C6-C60)arylsilyl, and the aryloxy and triarylsilyl of Q may be further substituted by (C1-C60)alkyl or (C6-C60)aryl;

E represents O, S or Se; ring J represents oxazole, thiazole, imidazole, oxadiazole, thiadiazole, benzoxazole, benzothiazole, benzimidazole, pyridine or quinoline;
ring K represents pyridine or quinoline, and ring K may be further substituted by (C1-C60)alkyl, or phenyl or naphthyl with or without (C1-C60)alkyl substituent(s);
R₄₀₁ through R₄₀₄ independently represent hydrogen, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring; the pyridine or quinoline may form a chemical bond with R₄₀₁ to provide a fused ring; and
ring J or the aryl group of R₄₀₁ through R₄₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

The compounds having electroluminescent peak of wavelength of not less than 560 nm, can be exemplified by the following compounds, but they are not restricted thereto.

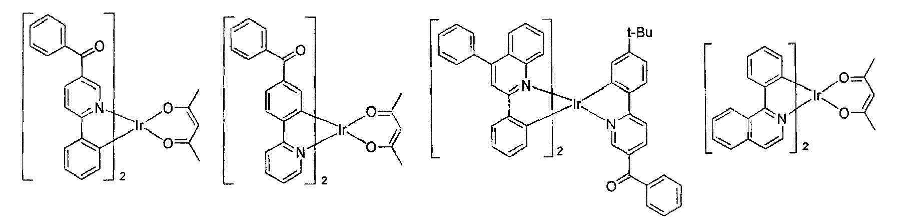

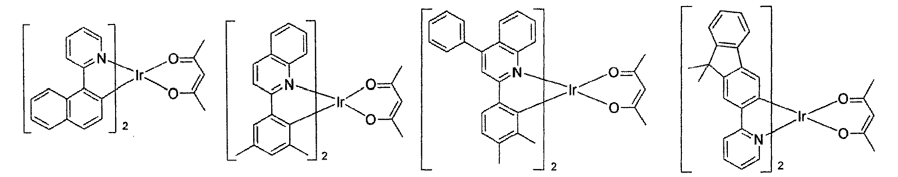

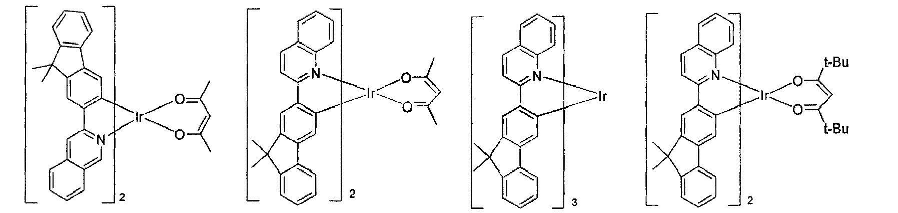

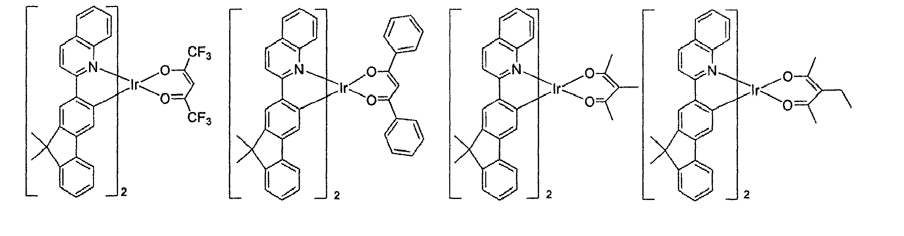

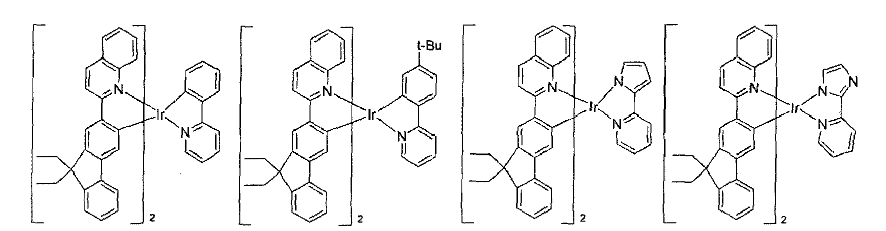

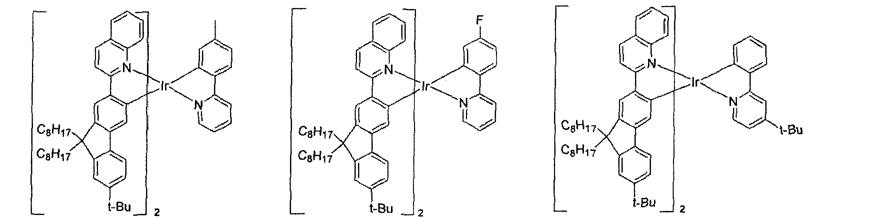

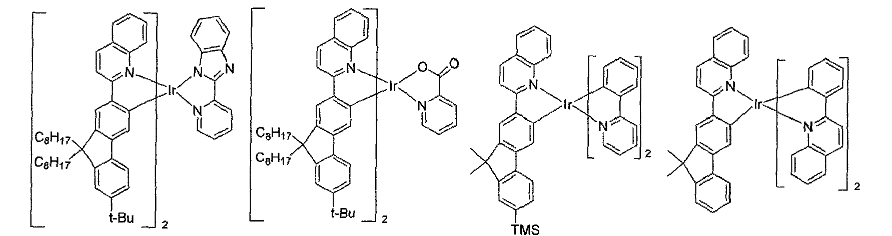

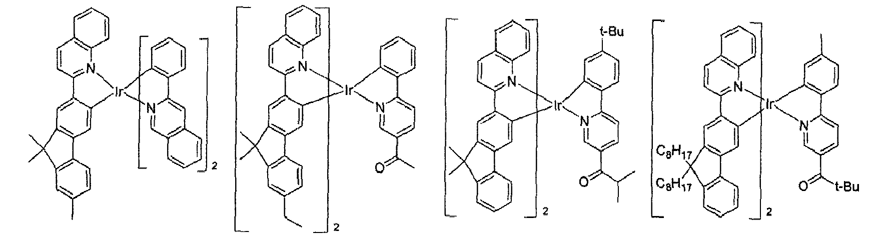

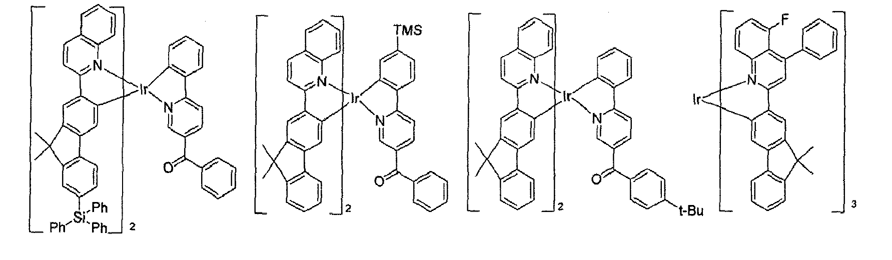

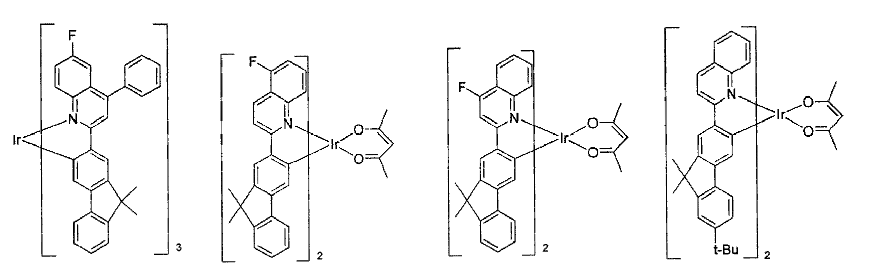

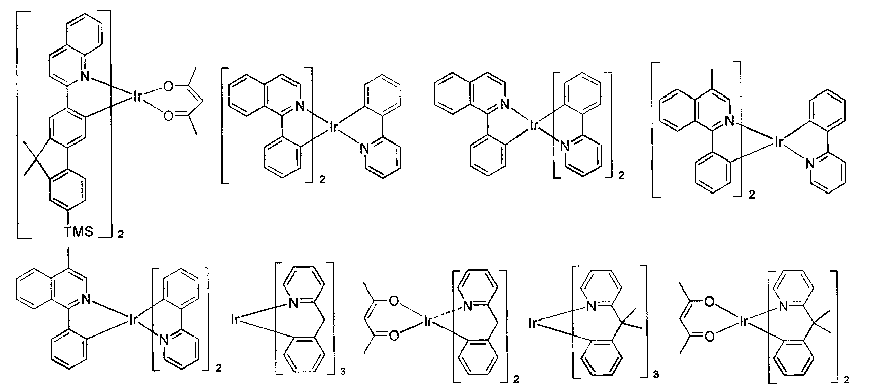

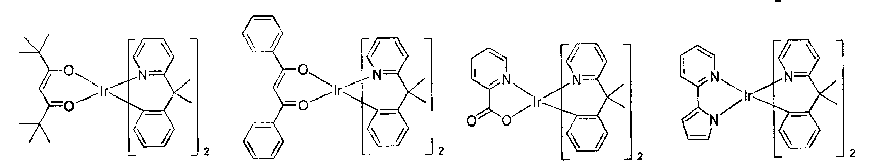

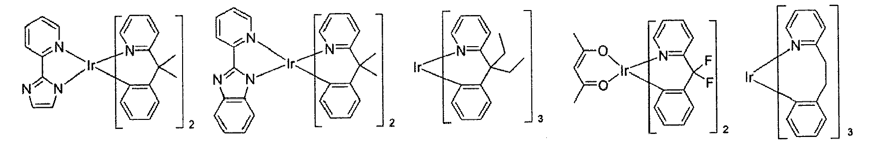

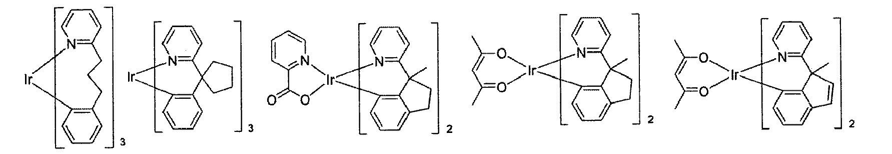

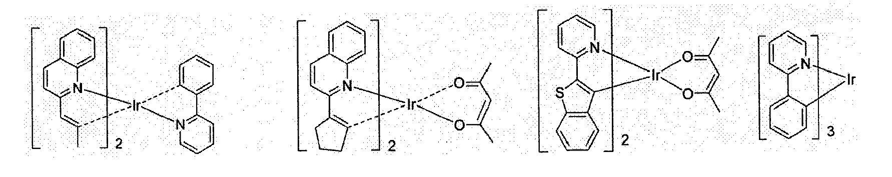

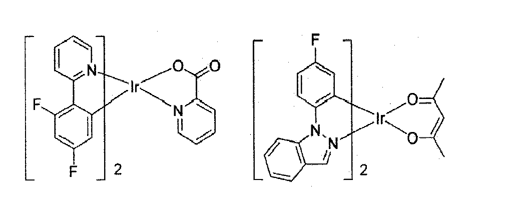

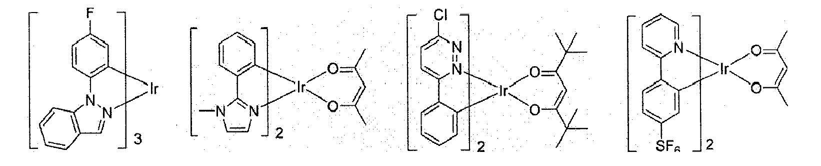

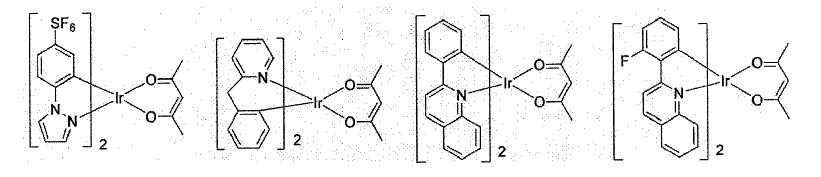

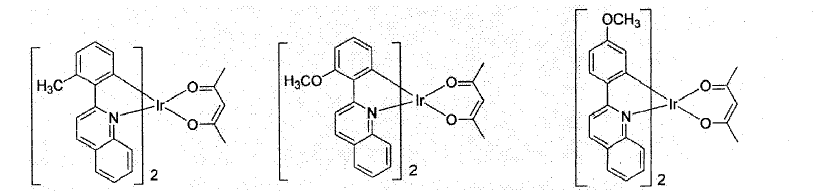

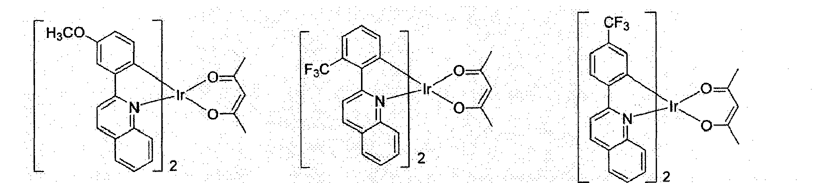

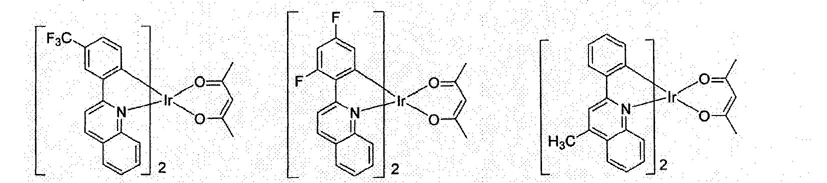

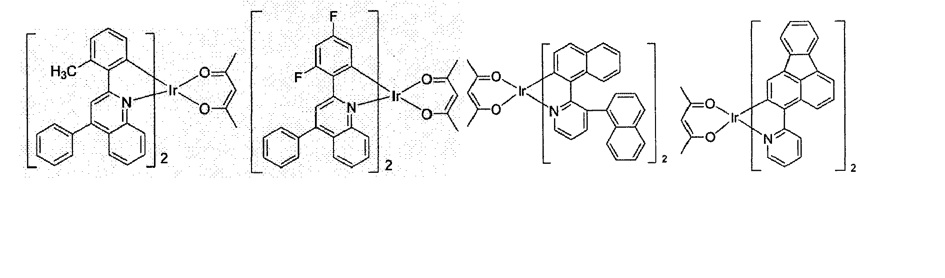

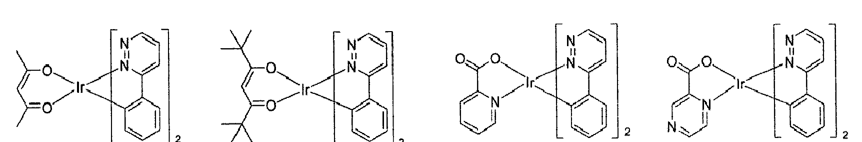

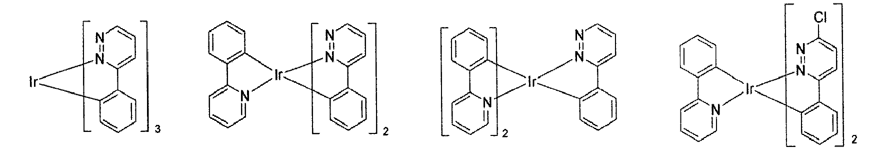

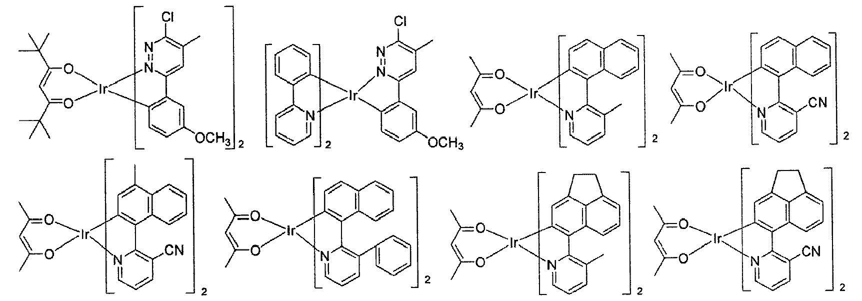

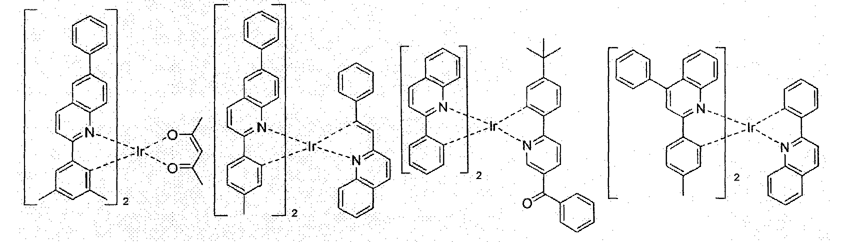

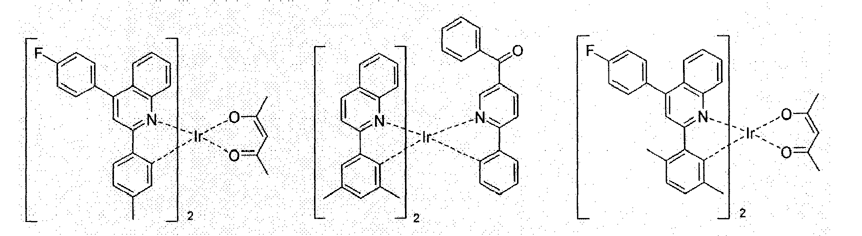

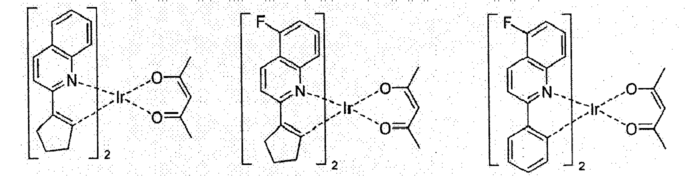

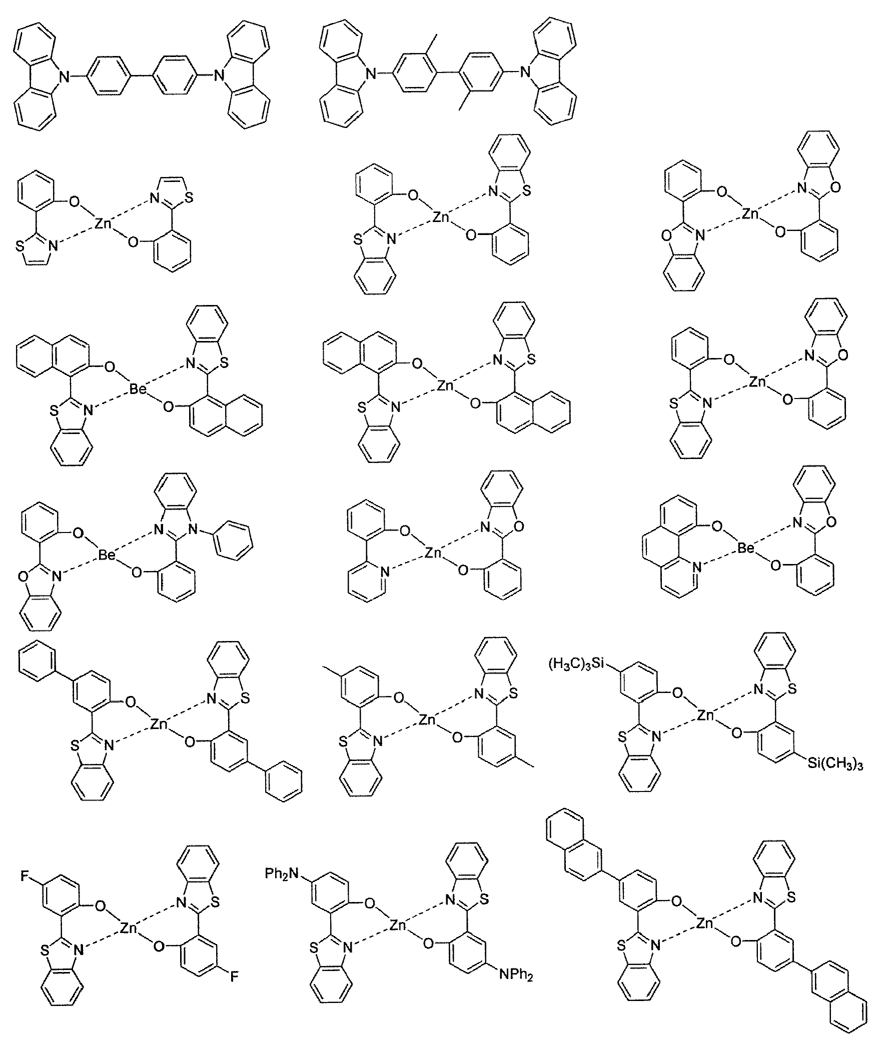

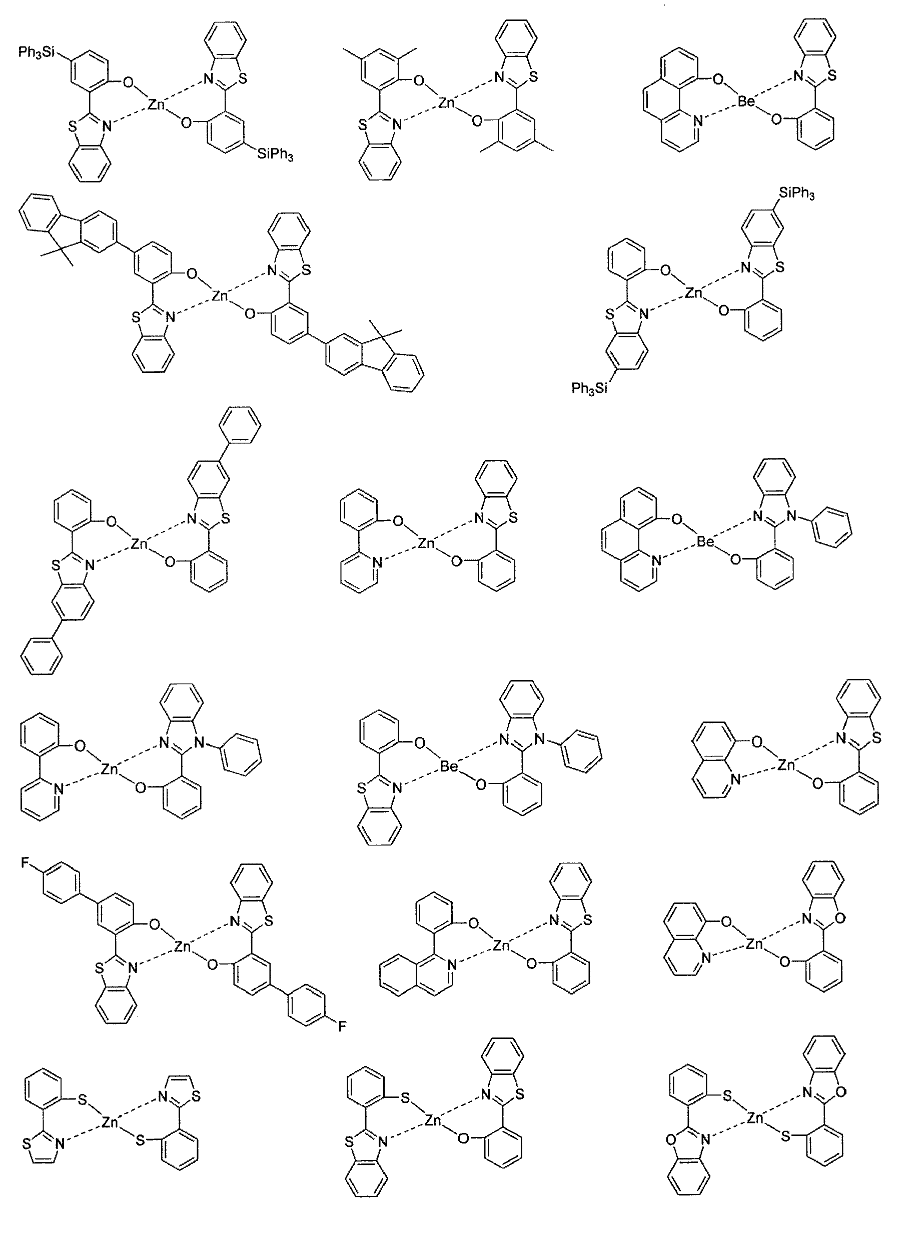

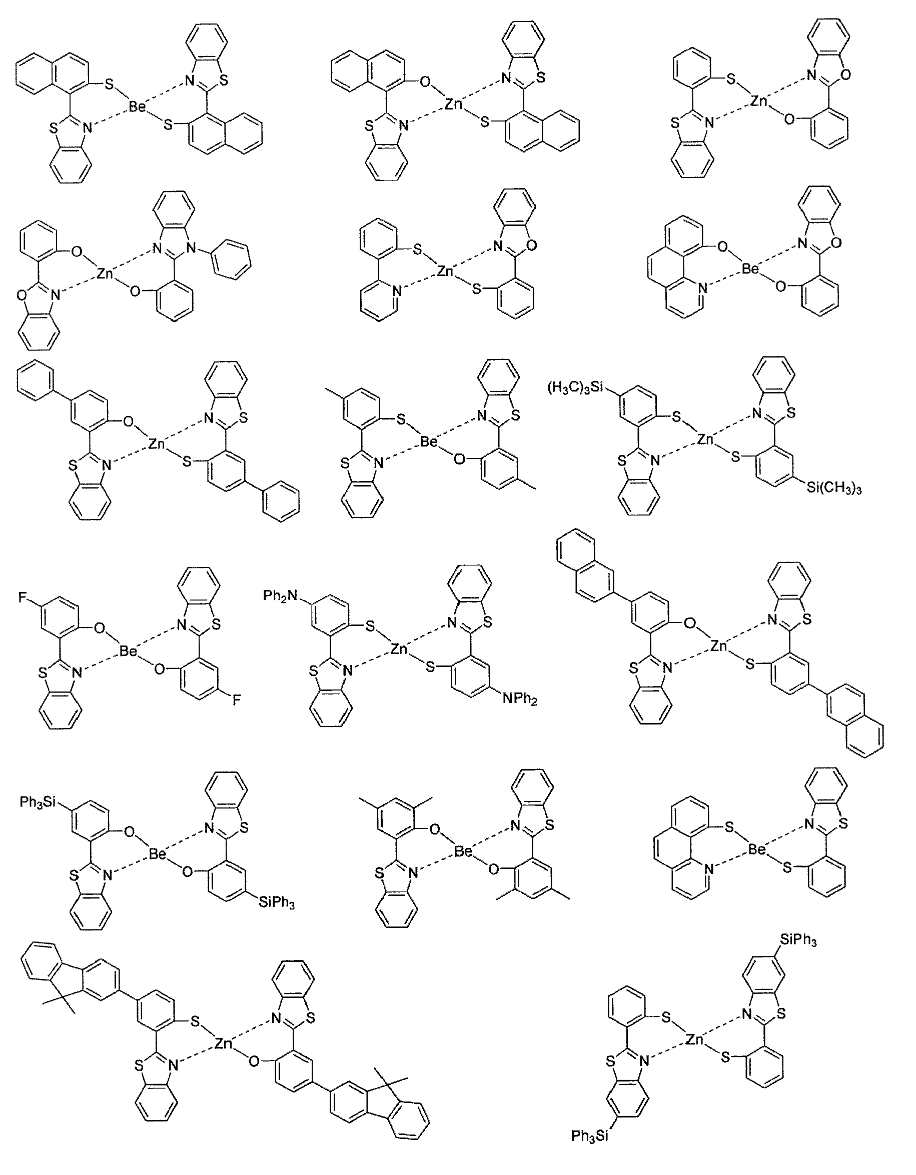

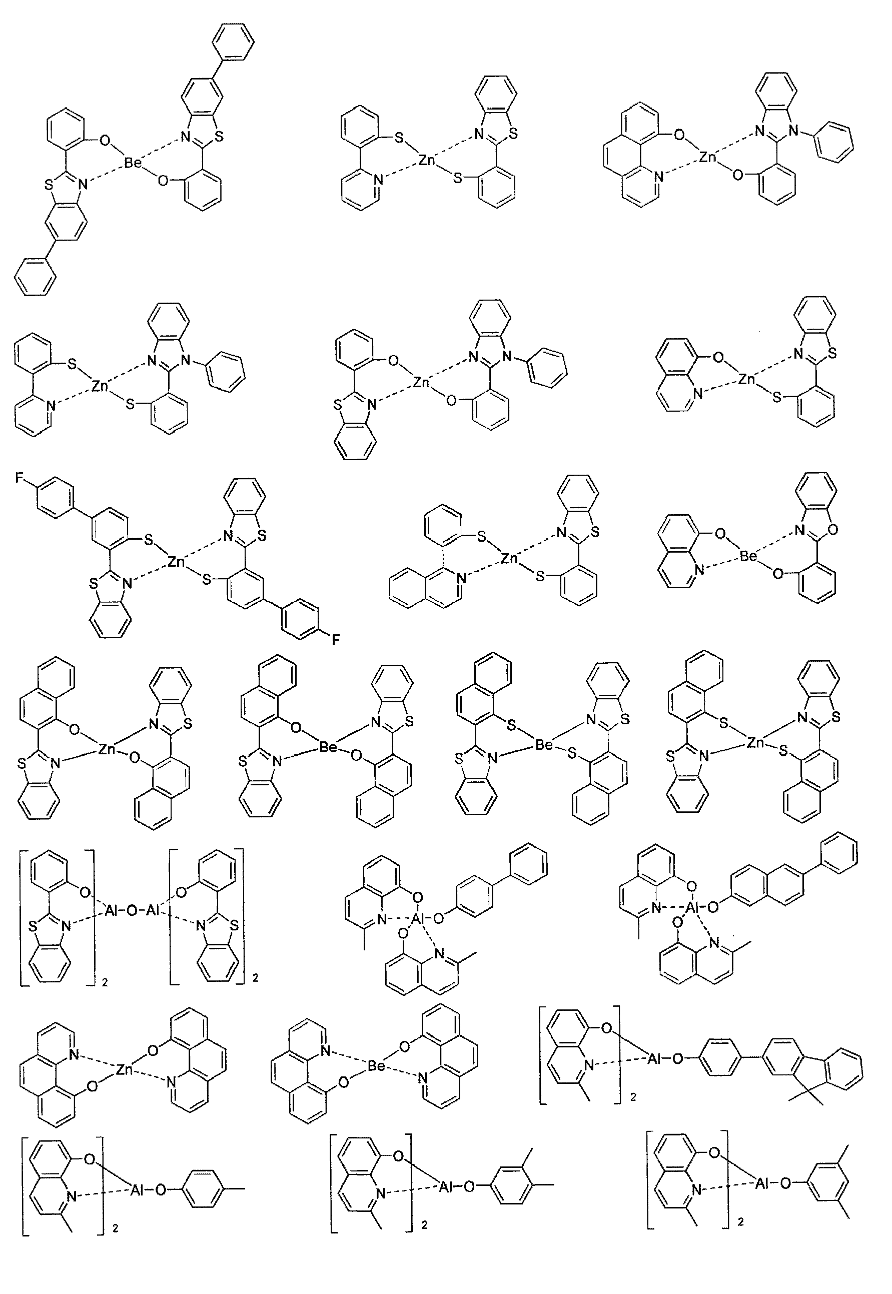

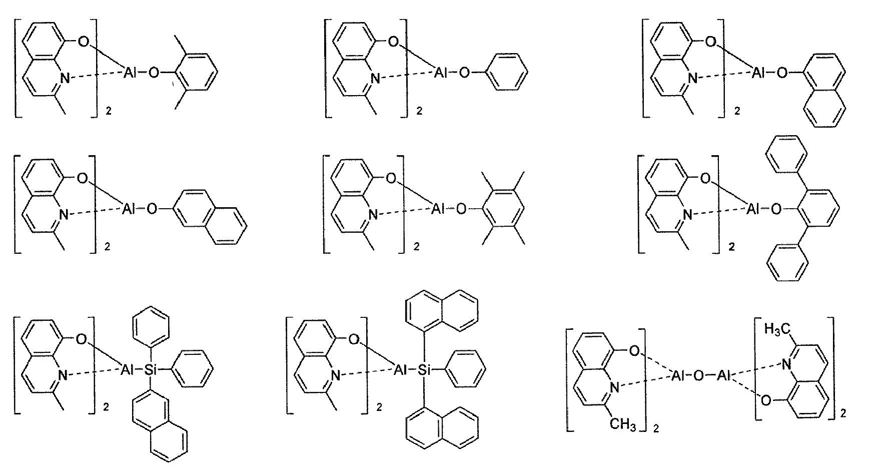

In an organic electroluminescent device according to the present invention, it is preferable to place one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal, or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the invention, having high luminous efficiency and excellent color purity and life property of material, can be advantageously employed for manufacturing OLED's having very good operation life.

### Best Mode

The present invention is further described with respect to the representative compounds of the invention, by describing the organic electroluminescent compounds, the processes for preparing the same, and luminescent properties of the device manufactured therefrom in the Examples below, which are provided for illustration of the embodiments only but are not intended to limit the scope of the invention by any means.

### [Preparation Example 1] Preparation of Compound (1)

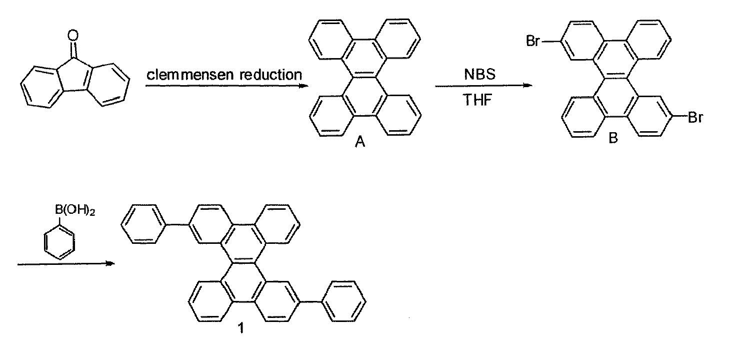

### Preparation of Compound (A)

A 500 mL round-bottomed flask was charged with Zn (4.8 g), HgCl₂ (0.48 g), distilled water (8 mL) and aqueous HCl solution (0.2 ml), and the mixture was stirred. Then, distilled water (3 mL), aqueous HCl solution (7 mL), toluene (7 mL) and 9H-fluoren-9-one (2 g) were added thereto, and the resultant mixture was stirred under reflux for 24 hours. During the reaction, aqueous HCl solution (2 mL) was added thereto every 6 hours (approx.). When the reaction was completed, the reaction mixture was washed with distilled water and ethyl acetate, and dried over MgSO₄. After removing the solvent by using a rotary evaporator, the organic residue was purified via column chromatography to obtain Compound (A) (1.6 g, 44%).

### Preparation of Compound (B)

Compound (A) (1.6 g, 4.87 mmol) and N-bromosuccinimide (1.82 g, 10.23 mmol) were dissolved in dichloromethane (50 mL) under nitrogen atmosphere, and the solution was stirred at 25°C for one day. Then, the reaction was quenched by adding distilled water (100 mL), and the mixture was extracted with dichloromethane (150 mL). The extract was dried under reduced pressure, and the residue was purified by column chromatography to obtain Compound (B) (2.0 g, 4.13 mmol).

### Preparation of Compound (1)

Compound (B) (2.0 g, 4.13 mmol), phenylboronic acid (1.3 g, 10.33 mmol), and tetrakispalladium (0) triphenylphosphine (Pd(PPh₃)₄) (0.6 g, 0.41 mmol) were dissolved in toluene (100 mL) and ethanol (50 mL). Aqueous 2M sodium carbonate solution (50 mL) was added thereto, and the mixture was stirred under reflux at 120°C for 4 hours. Then the reaction mixture was cooled to 25°C, and distilled water (200 mL) was added thereto to quench the reaction. The mixture was extracted with ethyl acetate (150 mL), and dried under reduced pressure. Purification via column chromatography gave the target compound (Compound 1) (1.6 g, 3.33 mmol).

According to the procedure of Preparation Example 1, organic electroluminescent compounds (Compounds 1 to 481) were prepared, and the ¹H NMR and MS/FAB data of those organic electroluminescent compounds are shown in Table 1.

**Table 1**

| compound | ¹H NMR(CDCl₃, 200 MHz) | MS/FAB | |
|---|---|---|---|
| | | found | calculated |
| 1 | δ = 7.41(2H, m), 7.51∼7.52(8H, m), 7.82∼7.88(4H, m), 8.04(2H, m), 8.12(2H, m), 8.18(2H, m), 8.93(2H, m), 9.15(2H, m) | 480.60 | 480.19 |
| 2 | δ = 7.58∼7.59(6H, m), 7.73(2H, m), 7.82∼7.92(6H, m), 8∼8.04(6H, m), 8.12(2H, m), 8.18(2H, m), 8.93(2H, m), 9.15(2H, m) | 580.71 | 580.22 |
| 4 | δ = 1.41 (18H, s), 7.82∼7.88(6H, m), 8.04(2H, m), 8.12(2H, m), 8.93∼8.96(4H, m) | 440.62 | 440.25 |
| 5 | δ = 7.55(4H, m), 7.61(2H, m), 7.82∼7.88(4H, m), 8.04∼8.12(8H, m), 8.18(2H, m), 8.42(2H, m), 8.55(2H, m), 8.93(2H, m), 9.15(2H, m) | 580.71 | 580.22 |
| 10 | δ = 7.82∼7.93(14H, m), 8.04(2H, m), 8.12(6H, m), 8.18(2H, m), 8.93(6H, m), 9.15(2H, m) | 680.83 | 680.25 |
| 15 | δ = 7.35(2H, m), 7.6(2H, m), 7.78∼7.88(6H, m), 7.98(2H, m), 8.06∼8.12(6H, m), 8.21(2H, m), 8.55(2H, m), 8.93(2H, m), 9.66(2H, m) | 582.69 | 582.21 |
| 19 | δ = 1.35(18H, s), 7.37∼7.38(8H, m), 7.82∼7.88(4H, m), 8.04(2H, m), 8.12(2H, m), 8.18(2H, m), 8.93(2H, m), 9.15(2H, m) | 592.81 | 592.31 |
| 24 | δ = 2.34(12H, s), 7.31(2H, m), 7.6(4H, m), 7.82∼7.88(4H, m), 8.04(2H, m), 8.12(2H, m), 8.18(2H, m), 8.93(2H, m), 9.15(2H, m) | 536.70 | 536.25 |
| 32 | δ = 7.5∼7.52(4H, m), 7.82∼7.88(6H, m), 7.98∼8.04(8H, m), 8.12(2H, m), 8.18(2H, m), 8.45(2H, m), 8.93(2H,m), 9.15(2H, m) | 692.89 | 692.16 |
| 36 | δ = 7.41(4H, m), 7.51∼7.52(16H, m), 7.66(6H, m), 7.82∼7.88(4H, m), 8.04(2H, m), 8.12(2H, m), 8.18(2H, m), 8.93(2H, m), 9.15(2H, m) | 784.98 | 784.31 |
| 52 | δ = 7.25(8H, m), 7.41∼7.52(14H, m), 7.82∼7.88(8H, m), 8.04(2H, m), 8.12(2H, m), 8.18(2H, m), 8.93(2H,m), 9.15(2H, m) | 784.98 | 784.31 |
| 71 | δ = 3.81 (4H, s), 6.51(4H, m), 6.69(4H, m), 6.98∼7.02(10H, m), 7.82∼7.88(6H, m), 8.12∼8.13(4H, m), 8.93(2H, m) | 686.84 | 686.27 |
| 83 | δ = 7.25∼7.33(3H, m), 7.45∼7.5(7H, m), 7.58∼7.63(5H, m), 7.69(1H, m), 7.77∼7.88(7H, m), 7.94∼8.04(4H,m), 8.12(3H, m), 8.18(3H, m), 8.55(1H, m), 8.93(2H, m), 9.15(2H, m) | 810.98 | 810.30 |
| 105 | δ = 6.92(2H, s), 7.3(4H, m), 7.45∼7.56(20H, m), 7.64(4H, m), 7.82∼7.88(4H, m), 8.04(2H, m), 8.12(2H, m), 8.18(2H, m), 8.93(2H, m), 9.15(2H, m) | 837.06 | 836.34 |
| 107 | δ = 6.63(8H, m), 6.69(4H, m), 6.81(4H, m), 7.2(8H, m), 7.54(4H, m), 7.82∼7.88(4H, m), 8.04(2H, m), 8.12(2H, m), 8.18(2H, m), 8.93(2H, m), 9.15(2H, m) | 815.01 | 814.33 |
| 113 | δ = 7.25(8H, m), 7.58∼7.59(6H, m), 7.73(2H, m), 7.82∼7.92(6H, m), 8∼8.04(6H, m), 8.12(2H, m), 8.18(2H, m), 8.93(2H, m), 9.15(2H, m) | 732.91 | 732.28 |
| 115 | δ = 7.47(4H, m), 7.58∼7.59(6H, m), 7.73(2H, m), 7.82∼7.92(10H, m), 8∼8.04(6H, m), 8.12(2H,m), 8.18(2H, m), 8.93(2H, m), 9.15(2H, m) | 732.91 | 732.28 |
| 117 | δ = 7.48(4H, m), 7.57∼7.59(8H, m), 7.7∼7.73(4H, m), 7.82∼7.92(6H, m), 8∼8.04(6H, m), 8.12(2H, m), 8.18(2H, m), 8.93(2H, m), 9.15(2H, m) | 732.91 | 732.28 |
| 121 | δ = 1.72(12H, s), 7.28(2H, m), 7.38(2H, m), 7.47(4H, m), 7.53∼7.55(3H, m), 7.61∼7.63(2H,m), 7.77∼7.93(12H, m), 8.04∼8.12(5H, m), 8.18(2H, m), 8.93(2H, m), 9.15(2H, m) | 865.11 | 864.38 |
| 128 | δ = 1.72(12H, s), 7.41(2H, m), 7.51∼7.52(8H, m), 7.63(4H, m), 7.77∼7.93(12H, m), 8.04(2H, m), 8.12(2H, m), 8.18(2H, m), 8.93(2H, m), 9.15(2H, m) | 865.11 | 864.38 |
| 132 | δ = 7.39∼7.41(10H, m), 7.51∼7.52(8H, m), 7.82∼7.91(12H, m), 8.04(2H, m), 8.12(2H, m), 8.18(2H, m), 8.93(2H, m), 9.15(2H, m) | 833.02 | 832.31 |
| 136 | δ = 7.39∼7.51(18H, m), 7.79∼7.91(20H, m), 8.04(2H, m), 8.12(2H, m), 8.18(2H, m), 8.93(2H, m), 9,15(2H, m) | 985.22 | 984.38 |
| 138 | δ = 7.25(8H, m), 7.39∼7.41(10H, m), 7.51∼7.52(8H, m), 7.82∼7.91(12H, m), 8.04(2H, m), 8.12(2H, m), 8.18(2H, m), 8.93(2H, m), 9.15(2H, m) | 985.22 | 984.38 |
| 141 | δ = 2.59(6H, s), 7.06(2H, m), 7.39∼7.41(10H, m), 7.51∼7.52(8H, m), 7.73(2H, m), 7.82∼7.91(14H,m), 8.04(2H, m), 8.12(2H, m), 8.18(2H, m), 8.93(2H, m), 9.15(2H, m) | 1013.27 | 1012.41 |
| 143 | δ = 7.41(4H, m), 7.51∼7.52(16H, m), 8.04(4H,m), 8.18(4H, m), 9.15(4H, m) | 632.79 | 632.25 |
| 144 | δ = 7.58∼7.59(12H, m), 7.73(4H, m), 7.92(4H,m), 8∼8.04(12H, m), 8.18(4H, m), 9.15(4H, m) | 833.02 | 832.31 |
| 146 | δ = 7.55(8H, m), 7.61(4H, m), 8.04∼8.08(12H,m), 8.18(4H, m), 8.42(4H, m), 8.55(4H, m), 9.15(4H, m) | 833.02 | 832.31 |
| 148 | δ = 1.41(36H, s), 7.85(4H, m), 8.04(4H, m), 8.96(4H, m) | 552.83 | 552.38 |
| 161 | δ = 1.35(36H, s), 7.37∼7.38(16H, m), 8.04(4H, m), 8.18(4H, m), 9.15(4H, m) | 857.21 | 856.50 |
| 167 | δ = 7.41(4H, m), 7.48∼7.57(28H, m), 7.7(4H, m), 8.04(4H, m), 8.18(4H, m), 9.15(4H, m) | 937.17 | 936.38 |
| 178 | δ = 1.51(24H, m), 2.09(8H, m), 7.28(4H, m), 7.38(4H, m), 7.55(4H, m), 7.63(4H, m), 7.77(4H, m), 7.87∼7.93(8H, m), 8.04(4H, m), 8.18(4H, m), 9.15(4H, m) | 120.58 | 1200.56 |
| 194 | δ = 7.99∼8.04(12H, m), 8.18(4H, m), 8.75(8H, m),9.15(4H, m) | 636.74 | 636.23 |
| 204 | δ = 7.48(8H, m), 7.57∼7.59(16H, m), 7.7∼7.73(8H, m), 7.92(4H, m), 8∼8.04(12H, m), 8.18(4H, m), 9.15(4H, m) | 1137.41 | 1136.44 |
| 217 | δ = 7.47(8H, m), 7.55(8H, m), 7.61 (4H, m), 7.85(8H, m), 8.04∼8.08(12H, m), 8.18(4H, m), 8.42(4H, m), 8.55(4H, m), 9.15(4H, m) | 1137.41 | 1136.44 |
| 224 | δ = 7.58∼7.59(6H, m), 7.73(2H, m), 7.82δ7.92(6H, m), 8∼8.04(6H, m), 8.12(2H, m), 8.18(2H, m), 8.93(2H, m),9.15(2H, m) | 580.71 | 580.22 |
| 225 | δ = 7.25(8H, m), 7.41(2H, m), 7.51∼7.52(8H, m), 7.82∼7.88(4H, m), 8.04(2H, m), 8.12(2H, m), 8.18(2H, m), 8.93(2H, m), 9.15(2H, m) | 632.79 | 632.25 |
| 226 | δ = 7.55(4H, m), 7.61(2H, m), 7.82∼7.88(4H, m), 8.04∼8.12(8H, m), 8.18(2H, m), 8.42(2H, m), 8.55(2H, m), 8.93(2H, m), 9.15(2H, m) | 580.71 | 580.22 |
| 227 | δ = 1.72(12H, s), 7.28(2H, m), 7.38(2H, m), 7.55(2H, m), 7.63(2H, m), 7.77∼7.93(10H, m), 8.04(2H, m),8.12(2H, m), 8.18(2H, m), 8.93(2H, m), 9.15(2H, m) | 712.92 | 712.31 |
| 228 | δ = 1.41(18H, s), 7.82∼7.88(6H, m), 8.04(2H,m), 8.12(2H, m), 8.93∼8.96(4H, m) | 40.62 | 440.25 |
| 246 | δ = 2.34(12H, s), 7.31(2H, m), 7.6(4H, m), 7.82∼7.88(4H, m), 8.04(2H, m), 8.12(2H, m), 8.18(2H, m), 8.93(2H, m), 9.15(2H, m) | 536.70 | 536.25 |
| 247 | δ = 7.41(2H, m), 7.48∼7.57(14H, m), 7.7(2H, m), 7.82∼7.88(4H, m), 8.04(2H, m), 8.12(2H, m), 8.18(2H, m), 8.93(2H, m), 9.15(2H, m) | 632.79 | 632.25 |
| 248 | δ = 7.41∼7.51(10H, m), 7.79∼7.88(12H, m), 8.04(2H, m), 8.12(2H, m), 8.18(2H, m), 8.93(2H, m), 9.15(2H, m) | 632.79 | 632.25 |
| 250 | δ = 7.5∼7.52(4H, m), 7.82∼7.88(6H, m), 7.98∼8.04(8H, m), 8.12(2H, m), 8.18(2H, m), 8.45(2H, m), 8.93(2H,m), 9.15(2H,m) | 692.89 | 692.16 |
| 254 | δ = 7.35(2H, m), 7.58(2H, m), 7.81∼7.88(6H, m), 8.06∼8.12(6H, m), 8.21(2H, m), 8.38(2H, m), 8.55(2H, m), 8.83(2H, m), 8.93(2H, m), 9.66(2H, m) | 684.78 | 684.23 |
| 263 | δ = 7.16∼7.19(8H, m), 7.28(2H, m), 7.35∼7.38(6H, m), 7.55(2H, m), 7.63(2H, m), 7.75∼7.93(14H, m), 8.04(2H, m), 8.12(2H, m), 8.18(2H, m), 8.93(2H, m), 9.15(2H, m) | 957.16 | 956.34 |
| 266 | δ = 7.25(4H, m), 7.53(4H, m), 7.82∼7.88(8H, m), 8.01∼8.04(4H, m), 8.12(2H, m), 8.18(4H, m), 8.93(2H, m), 9.15(2H, m) | 746.94 | 746.19 |
| 273 | δ = 7.25(8H, m), 7.41∼7.52(14H, m), 7.82∼7.88(8H, m), 8.04(2H, m), 8.12(2H, m), 8.18(2H, m), 8.93(2H, m), 9.15(2H, m) | 784.98 | 784.31 |
| 282 | δ = 7.47(4H, m), 7.58∼7.59(6N, m), 7.73(2H, m), 7.82∼7.92(10H, m), 8∼8.04(6H m), 8.12(2H,m), 8.18(2H, m), 8.93(2H, m), 9.15(2H, m) | 731.91 | 731.28 |
| 285 | δ = 7.41(2H, m), 7.51∼7.52(8H, m), 7.58(4H, m), 7.73(4H, m), 7.82∼7.92(8H, m), 8.04(2H, m), 8.12(2H, m), 8.18(2H, m), 8.93(2H, m), 9.15(2H, m) | 731.91 | 731.28 |
| 286 | δ = 1.69(12H, s), 6.94(2H, s), 7.22∼7.3(6H, m), 7.42(2H, m), 7.82∼7.88(4H, m), 7.98(2H, m), 8.12(4H, m), 8.93(2H, m), 9.09(2H, m) | 612.80 | 612.28 |
| 292 | δ = 7.25(8H, m), 7.55(4H, m), 7.61(2H, m), 7.82∼7.88(4H, m), 8.04∼8.12(8H, m), 8.18(2H, 8.42(2H, m), 8.55(2H, m), 8.93(2H, m), 9.15(2H, m) | 731.91 | 731.28 |
| 293 | δ = 1.72(12H, s), 7.25∼7.28(10H, m), 7.38(2H, m), 7.55(2H, m), 7.63(2H, m), 7.77∼7.93(10H, m), 8.04(2H, m), 8.12(2H, m), 8.18(2H, m), 8.93(2H, m), 9.15(2H, m) | 865.11 | 864.38 |
| 299 | δ = 7.58∼7.59(3H, m), 7.73(1 H, m), 7.82∼7.92(7H, m), 8∼8.04(3H, m), 8.12(3H, m), 8.18(1H, m), 8.93(3H,m), 9.15(1H, m) | 454.56 | 454.17 |
| 303 | δ = 7.55(2H, m), 7.61(1H, m), 7.82∼7.88(6H, m), 8.04∼8.12(6H, m), 8.18(1H, m), 8.42(1H, m), 8.55(1H, m), 8.93(3H, m), 9.15(1 H, m) | 454.56 | 454.17 |
| 316 | δ = 1.35(9H, s), 7.37∼7.38(4H, m), 7.82∼7.88(6H, m), 8.04(1H, m), 8.12(3H, m), 8.18(1H, m), 8.93(3H,m), 9.15(1H, m) | 460.61 | 460.22 |
| 325 | δ = 7.41(1H, m), 7.51(2H, m), 7.59(2H, m), 7.79∼7.88(8H, m), 8∼8.04(3H, m), 8.12(3H, m), 8.18(1H, m), 8.4(2H, m), 8.93(3H, m), 9.15(1 H, m) | 530.66 | 530.20 |
| 326 | δ = 7.41(1H, m), 7.51∼7.55(4H, m), 7.79∼7.88(8H, m), 8.01∼8.12(7H, m), 8.18(1H, m), 8.55(1H, m), 8.93(3H, m), 9.15(1H, m) | 530.66 | 530.20 |
| 329 | δ = 7.5∼7.52(2H, m), 7.82∼7.88(7H, m), 7.98∼8.04(4H, m), 8.12(3H, m), 8.18(1H, m), 8.45(1H, m), 8.93(3H, m), 9.15(1H, m) | 510.65 | 510.14 |
| 339 | δ = 3.83(3H, s), 7.05(2H, m), 7.68(2H, m), 7.82∼7.88(6H, m), 8.04(1H, m), 8.12(3H, m), 8.18(1H, m), 8.93(3H, m), 9.15(1H, m) | 434.53 | 434.17 |
| 345 | δ = 2.62(2H, m), 2.91(2H, m), 7.02∼7.06(2H, m), 7.15(2H, m), 7.28(1H, m), 7.38(1H, m), 7.55(1H,m), 7.63(1H, m), 7.77∼7.93(9H, m), 8.04(1H, m), 8.12(3H, m), 8.18(1H, m), 8.93(3H, m), 9.15(1 H, m) | 594.74 | 594.23 |
| 348 | δ = 7.11(4H, m), 7.26∼7.38(8H, m), 7.55(1H, m), 7.63(1H, m), 7.77∼7.93(9H, m), 8.04(1H, m), 8.12(3H, m), 8.18(1H, m), 8.93(3H, m), 9.15(1H, m) | 644.80 | 644.25 |
| 359 | δ = 7.22∼7.25(4H, m), 7.45∼7.5(3H, m), 7.58∼7.59(3H, m), 7.82∼7.88(8H, m), 8.04(1H, m), 8.12(3H, m),8.18(1H, m), 8.56(1H, m), 8.93(3H, m), 9.15(1H, m) | 596.72 | 596.23 |
| 363 | δ = 6.95(2H, m), 7.3(1H, m), 7.45(2H, m), 7.72(2H, m), 7.82∼7.88(6H, m), 7.98(1H, m), 8.12(4H, m), 8.93(3H, m), 9.09(1H, m) | 430.54 | 430.17 |
| 370 | δ = 6.97∼7.02(3H, m), 7.16∼7.21(6H,m),7.82∼7.88(7H, m), 8.12∼8.13(4H, m), 8.93(3H, m) | 525.66 | 525.16 |
| 382 | δ = 7.36∼7.42(3H, m), 7.48(1H, m), 7.74∼7.88(11H, m),8.03∼8.12(7H, m), 8.18(1H, m), 8.93(3H, m), 9.15(1H, m) | 586.66 | 586.19 |
| 392 | δ = 7.48(2H, m), 7.57∼7.59(4H, m), 7.7∼7.73(2H, m), 7.82∼7.92(7H, m), 8∼8.04(3H, m), 8.12(3H, m), 8.18(1H, m), 8.93(3H, m), 9.15(1H, m) | 530.66 | 530.20 |
| 396 | δ = 1.72(6H, s), 7.28(1H, m), 7.38(1H, m), 7.48(2H, m), 7.55∼7.63(3H, m), 7.7(1H, m), 7.77∼7.93(9H,m), 8.04(1H, m), 8.12(3H. m), 8.18(1H, m), 8.93(3H, m), 9.15(1H, m) | 596.76 | 596.25 |
| 397 | δ = 1.72(6H, s), 7.28(1H, m), 7.38(1H, m), 7.47(2H, m), 7.55(1H, m), 7.63(1H, m), 7.77∼7.93(11H, m), 8.04(1H, m), 8.12(3H, m), 8.18(1H, m), 8.93(3H, m), 9.15(1H, m) | 596.76 | 596.25 |
| 402 | δ = 7.58∼7.59(5H, m), 7.73(3H, m), 7.82∼7.92(9H, m), 8∼8.04(3H, m), 8.12(3H, m), 8.18(1H, m), 8.93(3H, 9.15(1H, m) | 580.71 | 580.22 |
| 412 | δ = 7.39∼7.41(5H, m), 7.48∼7.57(7H, m), 7.7(1H, m), 7.82∼7.91(10H, m), 8.04(1H, m), 8.12(3H, m), 8.18(1H, m), 8.93(3H, m), 9.15(1 H, m) | 656.81 | 656.25 |
| 416 | δ = 2.59(2H, m), 7.06(2H, m), 7.39∼7.41(5H, m), 7.51∼7.52(4H, m), 7.73(1H, m), 7.82∼7.91(11H, m), 8.04(1H, m), 8.12(3H, m), 8.18(1H, m), 8.93(3H, m), 9.15(1 H, m) | 670.84 | 670.27 |
| 418 | δ = 7.41(1H, m), 7.51∼7.52(4H, m), 7.58∼7.59(3H, m), 7.73(1H, m), 7.82∼7.92(5H, m), 8∼8.04(4H, m), 8.12(2H, m), 8.18(2H, m), 8.93(2H, m), 9.15(2H, m) | 530.66 | 530.20 |
| 420 | δ = 7.41(1H, m), 7.51∼7.55(6H, m), 7.61(1H, m), 7.82∼7.88(4H, m), 8.04∼8.12(6H, m), 8.18(2H,m), 8.42(1 H, m), 8.55(1 H, m), 8.93(2H, m), 9.15(2H, m) | 530.66 | 530.20 |
| 430 | δ = 1.35(9H, s), 7.37∼7.41 (5H, m), 7.51∼7.52(4H, m), 7.82∼7.88(4H, m), 8.04(2H, m), 8.12(2H, m), 8.18(2H, m), 8.93(2H, m), 9.15(2H, m) | 536.70 | 536.25 |
| 436 | δ = 2.34(6H, s), 7.31(1H, m), 7.41(1H, m), 7.51∼7.52(4H, m), 7.6(2H, m), 7.82∼7.88(4H, 8.04(2H, m), 8.12(2H, m), 8.18(2H, m), 8.93(2H, m), 9.15(2H, m) | 508.65 | 508.22 |
| 445 | δ = 2.62(2H, m), 2.91(2H, m), 7.02∼7.06(2H, m), 7.15(2H, m), 7.28(1H, m), 7.38∼7.41(2H, m), 7.51∼7.55(5H, m), 7.63(1H, m), 7.77∼7.93(7H, m),8.04(2H, m), 8.12(2H, m), 8.18(2H, m), 8.93(2H, m), 9.15(2H, m) | 670.84 | 670.27 |
| 448 | δ = 7.25(2H, m), 7.41(1H, m), 7.51∼7.53(6H, m), 7.82∼7.88(6H, m), 8.01∼8.04(3H, m), 8.12(2H,m), 8.18(3H, m), 8.93(2H, m), 9.15(2H, m) | 613.77 | 613.19 |
| 462 | δ = 7.25(4H, m), 7.41(1H, m), 7.51∼7.52(4H, m), 7.58∼7.59(3H, m), 7.73(1H, m), 7.82∼7.92(5H, m), 8∼8.04(4H, m), 8.12(2H, m), 8.18(2H, m), 8.93(2H, m), 9.15(2H, m) | 606.75 | 606.23 |
| 464 | δ = 7.41∼7.52(7H, m), 7.58∼7.59(3H, m), 7.73(1H, m), 7.82∼7.92(7H, m), 8∼8.04(4H, m), 8.12(2H, m), 8.18(2H, m), 8.93(2H, m), 9.15(2H, m) | 606.75 | 606.23 |
| 470 | δ = 1.72(6H, s), 7.28(1H, m), 7.38∼7.41(2H, m), 7.51∼7.55(7H, m), 7.63(1H, m), 7.77∼7.93(7H,m), 8.01∼8.04(4H, m), 8.12(2H, m), 8.18(2H, m), 8.55(2H, m), 8.93(2H, m), 9.15(2H, m) | 722.91 | 722.30 |
| 471 | δ = 1.69(6H, s), 6.94(1H, s), 7.22∼7.3(3H, m), 7.41∼7.42(2H, m), 7.51∼7.52(4H, m), 7.82∼7.88(4H, m), 7.98∼8.04(2H, m), 8.12(3H, m), 8.18(1H, m), 8.93(2H, m), 9.09(1 H, m), 9.15(1 H, m) | 546.70 | 546.23 |
| 475 | δ = 7.25(4H, m), 7.41(1H, m), 7.51∼7.55(6H, m), 7.61(1H, m), 7.82∼7.88(4H, m), 8.04∼8.12(6H, m), 8.18(2H, m), 8.42(1H, m), 8.55(1H, m), 8.93(2H, m), 9.15(2H, m) | 606.75 | 606.23 |
| 476 | δ = 1.72(6H, s), 7.25∼7.28(5H, m), 7.38∼7.41(2H, m), 7.51∼7.55(5H, m), 7.63(1H, m), 7.77∼7.93(7H,m), 8.04(2H, m), 8.12(2H, m), 8.18(2H, m), 8.93(2H, m), 9.15(2H, m) | 672.85 | 672.28 |
| 477 | δ = 1.72(6H, s), 7.28(1H, m), 7.38∼7.41(2H, m), 7.48∼7.63(9H, m), 7.7(1H, m), 7.77∼7.93(7H, m), 8.04(2H, m), 8.12(2H, m), 8.18(2H, m), 8.93(2H, m), 9.15(2H, m) | 672.85 | 672.28 |
| 478 | δ = 7.41(1H, m), 7.48∼7.61(10H, m), 7.7(1H, m), 7.82∼7.88(4H, m), 8.04∼8.12(6H, m), 8.18(2H,m), 8.42(1 H, m), 8.55(1H, m), 8.93(2H, m), 9.15(2H, m) | 606.75 | 606.23 |
| 479 | δ = 7.41∼7.55(9H, m), 7.61(1H, m), 7.82∼7.88(6H, m), 8.04∼8.12(6H, m), 8.18(2H, m), 8.42(1H, m), 8.55(1H, m), 8.93(2H, m), 9.15(2H, m) | 606.75 | 606.23 |
| 481 | δ = 7.41∼7.57(8H, m), 7.82∼7.88(6H, m), 8.04(2H, m), 8.12(2H, m), 8.18(2H, m), 8.42(1H, m), 8.7(1H,m), 8.93(2H, m), 9.15(2H, m), 9.24(1 H, m) | 557.68 | 557.21 |

### [Example 1] Manufacture of an OLED employing organic electroluminescent compound according to the invention

An OLED device was manufactured by using an electroluminescent material according to the invention.

First, a transparent electrode ITO thin film (15 Ω/□) (2) prepared from glass for OLED (produced by Samsung-Corning) (1) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA, of which the structure is shown below) was placed in a cell of the vacuum vapor-deposit device, which was then ventilated up to 10⁻⁶ torr vacuum in the chamber. Electric current was applied to the cell to evaporate 2-TNATA, thereby providing vapor-deposit of a hole injecting layer (3) having 60 nm thickness on the ITO substrate.

Then, to another cell of the vacuum vapor-deposit device, charged was N,N'-bis(α-naphthyl)-N,N'-diphenyl-4,4'-diamine (NPB, of which the structure is shown below), and electric current was applied to the cell to evaporate NPB, thereby providing vapor-deposit of a hole transport layer (4) of 20 nm of thickness on the hole injecting layer.

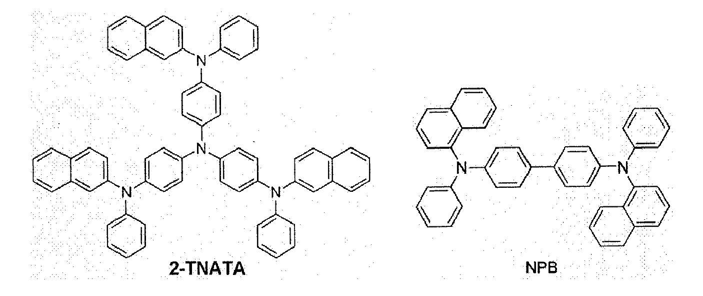

After forming the hole injecting layer and the hole transport layer, an electroluminescent layer was formed according to the following procedure. To one cell of a vacuum vapor-deposit device, charged was a compound according to the present invention (e.g. Compound 10) as electroluminescent material, while DSA-Ph (of which the structure is shown below) was charged to another cell. The two cells were simultaneously heated to carry out vapor-deposition at the rate of vapor-deposition of DSA-Ph of 2 to 5 wt%, to vapor-deposit an electroluminescent layer (5) having 30 nm thickness on the hole transport layer.

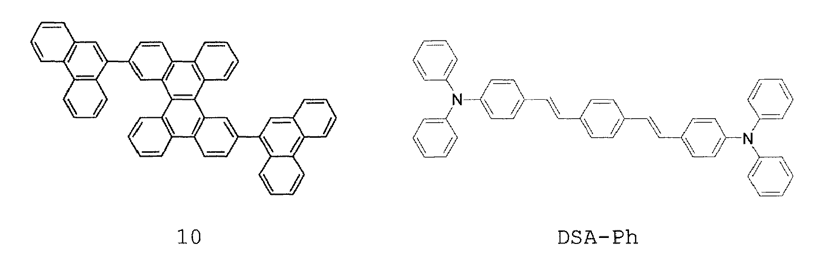

Then, tris(8-hydroxyquinoline)aluminum (III) (Alq) (of which the structure is shown below) was vapor-deposited as an electron transport layer (6) with a thickness of 20 nm, and then lithium quinolate (Liq) was vapor-deposited as an electron injecting layer (7) with a thickness of 1 to 2 nm. Thereafter, an Al cathode (8) was vapor-deposited with a thickness of 150 nm by using another vacuum vapor-deposit device to manufacture an OLED.

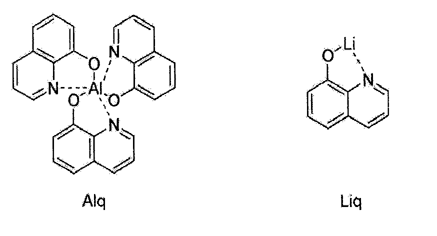

Each compound was employed as electroluminescent material for an OLED after purifying it via vacuum sublimation at 10⁻⁶ torr.

### [Comparative Example 1] Manufacture of an OLED by using conventional electroluminescent material

After forming a hole injecting layer (3) and a hole transport layer (4) according to the same procedure as described in Example 1, dinaphthylanthracene (DNA) was charged to one cell of said vacuum vapor-deposit device, and DSA-Ph (as was in Example 1) was charged to another cell. Then an electroluminescent layer (5) was vapor-deposited on the hole transport layer with a thickness of 30 nm at a vapor-deposition rate of 100:3.

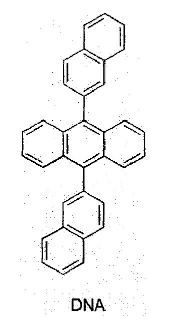

Then, an electron transport layer (6) and electron injecting layer (7) were vapor-deposited according to the same procedure as in Example 1, and an Al cathode (8) was vapor-deposited thereon with a thickness of 150 nm by using another vacuum vapor-deposit device to manufacture an OLED.

### [Example 2] Manufacture of an OLED employing the compound according to the invention

After forming a hole injecting layer and a hole transport layer according to the same procedure as in Example 1, a compound according to the present invention (e.g., Compound 10) was charged to one cell of said vacuum vapor-deposit device as electroluminescent material, and Compound (E) (of which the structure is shown below) was charged to another cell. The two substances were evaporated at different rates to provide doping at 2 to 5% by weight on the basis of the host, to vapor-deposit an electroluminescent layer of the thickness of 30 nm on the hole transport layer.

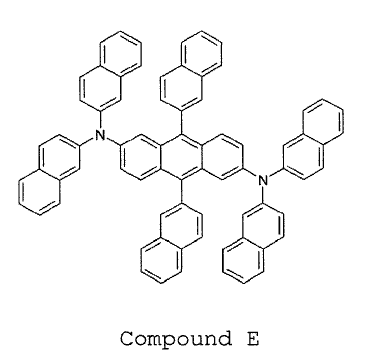

After vapor-depositing an electron transport layer and an electron injecting layer according to the same procedure as in Example 1, an Al cathode was vapor-deposited with a thickness of 150 nm by using another vacuum vapor-deposit device to manufacture an OLED.

### [Comparative Example 2] Manufacture of an OLED by using conventional electroluminescent material

After forming a hole injecting layer and a hole transport layer according to the same procedure as described in Example 2, tris(8-hydroxyquinoline)-aluminum (III) (Alq) was charged to another cell of said vacuum vapor-deposit device, and Coumarin 545T (C545T) was charged to still another cell. The two substances were evaporated at different rates to carry out doping, thereby vapor-depositing an electroluminescent layer with a thickness of 30 nm on the hole transport layer. The doping concentration preferably is from 1 to 3% by weight on the basis of Alq.

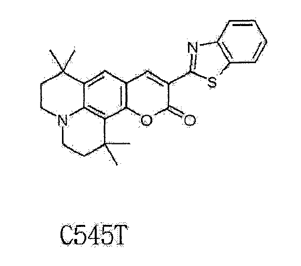

After vapor-depositing an electron transport layer and an electron injecting layer according to the same procedure as in Example 1, an Al cathode was vapor-deposited with a thickness of 150 nm by using another vacuum vapor-deposit device to manufacture an OLED.

### [Example 3] Electroluminescent properties of OLED's manufactured

The luminous efficiencies of the OLED's comprising the organic electroluminescent compounds according to the present invention (Examples 1 and 2) or conventional EL compound (Comparative Examples 1 and 2) were measured at 5,000 cd/m², respectively, and the results are shown in Table 2.

**Table 2**

| No. | Host | Dopant | Doping concentration (wt%) | Luminous efficiency (cd/A) | Color |
|---|---|---|---|---|---|
| | | | | @5000cd/m² | |
| 1 | 2 | DSA-Ph | 3 | 8.1 | Blue |
| 2 | 6 | DSA-Ph | 3 | 8.5 | Blue |
| 3 | 13 | DSA-Ph | 3 | 7.2 | Blue |
| 4 | 26 | DSA-Ph | 3 | 7.6 | Blue |
| 5 | 32 | DSA-Ph | 3 | 7.3 | Blue |
| 6 | 49 | DSA-Ph | 3 | 7.9 | Blue |
| 7 | 66 | DSA-Ph | 3 | 8.4 | Blue |
| 8 | 160 | DSA-Ph | 3 | 7.5 | Blue |
| 9 | 234 | DSA-Ph | 3 | 7.2 | Blue |
| 10 | 250 | DSA-Ph | 3 | 8.3 | Blue |
| 11 | 329 | DSA-Ph | 3 | 7.9 | Blue |
| 12 | 27 | Compoud E | 3 | 18.2 | Green |
| 13 | 60 | Compoud E | 3 | 18.6 | Green |
| 14 | 108 | Compoud E | 3 | 18.2 | Green |
| 15 | 131 | Compoud E | 3 | 18.9 | Green |
| 16 | 156 | Compoud E | 3 | 18.7 | Green |
| 17 | 204 | Compoud E | 3 | 20.2 | Green |
| 18 | 253 | Compoud E | 3 | 20.8 | Green |
| 19 | 268 | Compoud E | 3 | 21.6 | Green |
| 20 | 415 | Compoud E | 3 | 19.1 | Green |
| 21 | 456 | Compoud E | 3 | 19.3 | Green |
| 22 | 470 | Compoud E | 3 | 18.5 | Green |
| Comp. 1 | DNA | DSA-Ph | 3 | 7.3 | Jade green |
| Comp.2 | Alq | Compound C545T | 1 | 10.3 | Green |

As can be seen from Table 2, when the organic electroluminescent compound according to the invention was applied to a blue electroluminescent device, with same type of doping of DSA-Ph, the device realized far better color purity as compared to the device employing DNA according to Comparative Example 1 (conventional electroluminescent material), though having comparable luminous efficiency.

Further, the material according to the invention was applied to green electroluminescent devices. As can be seen from Table 1, the device employing Compound (1027) (an electroluminescent compound according to the invention) with 3.0% doping of Compound (E) showed more than twice of luminous efficiency as compared to the device employing Alq:C545T as conventional material (Comparative Example 2).

Accordingly, the organic electroluminescent compounds according to the present invention can be used as blue or green electroluminescent material of high efficiency. Moreover, the device, to which the host material according to the invention was applied, showed noticeable improvement in view of color purity. The improvement in both color purity and luminous efficiency proves that the materials of the present invention have excellent properties.

## Claims

1. An organic electroluminescent compound represented by Chemical Formula (1): 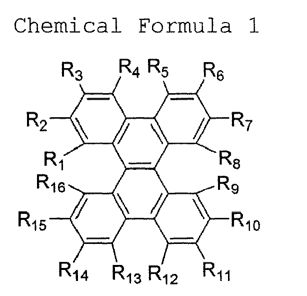 In Chemical Formula (1),
R₁ through R₁₆ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked to an adjacent substituent via (C3-C60)alkylene or (C3-C60)alkenylene to form an alicyclic ring, or a monocyclic or polycyclic aromatic ring, which may be selected from the following substituents: 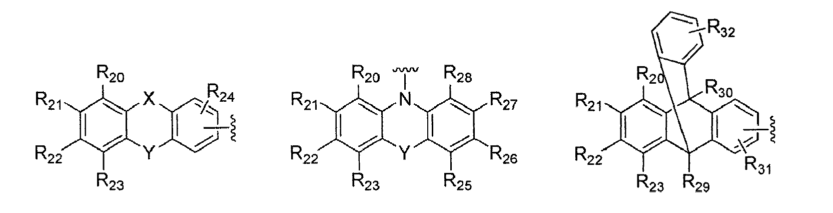 wherein, R₂₀ through R₃₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀ through R₃₂ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or -(CR₃₃R₃₄)ₙ-, -N(R₃₅)-, -S-, -O-, -Si(R₃₆)(R₃₇)-, -P(R₃₈)-, -C(=O)-, -B(R₃₉)-, -In(R₄₀)-, -Se-, -Ge(R₄₁)(R₄₂)-, -Sn(R₄₃)(R₄₄)-, Ga(R₄₅)- or -(R₄₆)C=C(R₄₇)-; n is an integer from 1 to 4; R₃₃ through R₄₇ represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked via (C3-C60)alkylene or (C3-C60)alkenylene with or without a fused ring to form an alicyclic ring, or a monocyclic or polycyclic aromatic ring; and
the alkyl, alkenyl, alkynyl, cycloalkyl, heterocycloalkyl, aryl, heteroaryl, adamantyl, bicycloalkyl, arylsilyl, alkylsilyl, alkylamino and arylamino of R₁ through R₄₇ may be further substituted by halogen, (C1-C60)alkyl with or without halogen substituent(s), (C6-C60)aryl, (C3-C60)heteroaryl with or without (C6-C60)aryl substituent, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, or an alicyclic ring, or a monocyclic or polycyclic aromatic ring formed by linkage via (C3-C60)alkylene or (C3-C60)alkenylene with or without a fused ring.

2. The organic electroluminescent compound according to claim 1, which is selected from the compounds represented by Chemical Formula (2): 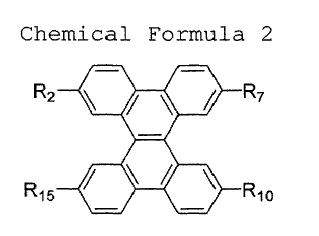 wherein, R₂, R₇, R₁₀ and R₁₅ are defined as in claim 1.

3. The organic electroluminescent compound according to claim 1, wherein R₁ through R₁₆ are independently selected from hydrogen, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluorethyl, trifluoroethyl, perfluoropropyl, perfluorobutyl, methoxy, ethoxy, butoxy, hexyloxy, cyclopentyl, cycloheptyl, cyclooctyl, fluoro, cyano, trimethylsilyl, triethylsilyl, tripropylsilyl, tri(t-butyl)silyl, t-butyldimethylsilyl, dimethylphenylsilyl, triphenylsilyl, and the following structures: 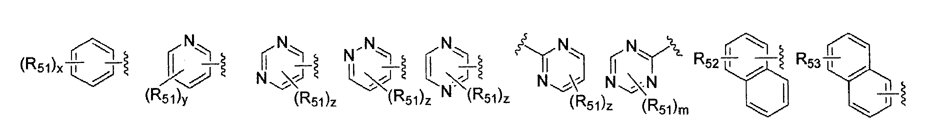 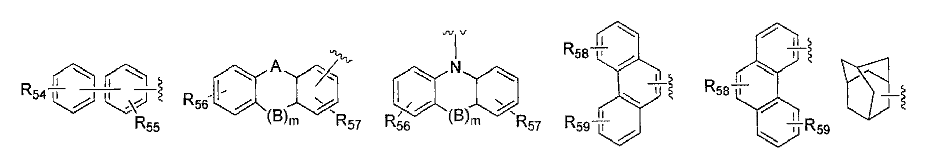 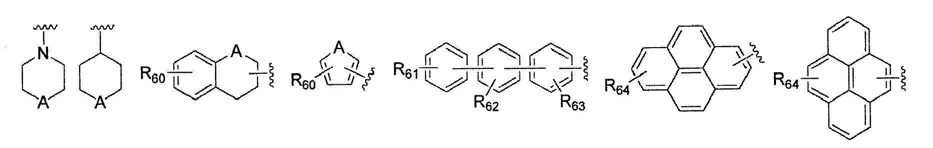 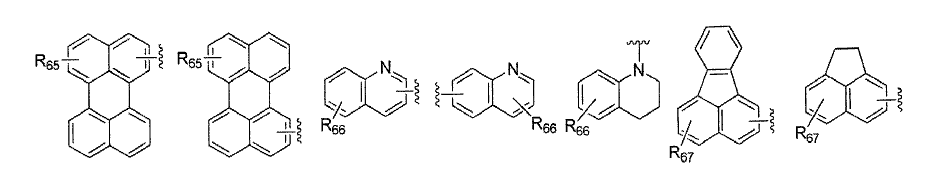 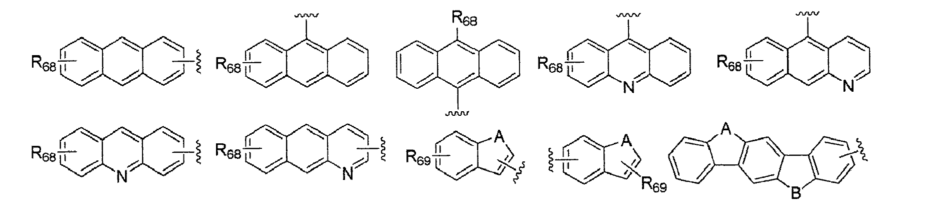 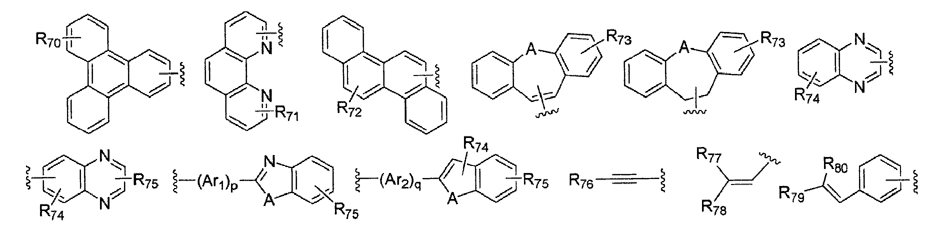 wherein, R₅₁ through R₈₀ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
the alkyl, aryl, heteroaryl, heterocycloalkyl, cycloalkyl, trialkylsilyl, dialkylarylsilyl, triarylsilyl, adamantyl, bicycloalkyl, alkenyl, alkynyl, alkylamino or arylamino of R₅₁ through R₈₀ may be further substituted by halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
A and B independently represent a chemical bond, or they are selected from -C(R₉₁)(R₉₂)-, -N(R₉₃)-, -0-, -S-, -Si(R₉₄)(R₉₅)-, -P(R₉₆)-, -C(=O) and -(R₉₇₎C=C(R₉₈)-, wherein R₉₁ through R₉₈ independently represent hydrogen, (C1-C60)alkyl, (C1-C30)alkoxy, cyano, -CF₃, halogen, (C6-C60)aryl, tri(C1-C30)alkylsilyl, di(C1-C30)alkyl(C6-C30)arylsilyl or tri(C6-C30)arylsilyl; the alkyl or aryl of R₉₁ through R₉₈ may be further substituted by one or more substituent(s) selected from (C1-C60)alkyl, (C1-C30)alkoxy, halogen, cyano, tri(C1-C30)alkylsilyl, di(C1-C30)alkyl(C6-C30)arylsilyl, tri(C6-C30)arylsilyl, (C5-C60)heteroaryl and (C6-C60)aryl; or R₉₁ and R₉₂, R₉₄ and R₉₅, or R₉₇ and R₉₈ may be linked via (C3-C20)alkylene or (C3-C20)alkenylene with or without a fused ring to form an alicyclic ring, or a monocyclic or polycyclic aromatic ring;
Ar₁ and Ar₂ independently represent (C6-C60)arylene or (C5-C60)heteroarylene; the arylene or heteroarylene of Ar₁ and Ar₂ may be further substituted by one or more substituent(s) selected from (C1-C60)alkyl, halogen, cyano, (C1-C60)alkoxy, (C3-C60)cycloalkyl, (C6-C60)aryl, (C3-C60)heteroaryl, adamantyl, (C7-C60)bicycloalkyl, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, tri(C1-C30)alkylsilyl, di(C1-C30)alkyl(C6-C30)arylsilyl and tri(C6-C30)arylsilyl;
x is an integer from 0 to 5, y is an integer from 0 to 4, z is an integer from 0 to 3; and
m, p and q represent an integer from 0 to 2.

4. The organic electroluminescent device which is comprised of a first electrode; a second electrode; and at least one organic layer(s) interposed between the first electrode and the second electrode; wherein the organic layer comprises one or more organic electroluminescent compound represented by Chemical Formula (1): 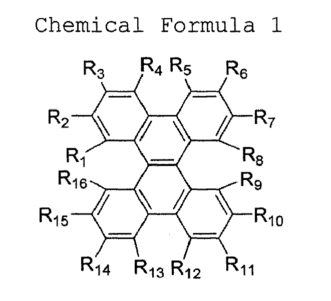 In Chemical Formula (1),
R₁ through R₁₆ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked to an adjacent substituent via (C3-C60)alkylene or (C3-C60)alkenylene to form an alicyclic ring, or a monocyclic or polycyclic aromatic ring, which may be selected from the following substituents: 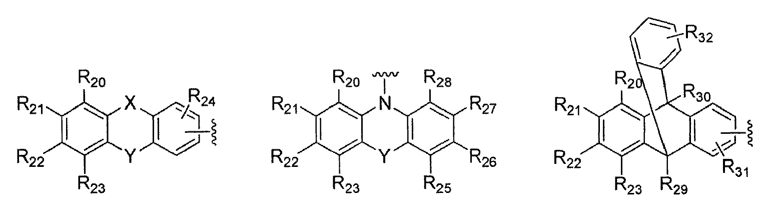 wherein, R₂₀ through R₃₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀ through R₃₂ may be linked to an adjacent substituent via (C3-C60)alkylene or (C3-C60)alkenylene with or without a fused ring to form an alicyclic ring, or a monocyclic or polycyclic aromatic ring; X and Y independently represent a chemical bond, or -(CR33R34)n-, -N(R35)-, -S-, -O-, -Si(R36)(R37)-, -P(R38)-, -C(=O)-, -B(R39)-, -In(R40)-, -Se-, -Ge(R41)(R42)-, -Sn(R43)(R44)-, -Ga(R45)- or -(R46)C=C(R47)-; n is an integer from 1 to 4; R33 through R47 represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked via (C3-C60)alkylene or (C3-C60)alkenylene with or without a fused ring to form an alicyclic ring, or a monocyclic or polycyclic aromatic ring; and the alkyl, alkenyl, alkynyl, cycloalkyl, heterocycloalkyl, aryl, heteroaryl, adamantyl, bicycloalkyl, arylsilyl, alkylsilyl, alkylamino and arylamino of R1 through R47 may be further substituted by halogen, (C1-C60)alkyl with or without halogen substituent(s), (C6-C60)aryl, (C3-C60)heteroaryl with or without (C6-C60)aryl substituent, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, or an alicyclic ring, or a monocyclic or polycyclic aromatic ring formed by linkage via (C3-C60)alkylene or (C3-C60)alkenylene with or without a fused ring and one or more dopant(s) selected from the compounds represented by one of Chemical Formulas (3) to (5): 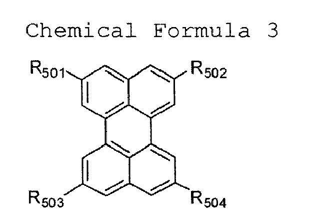 In Chemical Formula (3), R501 through R504 independently represent hydrogen, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl; or each of R₅₀₁ through R₅₀₄ may be linked to an adjacent substituent via (C3-C60)alkylene or (C3-C60)alkenylene with or without fused ring to form an alicyclic ring, or a monocyclic or polycyclic aromatic ring; the alkyl, alkenyl, alkynyl, cycloalkyl, heterocycloalkyl, aryl, heteroaryl, arylsilyl, alkylsilyl, alkylamino, arylamino of R₅₀₁ through R₅₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl. 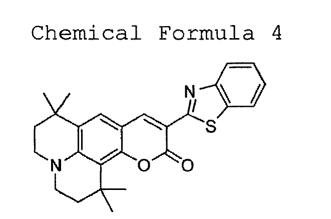 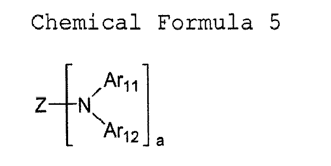 In Chemical Formula (5), Ar₁₁ and Ar₁₂ independently represent (C1-C60)alkyl, (C6-C60)aryl, (C4-C60)heteroaryl, (C6-C60)arylamino, (Cl-C60)alkylamino, 5- or 6-membered heterocycloalkyl containing one or more heteroatom(s) selected from N, O and S, or (C3-C60)cycloalkyl, or Ar₁₁ and Ar₁₂ may be linked via (C3-C60)alkylene or (C3-C60)alkenylene with or without a fused ring to form an alicyclic ring, or a monocyclic or polycyclic aromatic ring, or they are selected from the arylene groups having one of the structures shown below: 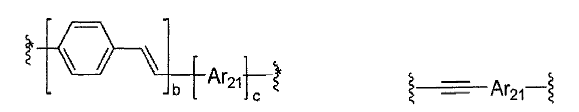 Z represents (C6-C60)arylene, (C4-C60)heteroarylene or arylene having one of the structures shown below: 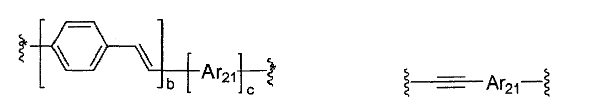 wherein, Ar₂₁ represents (C6-C60)arylene or (C4-C60)heteroarylene;
a is an integer from 1 to 4, b is an integer from 1 to 4, and c is an integer of 0 or 1; and the alkyl, aryl, heteroaryl, arylamino, alkylamino, cycloalkyl or heterocycloalkyl of Ar₁₁ and Ar₁₂; or the arylene or heteroarylene of Z and Ar₂₁ may be further substituted by one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

5. The organic electroluminescent device according to claim 4, wherein the organic layer comprises one or more compounds selected from arylamine compounds and styrylarylamine compounds.

6. The organic electroluminescent device according to claim 4, wherein the organic layer comprises one or more metal(s) selected from a group consisting of organic metals of Group 1, Group 2, 4^{th} period and 5^{th} period transition metals, lanthanide metals and d-transition elements in the Periodic Table of Elements.

7. The organic electroluminescent device according to claim 5, wherein the organic layer comprises a charge generating layer as well as an electroluminescent layer.

8. A white electroluminescent device which comprises an organic electroluminescent compound represented by Chemical Formula (1): 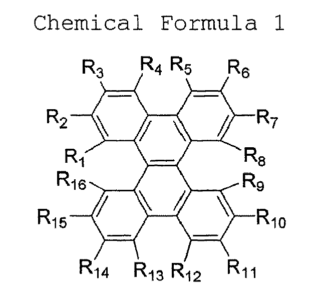 In Chemical Formula (1),
R₁ through R₁₆ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked to an adjacent substituent via (C3-C60)alkylene or (C3-C60)alkenylene to form an alicyclic ring, or a monocyclic or polycyclic aromatic ring, which may be selected from the following substituents: 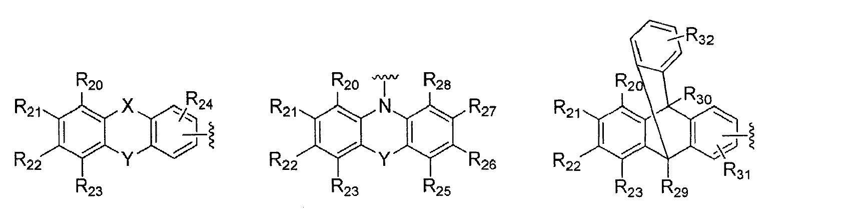 wherein, R₂₀ through R₃₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60) alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀ through R₃₂ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or -(CR₃₃R₃₄)ₙ-, -N(R35), -S-, -O-, -Si(R₃₆)(R₃₇)-, -P(R₃₈)-, -C(=O)-, -B(R₃₉)-, -In(R₄₀)-, -Se-, -Ge(R₄₁)(R₄₂)-, -Sn(R₄₃)(R₄₄)-, -Ga(R₄₅)- or -(R₄₆)C=C(R₄₇)-; n is an integer from 1 to 4; R₃₃ through R₄₇ represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamin (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked via (C3-C60)alkylene or (C3-C60)alkenylene with or without a fused ring to form an alicyclic ring, or a monocyclic or polycyclic aromatic ring; and the alkyl, alkenyl, alkynyl, cycloalkyl, heterocycloalkyl, aryl, heteroaryl, adamantyl, bicycloalkyl, arylsilyl, alkylsilyl, alkylamino and arylamino of R₁ through R₄₇ may be further substituted by halogen, (C1-C60)alkyl with or without halogen substituent(s), (C6-C60)aryl, (C3-C60)heteroaryl with or without (C6-C60)aryl substituent, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, or an alicyclic ring, or a monocyclic or polycyclic aromatic ring formed by linkage via (C3-C60)alkylene or (C3-C60)alkenylene with or without a fused ring.

9. An organic solar cell which comprises an organic electroluminescent compound represented by Chemical Formula (1): 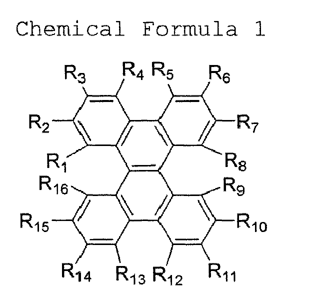 In Chemical Formula (1),
R₁ through R₁₆ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked to an adjacent substituent via (C3-C60)alkylene or (C3-C60)alkenylene to form an alicyclic ring, or a monocyclic or polycyclic aromatic ring, which may be selected from the following substituents: 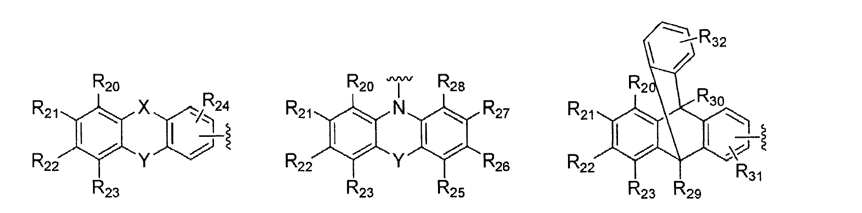 wherein, R₂₀ through R₃₂ independently represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₂₀ through R₃₂ may be linked to an adjacent substituent via (C3-C60)alkylene or (C3-C60)alkenylene with or without a fused ring to form an alicyclic ring, or a monocyclic or polycyclic aromatic ring;
X and Y independently represent a chemical bond, or -(CR₃₃R₃₄)ₙ-, -N(R₃₅)-, -S-, -O-, -Si(R₃₆)(R₃₇)-, -P(R₃₈)-, -C(=O)-, -B(R₃₉)-, -In(R₄₀)-, -Se-, -Ge(R₄₁)(R₄₂)-, -Sn(R₄₃)(R₄₄)-, -Ga(R₄₅)- or -(R₄₆)C=C(R₄₇)-; n is an integer from 1 to 4; R₃₃ through R₄₇ represent hydrogen, halogen, (C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of them may be linked via (C3-C60)alkylene or (C3-C60)alkenylene with or without a fused ring to form an alicyclic ring, or a monocyclic or polycyclic aromatic ring; and the alkyl, alkenyl, alkynyl, cycloalkyl, heterocycloalkyl, aryl, heteroaryl, adamantyl, bicycloalkyl, arylsilyl, alkylsilyl, alkylamino and arylamino of R₁ through R₄₇ may be further substituted by halogen, (C1-C60)alkyl with or without halogen substituent(s), (C6-C60)aryl, (C3-C60)heteroaryl with or without (C6-C60)aryl substituent, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro, hydroxyl, or an alicyclic ring, or a monocyclic or polycyclic aromatic ring formed by linkage via (C3-C60)alkylene or (C3-C60)alkenylene with or without a fused ring.
